# EUROPEAN PATENT APPLICATION

(11) **EP 4 378 950 A1**
(43) Date of publication of application: **05.06.2024**
(21) Application number: 22849889.5
(22) Date of filing: 27.07.2022
(51) Int. Cl.: C07K 7/06, A61K 8/64, A61Q 19/00, A61P 21/00, A61K 38/00

(54) **OPTIMIZED SHORTENED PEPTIDE THAT BINDS TO ACETYLCHOLINE RECEPTOR, AND USE THEREOF**

(30) Priority: 27.07.2021 KR 20210098837; 26.07.2022 KR 20220092749
(71) Applicant: Skinmed Co., Ltd., Daejeon 34050 (KR)
(72) Inventor: KIM, Sung Hyun, Sejong 30098 (KR); BANG, Jin Ho, Cheongju-si, Chungcheongbuk-do 28166 (KR); LEE, Jeung Hoon, Daejeon 34124 (KR); LEE, Min Ho, Daejeon 34087 (KR); SHIN, Yong Chul, Jinju-si, Gyeongsangnam-do 52817 (KR); CHOI, Won Il, Seoul 04500 (KR)
(74) Representative: Kraus & Lederer PartGmbB
(86) International application number: PCT/KR2022/011078
(87) International publication number: WO 2023/008913

(57) **Abstract**

The present disclosure relates to an optimized shortened peptide that binds to an acetylcholine receptor, and a use thereof. It is expected that it is possible to develop a cosmetic composition for alleviating wrinkles, pharmaceuticals for preventing or treating acetylcholine receptor-related diseases, and health functional foods for improvement of the acetylcholine receptor-related diseases, by using the optimized shortened peptide of the present disclosure, which is shorter than conventional acetylcholine receptor inhibitory peptides but has a high acetylcholine receptor binding force and an acetylcholine receptor inhibitory effect.

## Description

### Technical Field

The present disclosure relates to an acetylcholine receptor-binding optimized shortened peptide and a use thereof. More specifically, the present disclosure relates to an acetylcholine receptor-binding, optimized shortened peptide expressed in a certain manner for the optimization of peptides, which inhibits the function of the acetylcholine receptor by binding thereto, and a use thereof.

### Background Art

Acetylcholine is a chemical substance present in the nervous tissue of animals, secreted at the nerve endings, and plays a role in transmitting nerve stimulation to muscles. The transmitter substances secreted from nerve endings include acetylcholine in motor and parasympathetic nerves and epinephrine (adrenaline) in sympathetic nerves. When acetylcholine is secreted, it exhibits physiological actions such as lowering blood pressure, inhibiting heartbeats, contracting intestines, and contracting skeletal muscles. For muscles to contract, nerves have to command muscles to contract at the junction where nerves and muscles meet (neuromuscular junction), where nerves secrete acetylcholine, and this substance binds to the acetylcholine receptors on the muscles, enabling muscle contraction (Vincent, A., 1985; Lindstrom, J.M., et al., 1976). Blocking the peripheral acetylcholine receptors that dominate the femoral skeletal muscles causes paralysis of muscle movement, and blocking the acetylcholine receptors of smooth muscles and cardiac muscles responsible for breathing or heart movements leads to paralysis of breathing or heart movements.

Acetylcholine receptors are classified into muscarinic acetylcholine receptors (mAchR) and nicotinic acetylcholine receptors (nAchR). Muscarinic acetylcholine receptors are G protein-coupled receptors that can be activated by muscarine and activate different signaling mechanisms depending on the subtype. They are distributed all over the body, including the central nervous system and peripheral organs, mainly mediating the physiological actions of acetylcholine secreted from postganglionic fibers of the parasympathetic nervous system.

Nicotinic acetylcholine receptors are receptors that mimic the pharmacological actions caused by nicotine and are ion channels manipulated by neurotransmitters. They non-selectively allow the passage of ions such as sodium, potassium, and calcium through the opening and closing of ion channels, regulating electronic signaling between nerve and muscle cells. Nicotinic acetylcholine receptors can be divided into muscle type and neuronal type based on the expression site. Muscle-type nicotinic acetylcholine receptors are expressed at the neuromuscular junction where motor neurons meet skeletal muscles and contribute to inducing end plate potential (EPP) in skeletal muscle cell membranes when acetylcholine is secreted from motor neurons. Meanwhile, neuronal-type nicotinic acetylcholine receptors are expressed in peripheral ganglia of the autonomic nervous system (ANS) and contribute to exciting postganglionic fibers by acetylcholine secreted from preganglionic fibers.

Drugs that interfere or inhibit the activity of acetylcholine or mimic its actions are being used very effectively. Acetylcholine receptor agonists are used to treat severe myasthenia gravis and Alzheimer's disease. In the case of severe myasthenia gravis, it is an autoimmune disease caused by the body producing antibodies against nicotinic acetylcholine receptors, inhibiting normal acetylcholine signal transmission. Using acetylcholine esterase (AChE) inhibitors can increase the time each receptor can interact with acetylcholine in the synaptic gap between nerve and muscle before inactivation of the acetylcholine, thus treating severe myasthenia gravis.

Moreover, interference with the secretion of acetylcholine can inhibit muscle contraction, causing paralysis of muscles and smoothing wrinkles; this is utilized in Botox. Botox blocks the process of acetylcholine secretion, which is essential for muscle contraction at the motor nerve terminals. As a result, muscles become immobile, and wrinkles caused by muscles disappear. The muscle-relaxing effect of Botox gradually fades after 3-6 weeks, necessitating repeated administrations.

Furthermore, cosmetic peptides have been developed using the mechanism of smoothing wrinkles by inhibiting the binding of acetylcholine to its receptors. An example is Synake by DSM, a snake venom-derived peptide, known among wrinkle-improving peptides for having the best clinical effect (approximately 52%) and is widely used as an ingredient in peptide cosmetics.

The inventors, while researching acetylcholine receptor-binding peptides, screened and secured peptides with high binding affinity to the acetylcholine receptors. They confirmed that these peptides inhibit acetylcholine binding by binding to the acetylcholine receptors, thus inhibiting acetylcholine receptor action. However, as acetylcholine receptor-binding peptides were provided as peptide fragments, there were issues such as increased manufacturing costs with longer peptides and reduced skin permeability when manufactured as cosmetics. Consequently, there was a need for research on more shortened peptides while maintaining high binding affinity to the acetylcholine receptors.

As prior art, Korean Patent No. 10-2020-0080179 A issued to the present inventors discloses acetylcholine receptor inhibitory peptides and uses thereof. Still, only 8mer, 11mer, 14mer, and 18mer peptides are listed for acetylcholine receptor inhibitory peptides, and the optimized shortened peptides of the present disclosure and their effects are not mentioned. Also, Korean Patent No. 1216008 discloses acetylcholine receptor-binding peptides selected using biopanning, but does not include the peptide and library containing the amino acid sequence of the present disclosure.

Moreover, Korean Patent No. 2018-0028748 A describes a peptide regulating the release of neurotransmitters including acetylcholine, and the effect thereof on reducing wrinkles, but does not disclose the binding affinity of peptides containing the amino acid sequence of the present disclosure to acetylcholine receptors and the resulting inhibition of acetylcholine receptor action. Korean Patent No. 2014-0139010 A mentions a peptide for enhancing transdermal penetration but differs in composition and effect from the action-inhibiting peptide through acetylcholine receptor binding of the present disclosure.

### Disclosure of Invention

### Technical Problem

The present disclosure aims primarily to provide an optimized shortened peptide that binds to an acetylcholine receptor. Additionally, the present disclosure is to provide an optimized shortened peptide that binds to acetylcholine receptors.

In addition, the present disclosure aims to provide a cosmetic composition for wrinkle reduction, containing an optimized shortened peptide that binds to an acetylcholine receptor. Furthermore, the present disclosure is to provide a composition for preventing or treating diseases related to excessive activity of acetylcholine receptors, containing an optimized shortened peptide that binds to an acetylcholine receptor.

The present disclosure aims to provide a health functional food composition and a medical device composition for alleviating diseases related to excessive activity of acetylcholine receptors, each containing an optimized shortened peptide that bind to an acetylcholine receptor.

### Solution to Problem

To accomplish the tasks, the present disclosure provides an optimized shortened peptide that binds to an acetylcholine receptor.

As used herein, the term "amino acid" or "any amino acid" encompasses both natural amino acids and other amino acids, such as non-natural amino acids, amino acids not encoded by genetic sequences, including both L- and D- isomers, used in the synthesis of peptides in the field of peptides.

The natural amino acids may be alanine (Ala, A), cysteine (Cys, C), aspartic acid (Asp, D), glutamic acid (Glu, E), phenylalanine (Phe, F), glycine (Gly, G), histidine (His, H), isoleucine (Ile, I), lysine (Lys, K), leucine (Leu, L), methionine (Met, M), asparagine (Asn, N), proline (Pro, P), glutamine (Gln, Q), arginine (Arg, R), serine (Ser, S), threonine (Thr, T), valine (Val, V), tryptophan (Trp, W), and tyrosine (Tyr, Y).

The other amino acids include a wide variety of modified or unusual amino acids, examples of which include 2-aminoadipic acid ( 2-aminohexanedioic acid), α-asparagine, 2-aminobutanoic acid, 2-aminocapric acid (2-aminodecanoic acid), α-glutamine, α-aminoisobutyric acid (α-methylalanine), 2-aminopimelic acid (2-aminohepanedioic acid), γ-amino-β-hydroxybenzenepentanoic acid, 2-aminosuberic acid (2-aminooctanedioic acid), 2-carboxyazetidine, β-alanine, β-aspartic acid, 3, 6-diaminohexanoic acid ( β-lysine), butanoic acid, 4-amino-3-hydroxybutanoic acid, γ-amino-β-hydroxycyclohexanepentanoic acid, 3-cyclohexylalanine, N5-aminocarbonylornithine, 3-sulfoalanine, 2,3-diaminopropioic acid, 2,7-diaminosuberic acid (2,7-diaminooctanedioic acid), S-ethylthiocysteine, γ-glutamic acid, γ-carboxylglutamic acid, hydroxyacetic acid (glycolic acid), pyroglutamic acid, homogrginine, homocysteine, homohistidine, 2-hydroxyisovaleric acid, homoserine, 2-hydroxypentanoic acid, 5-hydroxylysine, 4-hydroxyproline, isovaline, 2-hydroxypropanoic acid (lactic acid), mercaptoacetic acid, mercaptobutanoic acid, 3-hydroxy-4-methylproline, mercaptopropanoic acid, 3-naphthylalanine, norleucine, nortyrosine, norvaline, 2-carboxyoctahydroindole, ornithine, penicillamine (3-mercaptovaline), 2-phenylglycine, 2-carboxypiperidine, sarcosine (N-methylglycine), 1-amino-1-carboxycyclopentane, statin (4-amino-3-hydroxy-6-methylheptanoic acid), 3-thienylalanine, 3-carboxyisoquinoline, 3-methylvaline, ε-N-trimethyllysine, 3-thiazolylalanine, α-amino-2,4-dioxopyrimidinepropanoic acid, etc.

As used herein, the "peptide" refers to a polymer made up of two or more amino acids linked by amide or peptide bonds.

In the present disclosure, "acetylcholine receptor" (AchR) refers to a receptor that binds to acetylcholine secreted from nerve endings, acting as a pathway for transmitting stimulation by acetylcholine. For example, when a muscle needs to contract, the nerve tells the muscle to contract, and the nerve secretes acetylcholine at the neuromuscular junction. When the secreted acetylcholine binds to the acetylcholine receptor in the muscle, the muscle contracts.

The acetylcholine receptors in the present disclosure are classified into muscarinic acetylcholine receptors and nicotinic acetylcholine receptors, with nicotinic acetylcholine receptors being preferred in the present disclosure.

The present disclosure provides an acetylcholine receptor-binding peptide consisting of the amino acid sequence represented by the following Chemical Formula 1.

[Chemical Formula 1] (R/K)XXX(R/K)

(wherein R/K is arginine or lysine, X is any amino acid)

The peptide consists of a sequence of five amino acids, where the first and fifth amino acids are each arginine or lysine, and the second, third, and fourth amino acids are any amino acids.

The acetylcholine receptor-binding peptide consists of a sequence of five amino acids as expressed in [Chemical Formula 1], where the 1^{st} and 5^{th} amino acids, K or R, may be critical for binding to acetylcholine receptors. In the amino acid sequence represented by Chemical Formula 1, each of the 2^{nd}, 3^{rd}, and 4^{th} amino acids may be any amino acid. This indicates that provided that the 1ₛₜ and 5^{th} amino acids are K or R, the peptide can still show a certain level of acetylcholine receptor binding capacity even though the 2^{nd}, 3^{rd}, and 4^{th} amino acids change.

The present disclosure provides an acetylcholine receptor-binding peptide consisting of the amino acid sequence represented by the following Chemical Formula 1-1.

[Chemical Formula 1-1] (R/K) XYZ (R/K)

(wherein R/K represents either arginine or lysine, and XYZ represents a sequence of any three consecutive amino acids, with X being an amino acid selected from R, Q, G, V, L, S, and W, Y being an amino acid selected from R, Q, L, I, F, V, and Y, and Z being an amino acid selected from R, S, L, C, Y, Q, and T)

The amino acid sequence represented by Chemical Formula 1-1 may be selected from the group consisting of the amino acid sequences of SEQ ID NOS: 1 to 600.

The present disclosure also provides an acetylcholine receptor-binding peptide consisting of the amino acid sequence represented by the following Chemical Formula 1-2.

[Chemical Formula 1-2] XRRQRR

(wherein R represents arginine, Q represents glutamine, and X represents any one amino acid).

The amino acid sequence represented by Chemical Formula 1-2 may be selected from the group consisting of the amino acid sequences of SEQ ID NOS:3601 to 3620.

The present disclosure further provides an acetylcholine receptor-binding peptide consisting of the amino acid sequence represented by the following Chemical Formula 1-3.

[Chemical Formula 1-3] RRQRRX

(Wherein R represents arginine, Q represents glutamine, and X represents any one amino acid)

The amino acid sequence represented by Chemical Formula 1-3 may be selected from the group consisting of the amino acid sequences of SEQ ID NOS: 3621 to 3640.

The present disclosure also provides an acetylcholine receptor-binding peptide consisting of the amino acid sequence represented by the following Chemical Formula 2.

[Chemical Formula 2] (R/K) (R/K) XXX (R/K)

(Wherein R/K represents either arginine or lysine, and X represents any one amino acid)

The acetylcholine receptor-binding peptide includes a sequence of 6 amino acids as expressed in Chemical Formula 2, where the 1^{st}, 2^{nd}, and 6^{th} amino acids, each being K or R, may be critical for binding to acetylcholine receptors. In the amino acid sequence represented by Chemical Formula 2, each of the 3^{rd}, 4^{th}, and 5^{th} amino acids may be any amino acid. This indicates that provided that the 1^{st}, 2^{nd}, and 6^{th} amino acids are K or R, the peptide can still show a certain level of acetylcholine receptor binding capacity even though the 3^{rd}, 4^{th}, and 5^{th} amino acids change.

The present disclosure provides an acetylcholine receptor-binding peptide consisting of the amino acid sequence represented by the following Chemical Formula 2-1.

[Chemical Formula 2-1] (R/K) (R/K)XYZ (R/K)

(wherein R/K represents either arginine or lysine, XYZ represents a sequence of any three consecutive amino acids, with X being an amino acid selected from R, Q, G, V, L, S, and W, Y being an amino acid selected from R, Q, L, I, F, V, and Y, and Z being an amino acid selected from R, S, L, C, Y, Q, and T)

The amino acid sequence represented by Chemical Formula 2-1 may be selected from the group consisting of the amino acid sequences of SEQ ID NOS: 1201 to 1800.

The present disclosure provides an acetylcholine receptor-binding peptide consisting of the amino acid sequence represented by the following Chemical Formula 2-2.

[Chemical Formula 2-2] XRRGVRR

(wherein R represents arginine, G represents glycine, V represents valine, and X represents any one amino acid)

The amino acid sequence represented by Chemical Formula 2-2 may be selected from the group consisting of the amino acid sequences of SEQ ID NOS: 3641 to 3660.

The present disclosure provides an acetylcholine receptor-binding peptide consisting of the amino acid sequence represented by the following Chemical Formula 2-3.

[Chemical Formula 2-3] RRGVRRX

(wherein R represents arginine, G represents glycine, V represents valine, and X represents any one amino acid)

The amino acid sequence represented by Chemical Formula 2-3 may be selected from the group consisting of the amino acid sequences of SEQ ID NOS: 3661 to 3680.

The present disclosure provides an acetylcholine receptor-binding peptide consisting of the amino acid sequence represented by the following Chemical Formula 3.

[Chemical Formula 3] (R/K) XXX (R/K) (R/K)

(wherein R/K represents either arginine or lysine, and X represents any one amino acid)

The acetylcholine receptor-binding peptide consists of a sequence of 6 amino acids as expressed in Chemical Formula 3, where 1^{st}, 5^{th}, and 6^{th} amino acids, each being K or R, may be critical for binding to acetylcholine receptors. In the amino acid sequence represented by Chemical Formula 3, each of the 2^{nd}, 3^{rd}, and 4^{th} amino acids may be any amino acid. This indicates that provided that the 1^{st}, 5^{th}, and 6^{th} amino acids are K or R, the peptide can still show a certain level of acetylcholine receptor binding capacity even though the 2^{nd}, 3^{rd}, and 4^{th} amino acids change.

The present disclosure provides an acetylcholine receptor-binding peptide consisting of the amino acid sequence represented by the following Chemical Formula 3-1.

[Chemical Formula 3-1] (R/K)XYZ(R/K)(R/K)

(wherein R/K represents either arginine or lysine, XYZ represents a sequence of any three consecutive amino acids, with X being an amino acid selected from R, Q, G, V, L, S, and W, Y being an amino acid selected from R, Q, L, I, F, V, and Y, and Z being an amino acid selected from R, S, L, C, Y, Q, and T)

The amino acid sequence represented by Chemical Formula 3-1 may be selected from the group consisting of the amin acid sequences of SEQ ID NOS: 2401 to 3000.

The amino acid sequence of the acetylcholine receptor-binding peptide excludes the amino acid sequences described in Korean Patent No. 10-2020-0080179 A, which is the prior patent invention to the present disclosure, but includes the amino acid sequence RKSLLR disclosed in Korean Patent No. 10-2020-0080179 A.

The present disclosure pertains to an acetylcholine receptor-binding peptide with a modification to the N-terminus or C-terminus thereof.

In the acetylcholine receptor-binding peptide, the modification to the N-terminus or C-terminus may be palmitoylation, acetylation, formylation, PEGylation, or conjugation with 2-mercaptoacetic acid, 3-mercaptopropionic acid, 6-mercaptohexanoic acid, pyroglutamic acid, succinimide acid, amide, cystramine, methyl ester, ethyl ester, benzyl ester, or a fatty acid, such as at least one selected from the group consisting of myristic acid, stearic acid, palmitic acid, cholesterol, 6-amino hexanoic acid, and 8-amino octanoic acid.

The peptides of the present disclosure can be obtained through widely known methods in the field. Specifically, the peptides can be manufactured using genetic recombination and protein expression systems, or synthesized in vitro through chemical synthesis methods, including cell-free protein synthesis. More specifically, they can be synthesized using an automatic peptide synthesizer or produced using gene manipulation techniques, but are not limited to thereto. For example, a gene encoding a fusion protein comprising a fusion partner and the peptide of the present disclosure can be constructed by genetic manipulation, and the constructed gene can be transformed into a host microorganism. The fusion protein can then be expressed in the host microorganism, and the peptide of the present disclosure can be cleaved and separated from the fusion protein using proteolytic enzymes or compounds to produce the desired peptide.

The peptide of the present disclosure may exist in a salt form. Salt forms available in the present disclosure can be formed during the final isolation and purification of the compound or by reacting the amino groups with an appropriate acid. For example, salts may include acetate, adipate, alginate, citrate, aspartate, benzoate, benzene sulfonate, bisulfate, butyrate, camphorate, camphorsulfonate, digluconate, glycerophosphate, hemisulfate, heptanoate, hexanoate, formate, fumarate, hydrochloride, hydrobromide, hydroiodide, 2-hydroxyethanesulfonate, lactate, maleate, methanesulfonate, naphthalenesulfonate, nicotinate, 2-naphthalenesulfonate, oxalate, pamoate, pectinate, persulfate, 3-phenylpropionate, picrate, pivalate, propionate, succinate, tartrate, trichloroacetate, trifluoroacetate, phosphate, glutamate, bicarbonate, para-toluenesulfonate, undecanoate, and more but are not limited thereto. Additionally, the acids used to form acid addition salts may include inorganic acids such as hydrochloric acid, hydrobromic acid, sulfuric acid, and phosphoric acid, and organic acids such as oxalic acid, malic acid, succinic acid, and citric acid, but are not limited to thereto.

With respect to the peptides, their N- or C-termini may be modified by adding a targeting sequence, a tag, a labeled residue, or an amino acid sequence designed for increasing the stability or half-life of the peptides; an antibody or an antibody fragment thereof, human serum albumin (HSA), and the like designed for increasing targeting efficacy or stability.

The term "antibody" refers to a specific protein molecule that is directed to an antigenic site. Preferably, the antibody refers to an antibody that specifically binds to a specific protein or an immunogenic fragment thereof, and may include all of monoclonal antibodies (mAb), polyclonal antibodies (pAb), and recombinant antibodies. The antibody may be easily produced using a known technique widely known in the art.

The antibody may include a complete form having two full-length light chains and two full-length heavy chains as well as a functional fragment of an antibody molecule. The functional fragment of the antibody molecule refers to a fragment retaining at least an antigen-binding function, and includes Fab, F(ab'), F(ab')₂, F(ab)₂, Fv, and the like.

The peptides may be encapsulated or immobilized in nanoparticles, microparticles, metal particles, ceramic particles, hydrogels, and the like, for delivery to specific tissues or to ensure stability, but are not limited thereto.

The nanoparticles, microparticles, metal particles, ceramic particles, hydrogels, and the like may be biocompatible and non-toxic.

The acetylcholine receptor-binding peptides bind to acetylcholine receptors, thereby preventing the binding of acetylcholine to the receptors, and thus can inhibit the actions of the acetylcholine receptors. Preferably, the peptides can relieve wrinkles and suppress abnormal muscle contraction by inhibiting muscle contraction; and, during surgery, can secure the convenience of surgery by promoting muscle relaxation.

Furthermore, the present disclosure provides a polynucleotide encoding the acetylcholine receptor-binding peptide. A polynucleotide containing a nucleotide sequence that is homologous to the nucleotide sequence constituting the polynucleotide may also be included in the scope of the polynucleotide provided in the present disclosure as long as the polynucleotide can encode a peptide capable of exhibiting binding activity to the acetylcholine receptor. Such a polynucleotide is preferably a polynucleotide containing an amino acid sequence showing at least 80% homology, more preferably a polynucleotide containing an amino acid sequence showing at least 90% homology, and most preferably a polynucleotide containing an amino acid sequence showing at least 95% homology.

Furthermore, the present disclosure provides a cosmetic composition, for wrinkle relief, including the acetylcholine receptor peptide.

The acetylcholine receptor-binding peptide can relieve wrinkles by inhibiting the action of the acetylcholine receptor to prevent muscle contraction.

The cosmetic composition may further contain the acetylcholine receptor-binding peptide, and an adjuvant commonly used in the field of cosmetics, for example, a hydrophilic or lipophilic gelling agent, a hydrophilic or lipophilic activator, a preservative, an antioxidant, a solvent, a flavoring agent, a filler, a blocker, a pigment, a deodorant, or a dye.

The amount of the adjuvant is an amount that is commonly used in the art and, in any case, the adjuvant and the proportion thereof may be for selected so as not to adversely affect desirable properties of the cosmetic composition according to the present disclosure.

The cosmetic composition for wrinkle relief may be prepared by further containing an additive.

The additive may be a moisturizer, a functional raw material, a thickener, a softener, an emulsifier, a surfactant, a pH adjuster, and the like.

The moisturizer may include glycerin, propylene glycol, butylene glycol, hyaluronic acid, a ceramide component, and the like, but is not limited thereto.

The thickener may include a polymer, xanthan gum, and guar gum, but is not limited thereto.

The softener may include mineral oil, shea butter, or paraffin, but is not limited thereto.

The emulsifier may include dimethicone, beeswax, and the like.

The cosmetic composition for wrinkle relief may be used by mixing with a raw material having a wrinkle relief effect.

The raw material having a wrinkle relief effect may include vitamin A, a vitamin A derivative (retinyl palmitate, retinyl acetate, etc.), adenosine, and polyethoxylated retinamide, but is not limited thereto.

The cosmetic composition may be in the formulation of at least one selected from the group consisting of a lotion, a skin softener, a skin toner, an astringent, a cream, a foundation, an essence, a pack, a mask pack, a soap, a body cleanser, a cleansing foam, a body oil, and a body lotion, but is not limited thereto.

The cosmetic composition may be used every day, and may also be used even for an undetermined period, and preferably, the amount of use, the number of times of use, and the period of the cosmetic composition may be adjusted according to user's age, skin condition, or skin type.

Furthermore, the present disclosure provides a pharmaceutical composition for prevention or treatment of an acetylcholine receptor hyperactivity-associated disease, the pharmaceutical composition containing the acetylcholine receptor-binding peptide.

The pharmaceutical composition can bind to an acetylcholine receptor to inhibit the activation of the acetylcholine receptor, thereby preventing or treating the acetylcholine receptor hyperactivity-associated disease.

The acetylcholine receptor hyperactivity-associated disease refers to a disease in which the muscle contracts abnormally excessively, and examples thereof may be cervical dystonia, limb dystonia, truncal dystonia, blepharospasm, spasticity, hemifacial spasm, strabismus, nystagmus, tics, chronic pain, chronic migraine, neurogenic bladder, detrusor-sphincter dyssynergia, achalasia cardia, hyperhidrosis, neuropathic pain, skin wrinkles, square jaw and sialorrhea, pediatric cerebral palsy, post-stroke muscle stiffness, back pain, enlarged prostate, urinary incontinence, vocal cord nodules and correction, hemorrhoids, dentition, and the like.

In addition, the pharmaceutical composition can be used to secure the convenience of surgery by promoting muscle relaxation during surgery, and can be used as a therapeutic agent or adjuvant for diseases caused by nicotine addiction, used for wrinkle removal, and used for square jaw or calf correction, but is not limited thereto.

The pharmaceutical composition may contain the acetylcholine receptor-binding peptide and a pharmaceutically acceptable excipient.

The pharmaceutical composition may be formulated in the forms of: an oral formulation, such as a powder, granules, a tablet, a capsule, a suspension, an emulsion, a syrup, or an aerosol; an externally applied preparation; a suppository; and a sterile injectable solution, according to usual methods, respectively. Examples of a carrier, an excipient, and a diluent that may be contained in the pharmaceutical composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. The pharmaceutical composition may be prepared by using a diluent or an excipient that is usually used, such as a filler, an extender, a binder, a humectant, a disintegrant, or a surfactant. Solid preparations for oral administration include a tablet, a pill, a powder, granules, a capsule, and the like. These solid preparations may be prepared by mixing the acetylcholine receptor-binding peptide with at least one excipient, for example, starch, calcium carbonate, sucrose or lactose, gelatin, or the like. Also, a lubricant, such as magnesium stearate or talc, may be used in addition to simple excipients. Liquid preparations for oral administration correspond to a suspension, a liquid for internal use, an emulsion, a syrup, and the like, and may contain simple diluents that are frequently used, such as water and liquid paraffin, as well as several excipients, such as a humectant, a sweetener, a flavoring agent, and a preservative. Preparations for parenteral administration include a sterile aqueous solution, a non-aqueous solvent, a suspension, an emulsion, a freeze-drying agent, and a suppository. Examples of the non-aqueous solvent and suspension may include propylene glycol, polyethylene glycol, vegetable oils such as olive oil, injectable esters such as ethyl oleate, and the like. A base material for the suppository may include Witepsol, Macrogol, Tween 61, cocoa butter, laurin butter, glycerogelatin, and the like.

Although not particularly limited to the formulation, the pharmaceutical composition may be used as an externally-applied preparation for skin, having one formulation selected from an ointment agent, a lotion agent, a spray agent, a patch agent, a cream agent, a gel agent, and a gel. The pharmaceutical composition may contain an agent for increasing transdermal absorption, such as, but not limited to, dimethyl sulfoxide, dimethylacetamide, dimethylformamide, a surfactant, an alcohol, acetone, propylene glycol, or polyethylene glycol. The frequency of application may vary significantly depending on the age, sex, and weight of a subject to be treated, a specific disease or pathological condition to be treated, the severity of a disease or pathological condition, the route of administration, and the judgment of a prescriber. The frequency of application may range from once a month up to 10 times a day, preferably from once a week up to 4 times a day, more preferably from three times a week up to three times a day, still more preferably one or two times a day.

The pharmaceutical composition of the present disclosure may be administered to mammals, such as a rat, livestock, and a human, through various routes. All modes of administration may be expected, and for example, administration may be conducted orally, rectally, or by intravenous, intramuscular, subcutaneous, transdermal, endometrial, or intracerebrovascular injection. Preferably, administration may be conducted by transdermal injection.

Furthermore, the present disclosure is directed to a health functional food composition, containing the acetylcholine receptor-binding peptide, for alleviating an acetylcholine receptor hyperactivity-associated disease.

The health functional food composition may contain the acetylcholine receptor-binding peptide and a sitologically acceptable food supplement additive.

The health functional food composition of the present disclosure includes forms of a tablet, a capsule, a pill, a liquid preparation, and the like, and examples of foods to which peptide of the present disclosure can be added include various kinds of foods, beverages, gums, teas, vitamin complexes, and health functional foods.

In accordance with still another aspect of the present disclosure, there is provided a composition for a medicinal device, the composition containing the acetylcholine receptor-binding peptide.

The composition for a medicinal device may be a filler, but is not limited thereto.

As used herein, the term "filler" refers to a substance that can supplement skin tissues, and has the purpose of filling through injection for restoration of the resilient face, improvement of facial contour, and relief of wrinkles.

The composition for a medicinal device can relieve wrinkles by suppressing muscle contraction and can exhibit a contour improving effect, through the acetylcholine receptor-binding peptide, and microparticles, nanoparticles, and hydrogels, on which the acetylcholine receptor-binding peptide is immobilized, can be injected to fill tissue.

### Advantageous Effects of Invention

The present disclosure relates to acetylcholine receptor-binding optimized shortened peptides and uses thereof and, more specifically, to pentamers and hexamers containing arginine or lysine at both ends and having a certain amino acid sequence XYZ in the center, and further including arginine or lysine at the N or C terminal for the hexamers. Compared to existing longer peptides or existing pentamers and hexamers, the optimized shortened peptides of the present disclosure have significantly improved binding affinity for acetylcholine receptors and enhanced skin permeability. Thus, it is expected that the peptides can be developed into cosmetic compositions for wrinkle reduction, pharmaceuticals for the prevention or treatment of acetylcholine receptor-associated diseases, and health functional foods for health improvement.

### Brief Description of Drawings

FIG. 1 shows comparison of affinity for acetylcholine receptors among the precursor peptides 11mer and 8mer, and their shortened peptides 6mer, and Synake.
FIG. 2 shows alanine scanning results to identify the critical sequence of the shortened peptides 6mer and 5mer of Espep2.
FIG. 3 shows the result of binding affinity for acetylcholine receptors of the shortened peptides 6mer and 5mer derived from Espep2.
FIG. 4 shows graphs of acetylcholine receptor binding specificity of phages screened through biopanning for the optimized shortened library 6mer-1, 6mer-2, and 5mer.
FIG. 5 is a graph comparing the RU value, which is the binding affinity, between the top XYZ repeating amino acid combination and the bottom XYZ repeating amino acid combination in the 5mer library.
FIG. 6 is a graph comparing the RU value, which is the binding affinity, between the top XYZ repeating amino acid combination and the bottom XYZ repeating amino acid combination in the 6mer-1 library.
FIG. 7 is a graph comparing the RU value, which is the binding affinity, between the top XYZ repeating amino acid combination and the bottom XYZ repeating amino acid combination in the 6mer-2 library.
FIG. 8 shows the result of comparing the binding affinity for acetylcholine receptors among simple 5mer (5mer-ND) and 6mer (Espep2-6mer) derived from Espep2, the control Synake, and the optimized peptides 5mer (5mer-73 and 5mer-311) and 6mer (6mer-1-43, 6mer-1-210, 6mer-2-233, and 6mer-2-136) according to the present disclosure.
FIG. 9 shows the affinity of the 6mer-1-43 peptide according to the present disclosure for acetylcholine receptors over concentration.
FIG. 10 is a graph showing the inhibitory capacity of Synake on acetylcholine receptors.
FIG. 11 shows the inhibitory capacity of the 6mer-1-43 and 6mer-1-210 peptides according to the present disclosure on acetylcholine receptors.
FIG. 12 shows the inhibitory capacity of the 6mer-2-136 and 6mer-2-233 peptides according to the present disclosure on acetylcholine receptors.
FIG. 13 shows the inhibitory capacity of the 5mer-73 and 5mer-311 peptides according to the present disclosure on acetylcholine receptors.
FIG. 14 shows the inhibitory capacity of the palmitoylated peptide (Pal-6mer-1-43) of the 6mer-1-43 peptide according to the present disclosure on acetylcholine receptors.
FIG. 15 is the result of the inhibitory capacity of the palmitoylated 5mer-73 peptide (Pal-5mer-73) according to the present disclosure on acetylcholine receptors.
FIG. 16 shows comparison of inhibitory capacity on acetylcholine receptors between each peptide according to the present disclosure and Synake.
FIG. 17 shows results of assaying cytotoxicity of representative peptides according to the present disclosure for cytotoxicity.
FIG. 18 shows results of assaying skin permeability of the 11mer (Pal-Espep2) as the control group and the 5mer (Pal-5mer-73) according to the present disclosure.
FIG. 19 shows results of assaying clinical efficacy of the optimized short peptide (5mer-73, RRQRR) according to the present disclosure on eye skin wrinkles.
FIG. 20 shows results of assaying clinical efficacy of the optimized short peptide (5mer-73, RRQRR) according to the present disclosure on skin elasticity.
FIG. 21 shows results of assaying clinical efficacy of the optimized short peptide (5mer-73, RRQRR) according to the present disclosure on forehead wrinkle reduction.

### Best Mode for Carrying out the Invention

The present inventors previously selected acetylcholine receptor (AchR)-specific peptides with high binding affinity and specificity for AchR in the prior patent (Korean Patent No. 10-2020-0080179 A). These peptides were based on the Espep-2 peptide sequence WTWKGRKSLLR and further developed to identify crucial sequence regions important for acetylcholine receptor binding, resulting in the discovery of optimized 8mer, 11mer, and 14mer peptides with increased affinity to AchR. While these peptides demonstrated selective binding affinity to AchR through repeated biopanning, their length of 8-14mers causes problems such as the expensive production costs and difficulty in penetrating the skin. Particularly, as the peptide length increases, it not only leads to higher production costs but also negatively impacts skin penetration.

To address these issues, the present inventors have developed the invention during the process of identifying shorter peptides that retain selective binding affinity for AchR. Particularly, it was discovered that even the simply shortened 6-mer and 5-mer peptides RKSLLR and KSLLR, derived from Espep2, demonstrated superior binding affinity compared to the Synake peptide. Based on the structure of Espep2, the inventors searched for the optimal peptide structures for 6-mer and 5-mer peptides. To determine the optimized shortened peptides, a random library of 6-mer and 5-mer peptides was generated, screened, and analyzed for the peptide sequences. The results confirmed that peptides with specific amino acid sequences at certain positions exhibited higher acetylcholine receptor binding affinity, compared to the existing Espep2 (11-mer), 6-mer, and 5-mer peptides.

Hereinafter, preferable exemplary embodiments of the present disclosure will be described in detail. However, the present disclosure is not limited to the exemplary embodiments described herein and can be embodied in many different forms. Rather, these exemplary embodiments are provided so that the present disclosure will be thorough and complete and will fully convey the scope of the disclosure to those skilled in the art.

### <EXAMPLE 1: Affinity of Shortened Peptides for Construction of Optimized Shortened Library>

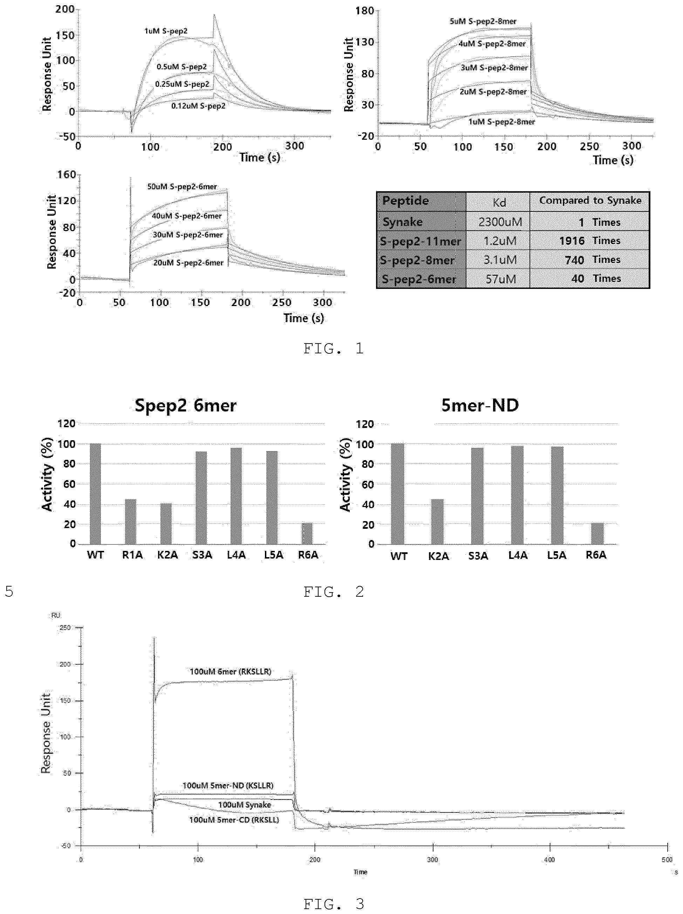

To construct an optimized shortened library from the previous Espep2 peptide, assessment was made of the affinity of peptides having sequences sequentially removed from the N- and C-terminals. Initially, surface plasmon resonance (SPR) experiments were conducted using a biosensor chip (Biacore3000, Biacore AB, Uppsala, Sweden). Selected acetylcholine receptor proteins were immobilized on a CM5 chip (Biacore) using EDC/NHS and observed for association and dissociation for up to 500 seconds. The observation conditions were set forth as follows: running buffer 20mM Tris (pH 7.4), rate 30ul/min, concentration 20 µM (peptide), and the results are depicted in FIG. 1.

As shown in FIG. 1, the affinity for acetylcholine receptor of the derivatives with reduced size by removing amino acids from the N and C terminals was 1.2 µM for 11mer (Espep2-11mer, WTWKGRKSLLR), 3.1 µM for 8mer (Espep2-8mer, KGRKSLLR), and 57 µM for 6mer (Espep2-6mer, RKSLLR), indicating higher affinity by 1916 times for the 11mer, 740 times for the 8mer, and 40 times for the 6mer, compared to Synake.

Synake, a snake venom-derived peptide, inhibits the binding of acetylcholine to its receptors, thus relieving wrinkles. The high affinity of the shortened Espep-2 peptides (11mer, 8mer, 6mer) for acetylcholine receptor protein, as seen in Figure 1, demonstrated superior performance compared to Synake, indicating their potential not only in cosmetic compositions for wrinkle relief, but also in preventing or treating diseases related to acetylcholine receptor hyperactivity by inhibiting the acetylcholine receptor.

Despite the excellent medical utility of botulinum toxin used in treating various diseases caused by muscle spasms and hyperactivity due to acetylcholine receptor hyperactivity, it possesses a highly toxic nature, capable of causing death to about a million people with just 1g, and is regulated under the Biological Weapons Convention. In consideration of this fact, short peptides were found to achieve high acetylcholine receptor inhibitory efficacy.

### <EXAMPLE 2: Alanine Scanning and Affinity of Shortened Peptides to Confirm Core Sequence>

To identify the crucial sequences in the shortened peptides, alanine scanning was conducted. Wild-type (WT) Espep2-6mer (RKSLLR) and Espep2-5mer (KSLLR) peptides were synthesized with each amino acid sequentially substituted with alanine (Ala, A), and their affinity was measured using the same method as in Example 1. The measurements are depicted in FIG. 2 after normalization to WT.

As shown in FIG. 2, it was found that positively charged amino acids near the N and C terminals are important for binding.

Moreover, among the six amino acids in the RKSLLR sequence, R residues at the N and C terminals are crucial. Based on these results, 5mer-ND (KSLLR) and 5mer-CD (RKSLL) peptides were synthesized by removing one sequence from each end of the N and C terminals of the 6mer, and their respective affinities were measured and the measurements are presented in FIG. 3.

As shown in FIG. 3, Espep2 6mer had an RU value of 177, indicating higher affinity than that of Synake, and the shortened 5mer-ND showed higher binding affinity than Synake. In the case of 5mer-CD, it exhibited slightly lower binding affinity compared to Synake. Thus, it was found the structure of the 5mer shortened peptide, including lysine (K) or arginine (R) at both ends, is advantageous for acetylcholine receptor binding.

### <EXAMPLE 3: Construction of Library of Shortened, 6mer-1, 6mer-2, 5mer-ND>

Based on the important sequences identified through alanine scanning in Example 2, optimized libraries were constructed for 6mer-1; (K/R)(K/R)XXX(K/R), 6mer-2; (K/R)XXX(K/R)(K/R), and 5mer-ND; (K/R)XXX(K/R) [(K/R) represents either K or R and X is a random amino acid].

### Example 3-1: Construction of Optimized Shortened Library Vectors

To construct a 6mer optimized peptide library, DNA sequences for 6mer-1-F (SfiI_AR_AARANNKNNKNNKARA_NotI), 6mer-2-F (SfiI_ARANNKNNKNNKARAARA_NotI), 5mer-ND-F (SfiI_ARANNKNNKNNKARA_NotI), and Back (AACAGTTTCTGCGGCCGC) (where N is A, T, G or C; K is G or T; and M is C or A) were synthesized (Bioneer, Daejeon, Korea). With the synthesized DNA library of Table 1 serving as templates, 5mer and 6mer inserts were subjected to PCR (94°C for 5 min, 60 cycles of 40°C for 30 sec and 72°C for 30 sec, and 72°C for 7 min) to generate double-stranded products, followed by purification (PCR purification kit, GeneAll, Seoul, Korea) to obtain the library genes. The library genes were inserted into a phagemid vector (pIGT). In this regard, the phagemid vector and insert DNA were treated with restriction enzymes. About 10 µg of insert DNA was reacted with SfiI (New England Biolabs (NEB, Ipswich)) and NotI (NEB, Ipswich) for 8 hours, and then the purified DNA was obtained using the PCR purification kit. Similarly, about 10 µg of phagemid vector was treated with SfiI and NotI for 8 hours, followed by reaction with CIAP (Calf Intestinal Alkaline Phosphatase) (NEB, Ipswich) for 1 hour and then purification by the PCR purification kit. The insert DNA was ligated to the phagemid vector using T4 DNA ligase (Bioneer, Daejeon, Korea) for 15 hours at 18°C and then precipitated with ethanol, and the DNA was dissolved in 100 µL of TE buffer.

**TABLE 1**

| Library | Amino acid sequence | Base sequence |
|---|---|---|
| 6mer-1 | (K or R)-(K or R)-XXX-(K or R), X=random amino acids | TTCTATGCGGCCCAGCTGGCCARAARANNKN NKNNKARAGCGGCCGCAGAAACTGTT |
| 6mer-2 | (K or R)-XXX-(K or R)-(K or R), X=random amino acids | TTCTATGCGGCCCAGCTGGCCARANNKNNKN NKARAARAGCGGCCGCAGAAACTGTT |
| 5mer-ND | (K or R)-XXX-(K or R), X=random amino acids | TTCTATGCGGCCCAGCTGGCCARANNKNNKN NKARAGCGGCCGCAGAAACTGTT |
| N : A or C or G or T | | |
| K : G or T | | |
| R : A or G | | |
| X : Random amino acids | | |

### EXAMPLE 3-2. Electroporation

The phagemid vector (10 µg) and 6mer or 5mer random insert DNA (3 µg) were ligated in 100 µL which was then divided into 10 parts, followed by electroporation. Competent cells were thawed on ice, and 200µL of competent cells were mixed with 4µE of the ligated solution, cooled, and placed in a prepared 0.2 cm cuvette which was then left on ice for 1 minute. After an electroporator (BioRAD, Hercules, Ca) was programmed under conditions of 25 µF and 2.5 kV at 200 Q, the prepared cuvette was dried and placed in the electroporator and a pulse was applied thereto. The time constant was 4.5-5 msec. Immediately thereafter, the cells were transferred to 1mL of LB broth containing 20mM glucose prewarmed to 37°C, and the resulting 25mL of cells were transferred to a 100-mL tube and then cultured at 37°C at 200rpm for one hour. Of the cell culture, 10µL was taken, diluted, and spread on an ampicillin agar plate to count the library. The remaining cells were cultured overnight in 1 L of LB with 20mM glucose and 50pg/mL ampicillin at 30°C. After centrifugation for 20 minutes at 4,000g and 4°C, the supernatant was discarded and the cell pellet was resuspended in 40 mL of LB. Then, glycerol was added to a final concentration of more than 20%, and stored at - 80°C.

### Example 3-3: Production of Recombinant Phage from Optimized Peptide Library

Recombinant phages were produced using the 6mer and 5mer optimized peptide library stored at -80°C. To 30 mL of SB broth was added 1 mL of the library stored at -80°C, followed by incubation at 37°C and 200 rpm for 20 minutes. Helper phage (1010 pfu) and ampicillin (final concentration 50 µg/mL) were added and cultured again under the same conditions for an hour. Then, the phages were transferred to 30mL of SB broth containing ampicillin (50pg/mL) and kanamycin (10µg/mL) and cultured under the same conditions for over 16 hours to produce recombinant phages. The culture was centrifuged at 5,000rpm for 10 minutes at 4°C, and the supernatant was mixed with PEG/NaCl in a 5:1 ratio, left on ice for 1 hour, then centrifuged at 13,000rpm for 20 minutes at 4°C. The supernatant was carefully discarded and the pellet was resuspended in 1 mL of PBS.

To ensure that all possible amino acid sequences were stochastically included in the library, the completed library number should be at least 3.2×104 for the 5mer optimized library, calculated as 2×2×20×20×20. Therefore, the 5mer library was constructed with a count of 1.95×106 in this study. For the 6mer optimized library, the number should be at least 6.4×104, calculated as 2×2×2×20×20×20. In this study, 1.23×106 and 1.53×106 libraries were obtained for 6mer-1 and 6mer-2, respectively, confirming successful library development. These results are summarized in Table 2 below. Additionally, partial sequencing of each library confirmed no errors in the designated sequence, and the results are given in Table 3.

**TABLE 2**

| | Name | Sequence | Sequence and No. of optimized library |
|---|---|---|---|
| 1 | Espep 2_6mer-1 | RKSLLR | (R/K) (R/K) XXX (R/K) 1.23×10 6 |
| 2 | Espep 2_6mer-2 | KSLLR (R/K) | (R/K) XXX (R/K) (R/K) 1.53×10 6 |
| 3 | Espep 2_5mer (ND) | KSLLR | (R/K)XXX(R/K) 1.95×10 6 |

**TABLE 3**

| No. | Name | Sequence |
|---|---|---|
| 1 | 5mer-ND-1 | AAQLAKGCCRAAAEQKLISEEDLSR@DDDD |
| 2 | 5mer-ND-2 | AAQLARWRRAAAEQKLISEEDLSR@DDDD |
| 3 | 5mer-ND-3 | AAQLAKWILKAAAEQKLISEEDLSR@DDDD |
| 4 | 5mer-ND-4 | AAQLAKSTCKAAAEQKLISEEDLSR@DDDD |
| 5 | 5mer-ND-5 | AAQLARSVQRAAAEQKLISEEDLSR@DDDD |
| 6 | 5mer-ND-6 | AAQLAKAQQRAAAEQKLISEEDLSR@DDDD |
| 7 | 5mer-ND-7 | AAQLARGLWKAAAEQKLISEEDLSR@DDDD |
| 8 | 5mer-ND-8 | AAQLARTDERAAAEQKLISEEDLSR@DDDD |
| 9 | 5mer-ND-9 | AAQLAKCYARAAAEQKLISEEDLSR@DDDD |
| 10 | 5mer-ND-10 | AAQLARKGTRAAAEQKLISEEDLSR@DDDD |
| 11 | 6mer-1-1 | AAQLARRGRGRAAAEQKLISEEDLSR@DDDD |
| 12 | 6mer-1-2 | AAQLARKALLRAAAEQKLISEEDLSR@DDDD |
| 13 | 6mer-1-3 | AAQLAKRQCSKAAAEQKLISEEDLSR@DDDD |
| 14 | 6mer-1-4 | AAQLARRCSRRAAAEQKLISEEDLSR@DDDD |
| 15 | 6mer-1-5 | AAQLARRRGYRAAAEQKLISEEDLSR@DDDD |
| 16 | 6mer-1-6 | AAQLAKRCVRRAAAEQKLISEEDLSR@DDDD |
| 17 | 6mer-1-7 | AAQLARKQLGKAAAEQKLISEEDLSR@DDDD |
| 18 | 6mer-1-8 | AAQLAKKSTTRAAAEQKLISEEDLSR@DDDD |
| 19 | 6mer-1-9 | AAQLARRQAQKAAAEQKLISEEDLSR@DDDD |
| 20 | 6mer-1-10 | AAQLAKRVSQRAAAEQKLISEEDLSR@DDDD |
| 21 | 6mer-2-1 | AAQLAKQLRKKAAAEQKLISEEDLSR@DDDD |
| 22 | 6mer-2-2 | AAQLAKRQLKRAAAEQKLISEEDLSR@DDDD |
| 23 | 6mer-2-3 | AAQLAKLSLRRAAAEQKLISEEDLSR@DDDD |
| 24 | 6mer-2-4 | AAQLARLVSRRAAAEQKLISEEDLSR@DDDD |
| 25 | 6mer-2-5 | AAQLAKVQLKKAAAEQKLISEEDLSR@DDDD |
| 26 | 6mer-2-6 | AAQLAKRFQKKAAAEQKLISEEDLSR@DDDD |
| 27 | 6mer-2-7 | AAQLAKSFRRKAAAEQKLISEEDLSR@DDDD |
| 28 | 6mer-2-8 | AAQLARVFSKKAAAEQKLISEEDLSR@DDDD |
| 29 | 6mer-2-9 | AAQLAKGLLKKAAAEQKLISEEDLSR@DDDD |
| 30 | 6mer-2-10 | AAQLAKSYYKRAAAEQKLISEEDLSR@DDDD |

### <EXAMPLE 4: Biopanning and Screening of Shortened Peptide Library>

### Example 4-1: Biopanning

A procedure in which immobilized antigens were treated with a phage library surface-expressing antibodies to thereby antibody candidates binding to the antigens is called biopanning, and the biopanning is composed of three steps; binding/washing/elution. The phages having antibodies with weak binding affinity were removed during a washing step, and resultantly, only phages expressing antibodies with strong binding affinity remained. This procedure can be repeated to discover antibody candidates with excellent antigen binding affinity and specificity. Therefore, biopanning was used to screen acetylcholine receptor-binding peptides with excellent binding affinity and specificity to acetylcholine receptors.

In eight wells of a 96-well plate, 5 µg/ml AchR αl was placed at 50 µl per well, and then left overnight at 4°C. Next day, the wells were washed once with 200 ul of Tris (20 mM, pH 7), followed by the addition of 200 ul of 2% bovine serum albumin (BSA), and then blocked at room temperature for 2 hours. Then, the solution was all discarded, and the wells were washed three times with 200 ul of Tris (20 mM, pH 7). After 400 µl of the random peptide recombinant phages (input phages) suspended in PBS in Example 3-3 was mixed with 400 ul of 2% BSA, the mixture was placed at 100 µl per well and then left at 30°C for 1 hour. The solution in the wells was all removed, and the wells were washed three times with Tris (20 mM, pH 7). Thereafter, 0.2 M glycine (pH 2.2) was placed at 100 µl per well, and the phages were isolated for 20 minutes, and then, the phages were collected in one tube and added with 200 ul of 1 M Tris (pH 9.0) to obtain output phages.

To repeat biopanning, 500 ul of the isolated phages were mixed with 5 ml of E. coli, followed by incubation at 37°C and 200 rpm for 30 minutes. Then, 1×1010 pfu helper phages and ampicillin were added to a final concentration of 50 µg/ml before additional incubation for 30 minutes. The culture was transferred to SB broth containing 50 µg/ml ampicillin and 10 µg/ml kanamycin, and cultured overnight in the same conditions, thereby reproducing random peptide recombinant phages. The reproduced random peptide recombinant phages were centrifuged at 4°C and 5,000 rpm for 10 minutes. The supernatant thus obtained and PEG/NaCl were mixed at 5:1 (v:v), left on ice for 1 hour, and centrifuged at 4°C and 13,000 rpm for 20 minutes. The supernatant was discarded while the precipitate was suspended in 1 ml of phosphate buffered saline (PBS), and used for a subsequent round of biopanning.

Random recombinant phages were reproduced in the same manner as in the foregoing, with the exception that the washing was 3, 3, 4, 4, 5, and 6 times repeated (0.05% PBST) as the round of biopanning increased.

To measure the number of input phages and output phages for each biopanning, the phages were mixed with E. coli having an absorbance at 600nm of 0.7 (OD600=0.7), and plated on agar plates containing ampicillin. The results are summarized in Tables 4-6 below.

**TABLE 4**

| 6mer-1 Biopanning | | | | |
|---|---|---|---|---|
| Roun d | No. of wash | Input phage | Output phage | Input/out put |
| 1st | 3 | 9.6 × 1010 | 2.3 × 107 | 23.9 × 10-5 |
| 2nd | 3 | 1.98 × 1012 | 1.98 × 108 | 10 × 10-5 |
| 3rd | 4 | 1.63 × 1012 | 5.8 × 107 | 3.55 × 10-5 |
| 4th | 4 | 3.77 × 1012 | 5.28 × 108 | 14 × 10-5 |
| 5th | 5 | 4.07 × 1012 | 7.2 × 108 | 17.6 × 10-5 |
| 6th | 6 | 3.13 × 1012 | 6.4 × 108 | 20.4 × 10-5 |

**TABLE 5**

| 6mer-2 Biopanning | | | | |
|---|---|---|---|---|
| Round | No. of wash | Input phage | Output phage | Input/output |
| 1st | 3 | 4.57 × 1011 | 1.45 × 108 | 31.7 × 10-5 |
| 2nd | 3 | 2.17 × 1012 | 5.23 × 108 | 24.1 × 10-5 |
| 3rd | 4 | 0.61 × 1012 | 5.1 × 107 | 8.36 × 10-5 |
| 4th | 4 | 5.21 × 1012 | 7.7 × 108 | 14.7 × 10-5 |
| 5th | 5 | 4.01 × 1012 | 6.27 × 108 | 15.6 × 10-5 |
| 6th | 6 | 3.87 × 1012 | 6.51 × 108 | 16.82 × 10-5 |

**TABLE 6**

| 5mer-ND Biopanning | | | | |
|---|---|---|---|---|
| Round | No. of wash | Input phage | Output phage | Input/output |
| 1st | 3 | 8.88 × 1011 | 3 × 108 | 33.7 × 10-5 |
| 2nd | 3 | 5.36 × 1011 | 1.09 × 108 | 20.33 × 10-5 |
| 3rd | 4 | 1.58 × 1012 | 9 × 106 | 0.56 × 10-5 |
| 4th | 4 | 3.55 × 1012 | 1.23 × 109 | 34.64 × 10-5 |
| 5th | 5 | 3.12 × 1012 | 1.54 × 109 | 49.35 × 10-5 |
| 6th | 6 | 2.83 × 1012 | 1.25 × 109 | 44.16 × 10-5 |

### Example 4-2. Enzyme-Linked Immunosorbent Assay (ELISA) Using Random Peptide Library Input Recombinant Phages

ELISA was performed on bovine serum albumin (BSA) and AchR using the input phages for each round of biopanning in Example 4-1.

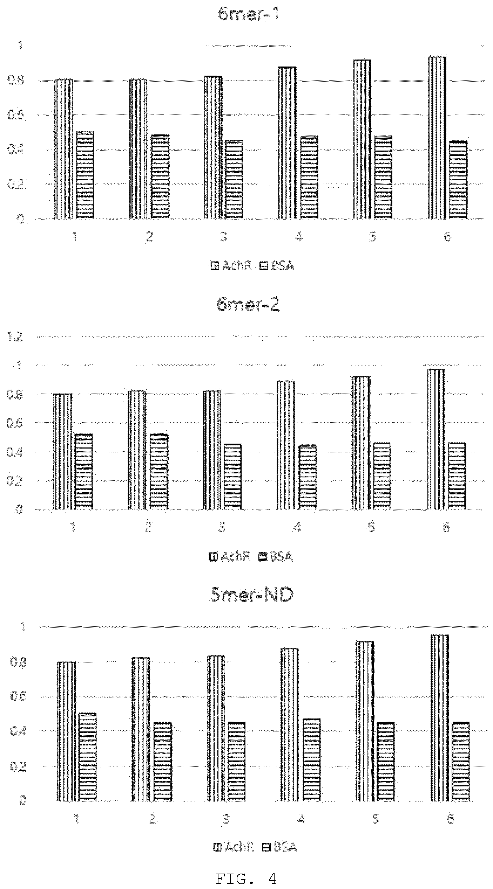

In a 96-well ELISA plate, 10 µg/ml AchR or BSA was added at 50 µl per well, and left overnight at 4°C. The next day, the wells were washed thrice with Tris (20 mM, pH7), followed by the addition of 2% BSA diluted with PBS, and then blocked at room temperature for 2 hours. After the solution was discarded, the wells were washed thrice with Tris (20 mM, pH7). After 800 µl of the input pages for each of 1st, 2nd, 3rd, 4th, 5th, and 6th rounds in Tables 4 to 6 in Example 4-1 were mixed with 200 µl of 10% BSA, the mixture was placed at 100 µl per well and then left at 30°C for 1 hour. The solution in the wells was all removed, and the wells were washed thrice with Tris (20 mM, pH 7). Then, horseradish peroxidase (HRP)-conjugated anti-M13 Ab (GE Healthcare) diluted 1:1,000 was added at 100 µl per well, followed by incubation at 30°C for 1 hour. After the wells were washed thrice with Tris (20 mM, pH 7), 100 µl of a tetramethylbenzidine (TMB) solution, which is a substrate of HRP, was added to each well to induce a color development reaction, and then the reaction was stopped by the addition of 100 µl of 1M HCl, and the absorbance (OD450) was measured at 450 nm. The results are depicted in FIG. 4. As shown in FIG. 4, with the increasing of the round of biopanning, the OD value of acetylcholine/BSA increases, indicating that phages with high specificity for the acetylcholine receptor were successfully screened.

### <EXAMPLE 5. Analysis of Amino Acid X in Each Library Peptide>

Following biopanning, an analysis was conducted to determine if specific amino acids, associated with the random sequence XXX in the middle part of the peptide sequence from each library, could be identified as having high binding affinity for the acetylcholine receptor. The sequences of phages from the 6th round of biopanning, which exhibited high specificity from the libraries in Example 4, were analyzed. It was noted that both 5mer and 6mer peptides showed a high frequency of certain amino acids. Labeling the XXX portion sequentially as XYZ, the individual occurrences of amino acids at the positions of X, Y, and Z for peptides with high repetition are presented in Table 7.

**TABLE 7**

| X position | No. of repetition | Y position | No. of repetition | Z position | No. of repetition |
|---|---|---|---|---|---|
| R | 189 | R | 179 | R | 182 |
| Q | 151 | Q | 111 | S | 117 |
| G | 109 | L | 107 | L | 104 |
| V | 96 | I | 96 | C | 78 |
| L | 87 | F | 86 | Y | 65 |
| S | 75 | V | 75 | Q | 64 |
| W | 74 | Y | 75 | T | 61 |
| F | 23 | P | 24 | W | 24 |
| T | 12 | G | 13 | G | 13 |
| M | 12 | W | 13 | V | 13 |
| Y | 11 | H | 12 | I | 13 |
| A | 11 | C | 12 | P | 13 |
| C | 11 | T | 11 | K | 12 |
| N | 11 | M | 11 | F | 12 |
| Y | 11 | S | 11 | H | 12 |
| K | 11 | D | 11 | A | 11 |
| | | A | 11 | N | 11 |
| | | | | D | 11 |

Specifically, amino acids found to occur much more frequently than other amino acids were: seven amino acids R, Q, G, V, L, S, and W for the position X; seven amino acids R, Q, L, I, F, V, and Y for position Y; and seven amino acids R, S, L, C, Q, T, and Y for position Z.

### <EXAMPLE 6: Binding Affinity Analysis of Peptides Composed of Top 7 Amino Acids in Repeated Sequence of XYZ>

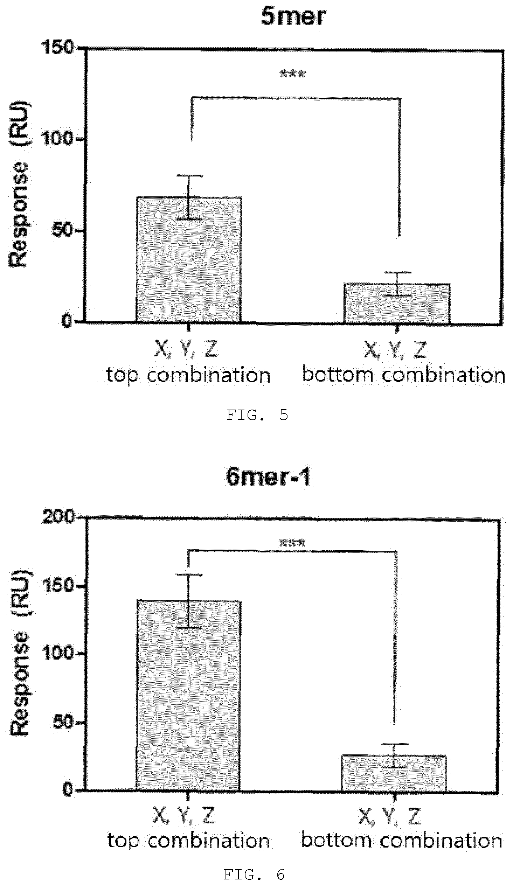

Based on the frequencies of occurrence of the top 7 amino acids from Table 7 in Example 5, 600 peptides comprising these amino acids (top combinations) and those comprising the 13 less frequently occurring amino acids (bottom combinations) were synthesized for each series of 5mer, 6mer-1, and 6mer-2. The binding affinity of each sequence for the acetylcholine receptor was measured using Surface Plasmon Resonance (SPR) analysis method. The binding affinity was measured in terms of Resonance Units (Ru), and the results are summarized in Tables 8-13. The peptides were tested at a concentration of 10µM. Additionally, the average binding affinities of the top and bottom combinations in each series are depicted in FIGS. 5-7.

**TABLE 8**

| 5mer Top sequence | | | | | | | |
|---|---|---|---|---|---|---|---|
| SEQ ID NO: | Unique No. | Sequence | Binding affinity | SEQ ID NO: | Unique No. | Sequence | Binding affinity |
| 1 | 5mer-1 | RVQCK | 54 | 301 | 5mer-301 | KWIRK | 64 |
| 2 | 5mer-2 | KRISR | 80 | 302 | 5mer-302 | RRILK | 70 |
| 3 | 5mer-3 | KRVLK | 66 | 303 | 5mer-303 | KGRYR | 87 |
| 4 | 5mer-4 | KRFLR | 58 | 304 | 5mer-304 | RSVRK | 76 |
| 5 | 5mer-5 | KLFLR | 54 | 305 | 5mer-305 | KRICK | 72 |
| 6 | 5mer-6 | RWQQK | 61 | 306 | 5mer-306 | KSYYR | 74 |
| 7 | 5mer-7 | RQLTK | 54 | 307 | 5mer-307 | KQRCK | 82 |
| 8 | 5mer-8 | RQRSR | 51 | 308 | 5mer-308 | RLRCR | 84 |
| 9 | 5mer-9 | RGVQR | 77 | 309 | 5mer-309 | KVQYR | 70 |
| 10 | 5mer-10 | RVQRR | 55 | 310 | 5mer-310 | KSRQR | 78 |
| 11 | 5mer-11 | KQRLR | 84 | 311 | 5mer-311 | RSYSR | 167 |
| 12 | 5mer-12 | RVFLR | 70 | 312 | 5mer-312 | KRYCR | 81 |
| 13 | 5mer-13 | RLISK | 52 | 313 | 5mer-313 | RSQLK | 75 |
| 14 | 5mer-14 | RWLCK | 63 | 314 | 5mer-314 | RWYTR | 80 |
| 15 | 5mer-15 | KGVCK | 75 | 315 | 5mer-315 | KLFSR | 77 |
| 16 | 5mer-16 | KLYTK | 66 | 316 | 5mer-316 | KQYLR | 73 |
| 17 | 5mer-17 | KRIYK | 70 | 317 | 5mer-317 | RRVQR | 86 |
| 18 | 5mer-18 | RQLRR | 69 | 318 | 5mer-318 | KRVCK | 64 |
| 19 | 5mer-19 | KRFQR | 72 | 319 | 5mer-319 | RVQTR | 74 |
| 20 | 5mer-20 | RGRYK | 78 | 320 | 5mer-320 | RVVQR | 75 |
| 21 | 5mer-21 | KWFTR | 63 | 321 | 5mer-321 | RSLSR | 68 |
| 22 | 5mer-22 | RQQLK | 66 | 322 | 5mer-322 | RRQSR | 84 |
| 23 | 5mer-23 | KWLYK | 86 | 323 | 5mer-323 | KWYYR | 77 |
| 24 | 5mer-24 | KSQCR | 67 | 324 | 5mer-324 | KSQRR | 58 |
| 25 | 5mer-25 | KVVYK | 60 | 325 | 5mer-325 | KRIQR | 50 |
| 26 | 5mer-26 | RQQQR | 65 | 326 | 5mer-326 | RRFTK | 68 |
| 27 | 5mer-27 | KGYLR | 67 | 327 | 5mer-327 | RVLCR | 86 |
| 28 | 5mer-28 | RLLSR | 74 | 328 | 5mer-328 | RWVLK | 61 |
| 29 | 5mer-29 | KGFCR | 63 | 329 | 5mer-329 | RRFCR | 79 |
| 30 | 5mer-30 | KSQLR | 70 | 330 | 5mer-330 | RWQLR | 86 |
| 31 | 5mer-31 | RRLQR | 65 | 331 | 5mer-331 | RGIQR | 79 |
| 32 | 5mer-32 | RQLSR | 75 | 332 | 5mer-332 | RRYCR | 85 |
| 33 | 5mer-33 | RRISK | 59 | 333 | 5mer-333 | RVLQR | 67 |
| 34 | 5mer-34 | KRIRK | 82 | 334 | 5mer-334 | KRFYK | 64 |
| 35 | 5mer-35 | RWQYK | 80 | 335 | 5mer-335 | KSLTR | 56 |
| 36 | 5mer-36 | RRLQK | 60 | 336 | 5mer-336 | KSVSR | 83 |
| 37 | 5mer-37 | RRLSR | 72 | 337 | 5mer-337 | KGRSR | 67 |
| 38 | 5mer-38 | RGYCK | 53 | 338 | 5mer-338 | RWILR | 61 |
| 39 | 5mer-39 | KQVSR | 67 | 339 | 5mer-339 | KWVRK | 87 |
| 40 | 5mer-40 | KSVCR | 67 | 340 | 5mer-340 | KGYRR | 51 |
| 41 | 5mer-41 | KVISK | 82 | 341 | 5mer-341 | RWYRR | 52 |
| 42 | 5mer-42 | RQRLR | 83 | 342 | 5mer-342 | KVYQR | 69 |
| 43 | 5mer-43 | KSFYR | 53 | 343 | 5mer-343 | KSFSR | 60 |
| 44 | 5mer-44 | RSRTR | 59 | 344 | 5mer-344 | KQFQR | 52 |
| 45 | 5mer-45 | RVFQR | 86 | 345 | 5mer-345 | RQQRR | 86 |
| 46 | 5mer-46 | KRQCK | 76 | 346 | 5mer-346 | KGISK | 57 |
| 47 | 5mer-47 | KGVRR | 70 | 347 | 5mer-347 | KRICR | 78 |
| 48 | 5mer-48 | RVYLR | 62 | 348 | 5mer-348 | KRQTK | 80 |
| 49 | 5mer-49 | KWRTR | 62 | 349 | 5mer-349 | KQVYR | 50 |
| 50 | 5mer-50 | KLLCR | 63 | 350 | 5mer-350 | KSYYK | 59 |
| 51 | 5mer-51 | RSLQR | 70 | 351 | 5mer-351 | KSFSK | 75 |
| 52 | 5mer-52 | RQQYK | 50 | 352 | 5mer-352 | RLVRK | 76 |
| 53 | 5mer-53 | RLVTR | 50 | 353 | 5mer-353 | KSQLK | 59 |
| 54 | 5mer-54 | KWLQR | 66 | 354 | 5mer-354 | RSQTR | 64 |
| 55 | 5mer-55 | RRFSR | 58 | 355 | 5mer-355 | KLLYR | 87 |
| 56 | 5mer-56 | KRFCK | 59 | 356 | 5mer-356 | KQVLR | 65 |
| 57 | 5mer-57 | KQLYR | 73 | 357 | 5mer-357 | RGFRK | 54 |
| 58 | 5mer-58 | KWVSK | 75 | 358 | 5mer-358 | KVRSK | 50 |
| 59 | 5mer-59 | KLQYR | 68 | 359 | 5mer-359 | KRFQK | 79 |
| 60 | 5mer-60 | RLFTK | 86 | 360 | 5mer-360 | RQLCK | 66 |
| 61 | 5mer-61 | RLILK | 66 | 361 | 5mer-361 | RSYQR | 58 |
| 62 | 5mer-62 | KQLTK | 70 | 362 | 5mer-362 | KQRLK | 62 |
| 63 | 5mer-63 | RLLRK | 86 | 363 | 5mer-363 | KRVRK | 59 |
| 64 | 5mer-64 | KVVTR | 76 | 364 | 5mer-364 | RWITR | 62 |
| 65 | 5mer-65 | RQIRR | 67 | 365 | 5mer-365 | KRRYK | 52 |
| 66 | 5mer-66 | KRLLR | 80 | 366 | 5mer-366 | RQLQK | 52 |
| 67 | 5mer-67 | RQLYR | 86 | 367 | 5mer-367 | RSVQK | 71 |
| 68 | 5mer-68 | RGFYK | 82 | 368 | 5mer-368 | KRIRR | 50 |
| 69 | 5mer-69 | RSRSR | 50 | 369 | 5mer-369 | RGFTR | 84 |
| 70 | 5mer-70 | RLLQR | 69 | 370 | 5mer-370 | KWRRK | 67 |
| 71 | 5mer-71 | KLQSK | 68 | 371 | 5mer-371 | RGISK | 86 |
| 72 | 5mer-72 | KLYRR | 50 | 372 | 5mer-372 | RSVCR | 54 |
| 73 | 5mer-73 | RRQRR | 179 | 373 | 5mer-373 | KVFTK | 87 |
| 74 | 5mer-74 | RQITK | 87 | 374 | 5mer-374 | KLFTR | 75 |
| 75 | 5mer-75 | KVQTK | 63 | 375 | 5mer-375 | KQLCR | 84 |
| 76 | 5mer-76 | RGYRR | 80 | 376 | 5mer-376 | KGQLR | 65 |
| 77 | 5mer-77 | KWRQK | 74 | 377 | 5mer-377 | KGFYK | 67 |
| 78 | 5mer-78 | KVICK | 79 | 378 | 5mer-378 | RRLSK | 65 |
| 79 | 5mer-79 | RWFLR | 82 | 379 | 5mer-379 | KSRLR | 79 |
| 80 | 5mer-80 | KSRTR | 83 | 380 | 5mer-380 | KRYLK | 82 |
| 81 | 5mer-81 | RGIYR | 79 | 381 | 5mer-381 | KGQRR | 87 |
| 82 | 5mer-82 | RQRYK | 84 | 382 | 5mer-382 | RLRQR | 62 |
| 83 | 5mer-83 | KGFSR | 64 | 383 | 5mer-383 | RWQRK | 56 |
| 84 | 5mer-84 | RVVRR | 67 | 384 | 5mer-384 | KSLRK | 75 |
| 85 | 5mer-85 | KGVLK | 74 | 385 | 5mer-385 | RGVYR | 51 |
| 86 | 5mer-86 | RRLCK | 55 | 386 | 5mer-386 | RSFLK | 78 |
| 87 | 5mer-87 | RQITR | 55 | 387 | 5mer-387 | RRILR | 52 |
| 88 | 5mer-88 | RRVQK | 73 | 388 | 5mer-388 | KLFCK | 78 |
| 89 | 5mer-89 | KRITK | 77 | 389 | 5mer-389 | KWRRR | 57 |
| 90 | 5mer-90 | RWYTK | 86 | 390 | 5mer-390 | KRFCR | 64 |
| 91 | 5mer-91 | RQIQK | 55 | 391 | 5mer-391 | KVFRR | 72 |
| 92 | 5mer-92 | RVRLK | 62 | 392 | 5mer-392 | RSRLR | 87 |
| 93 | 5mer-93 | KWVYR | 79 | 393 | 5mer-393 | KSLQR | 75 |
| 94 | 5mer-94 | RWFQR | 50 | 394 | 5mer-394 | KRRRK | 66 |
| 95 | 5mer-95 | RGRRK | 68 | 395 | 5mer-395 | RWVCK | 50 |
| 96 | 5mer-96 | RVLYR | 55 | 396 | 5mer-396 | KVVCR | 53 |
| 97 | 5mer-97 | KRQLR | 52 | 397 | 5mer-397 | RRITK | 52 |
| 98 | 5mer-98 | KWYCK | 68 | 398 | 5mer-398 | KGQCR | 71 |
| 99 | 5mer-99 | RWICK | 58 | 399 | 5mer-399 | KWIQR | 62 |
| 100 | 5mer-100 | KVIRK | 80 | 400 | 5mer-400 | RWRCK | 70 |
| 101 | 5mer-101 | RQQYR | 55 | 401 | 5mer-401 | RGVLR | 52 |
| 102 | 5mer-102 | RGFSK | 82 | 402 | 5mer-402 | RLRYR | 86 |
| 103 | 5mer-103 | RWFLK | 62 | 403 | 5mer-403 | RRLYK | 64 |
| 104 | 5mer-104 | RVVLK | 74 | 404 | 5mer-404 | KGYLK | 83 |
| 105 | 5mer-105 | RGQQR | 50 | 405 | 5mer-405 | KSYTK | 68 |
| 106 | 5mer-106 | KLLQR | 78 | 406 | 5mer-406 | KVYLR | 84 |
| 107 | 5mer-107 | RWVSR | 56 | 407 | 5mer-407 | RQIYK | 58 |
| 108 | 5mer-108 | KWQLK | 77 | 408 | 5mer-408 | KVFRK | 74 |
| 109 | 5mer-109 | RLQCR | 68 | 409 | 5mer-409 | KQIYK | 62 |
| 110 | 5mer-110 | KRRLK | 76 | 410 | 5mer-410 | RRFLR | 76 |
| 111 | 5mer-111 | KSVLK | 85 | 411 | 5mer-411 | KGRLR | 66 |
| 112 | 5mer-112 | KRYYR | 58 | 412 | 5mer-412 | RGRSR | 81 |
| 113 | 5mer-113 | KRLRK | 79 | 413 | 5mer-413 | RWLTR | 56 |
| 114 | 5mer-114 | KRQRR | 82 | 414 | 5mer-414 | KVRCK | 71 |
| 115 | 5mer-115 | RWLLR | 75 | 415 | 5mer-415 | KVFLR | 77 |
| 116 | 5mer-116 | RWRSK | 64 | 416 | 5mer-416 | KLYLR | 67 |
| 117 | 5mer-117 | RSVYK | 69 | 417 | 5mer-417 | KWLQK | 65 |
| 118 | 5mer-118 | RVICK | 87 | 418 | 5mer-418 | RWRTR | 53 |
| 119 | 5mer-119 | KQIRK | 60 | 419 | 5mer-419 | RVYTR | 67 |
| 120 | 5mer-120 | RQILK | 68 | 420 | 5mer-420 | KLFCR | 84 |
| 121 | 5mer-121 | RQVTR | 73 | 421 | 5mer-421 | RVVYK | 68 |
| 122 | 5mer-122 | RSYQK | 78 | 422 | 5mer-422 | KWICK | 87 |
| 123 | 5mer-123 | KWIRR | 53 | 423 | 5mer-423 | RRQLK | 60 |
| 124 | 5mer-124 | KGFSK | 58 | 424 | 5mer-424 | RVYLK | 87 |
| 125 | 5mer-125 | RVRQK | 67 | 425 | 5mer-425 | RGFYR | 80 |
| 126 | 5mer-126 | RGVTK | 61 | 426 | 5mer-426 | RRVSR | 57 |
| 127 | 5mer-127 | RWVTK | 86 | 427 | 5mer-427 | RGLYR | 87 |
| 128 | 5mer-128 | RGVCR | 56 | 428 | 5mer-428 | RQFLK | 86 |
| 129 | 5mer-129 | KSLLK | 63 | 429 | 5mer-429 | KSQYK | 63 |
| 130 | 5mer-130 | RRFRK | 80 | 430 | 5mer-430 | KWRTK | 80 |
| 131 | 5mer-131 | KLRRR | 72 | 431 | 5mer-431 | KVQLK | 81 |
| 132 | 5mer-132 | RVRCK | 83 | 432 | 5mer-432 | RQVRR | 52 |
| 133 | 5mer-133 | KGQRK | 84 | 433 | 5mer-433 | KWRCR | 73 |
| 134 | 5mer-134 | RQFSK | 61 | 434 | 5mer-434 | RVLQK | 74 |
| 135 | 5mer-135 | RRLLR | 64 | 435 | 5mer-435 | KLRQR | 69 |
| 136 | 5mer-136 | RRIYK | 75 | 436 | 5mer-436 | RVFYK | 60 |
| 137 | 5mer-137 | KGVSK | 77 | 437 | 5mer-437 | RVRLR | 50 |
| 138 | 5mer-138 | RQRCR | 71 | 438 | 5mer-438 | RVRSR | 76 |
| 139 | 5mer-139 | RLQYK | 73 | 439 | 5mer-439 | KRVTR | 65 |
| 140 | 5mer-140 | KWRLR | 69 | 440 | 5mer-440 | RQVTK | 57 |
| 141 | 5mer-141 | RWQQR | 50 | 441 | 5mer-441 | KLIRK | 53 |
| 142 | 5mer-142 | RQYSR | 52 | 442 | 5mer-442 | KVRLR | 61 |
| 143 | 5mer-143 | KGQQR | 64 | 443 | 5mer-443 | RGFTK | 80 |
| 144 | 5mer-144 | KRFSK | 58 | 444 | 5mer-444 | KQQYR | 65 |
| 145 | 5mer-145 | RSFSK | 62 | 445 | 5mer-445 | RLFTR | 77 |
| 146 | 5mer-146 | RWFTK | 52 | 446 | 5mer-446 | RGLQK | 53 |
| 147 | 5mer-147 | RRYQK | 66 | 447 | 5mer-447 | KGYYK | 76 |
| 148 | 5mer-148 | RSQTK | 50 | 448 | 5mer-448 | RQIRK | 74 |
| 149 | 5mer-149 | KQFLR | 80 | 449 | 5mer-449 | KGLYR | 65 |
| 150 | 5mer-150 | RVRTR | 64 | 450 | 5mer-450 | RVIQK | 78 |
| 151 | 5mer-151 | RVFTK | 66 | 451 | 5mer-451 | RQVLK | 53 |
| 152 | 5mer-152 | RRRRR | 56 | 452 | 5mer-452 | RGVSK | 82 |
| 153 | 5mer-153 | RSIQK | 72 | 453 | 5mer-453 | KGVCR | 68 |
| 154 | 5mer-154 | RSITR | 54 | 454 | 5mer-454 | RQVLR | 77 |
| 155 | 5mer-155 | KRYLR | 57 | 455 | 5mer-455 | RWLYK | 61 |
| 156 | 5mer-156 | KGLRK | 85 | 456 | 5mer-456 | RLVCK | 52 |
| 157 | 5mer-157 | KGRCR | 78 | 457 | 5mer-457 | RGQYK | 59 |
| 158 | 5mer-158 | RWQTK | 65 | 458 | 5mer-458 | RRFLK | 65 |
| 159 | 5mer-159 | RGQRR | 73 | 459 | 5mer-459 | RSQLR | 76 |
| 160 | 5mer-160 | RSIRR | 53 | 460 | 5mer-460 | RLQQR | 74 |
| 161 | 5mer-161 | KWVTK | 52 | 461 | 5mer-461 | KQLLK | 74 |
| 162 | 5mer-162 | RGYQR | 51 | 462 | 5mer-462 | RWQSK | 69 |
| 163 | 5mer-163 | KSLCK | 61 | 463 | 5mer-463 | KWYTK | 52 |
| 164 | 5mer-164 | KSICK | 54 | 464 | 5mer-464 | RGVTR | 57 |
| 165 | 5mer-165 | KWRSK | 56 | 465 | 5mer-465 | RWFRK | 57 |
| 166 | 5mer-166 | KSFQR | 55 | 466 | 5mer-466 | RRVRK | 53 |
| 167 | 5mer-167 | KQFRK | 64 | 467 | 5mer-467 | RRIYR | 83 |
| 168 | 5mer-168 | KLFSK | 84 | 468 | 5mer-468 | RWQCK | 54 |
| 169 | 5mer-169 | RWYRK | 61 | 469 | 5mer-469 | KSRRR | 69 |
| 170 | 5mer-170 | RLICK | 62 | 470 | 5mer-470 | KQRSK | 57 |
| 171 | 5mer-171 | KSIQR | 55 | 471 | 5mer-471 | RGYLR | 69 |
| 172 | 5mer-172 | RSRRR | 82 | 472 | 5mer-472 | RRYRK | 55 |
| 173 | 5mer-173 | RSVSR | 59 | 473 | 5mer-473 | RLVQR | 75 |
| 174 | 5mer-174 | KGRQR | 59 | 474 | 5mer-474 | RQFLR | 80 |
| 175 | 5mer-175 | RWQLK | 63 | 475 | 5mer-475 | RRFCK | 56 |
| 176 | 5mer-176 | RVLYK | 52 | 476 | 5mer-476 | RSICK | 70 |
| 177 | 5mer-177 | RWVRK | 77 | 477 | 5mer-477 | KGYSR | 58 |
| 178 | 5mer-178 | RRVLK | 87 | 478 | 5mer-478 | RVQTK | 75 |
| 179 | 5mer-179 | RLYTK | 55 | 479 | 5mer-479 | RQYYK | 70 |
| 180 | 5mer-180 | KQRYR | 74 | 480 | 5mer-480 | RWYLR | 75 |
| 181 | 5mer-181 | RSLLK | 72 | 481 | 5mer-481 | KSYCK | 78 |
| 182 | 5mer-182 | RVLSK | 70 | 482 | 5mer-482 | KQFCK | 75 |
| 183 | 5mer-183 | KRVYR | 64 | 483 | 5mer-483 | RSISR | 51 |
| 184 | 5mer-184 | KLQCR | 68 | 484 | 5mer-484 | RSFQK | 51 |
| 185 | 5mer-185 | RRYRR | 86 | 485 | 5mer-485 | KRQRK | 58 |
| 186 | 5mer-186 | KRLQR | 64 | 486 | 5mer-486 | RWLQR | 53 |
| 187 | 5mer-187 | RWVCR | 74 | 487 | 5mer-487 | RLVTK | 62 |
| 188 | 5mer-188 | KWFSK | 82 | 488 | 5mer-488 | KVRSR | 58 |
| 189 | 5mer-189 | RLLQK | 65 | 489 | 5mer-489 | KSYSR | 63 |
| 190 | 5mer-190 | RWRSR | 84 | 490 | 5mer-490 | RWLLK | 60 |
| 191 | 5mer-191 | RVRCR | 75 | 491 | 5mer-491 | KQIRR | 81 |
| 192 | 5mer-192 | KSFRK | 75 | 492 | 5mer-492 | RVVLR | 80 |
| 193 | 5mer-193 | RVIRK | 80 | 493 | 5mer-493 | KSIRR | 57 |
| 194 | 5mer-194 | KWQSK | 84 | 494 | 5mer-494 | RGFCK | 54 |
| 195 | 5mer-195 | KVVSR | 82 | 495 | 5mer-495 | KVVTK | 52 |
| 196 | 5mer-196 | RSYYR | 69 | 496 | 5mer-496 | KGIQK | 81 |
| 197 | 5mer-197 | RSRQK | 68 | 497 | 5mer-497 | KRRCR | 76 |
| 198 | 5mer-198 | KLISR | 74 | 498 | 5mer-498 | KLISK | 53 |
| 199 | 5mer-199 | KRYQR | 84 | 499 | 5mer-499 | RVLTK | 76 |
| 200 | 5mer-200 | KRVRR | 55 | 500 | 5mer-500 | KSLQK | 63 |
| 201 | 5mer-201 | RWIYR | 62 | 501 | 5mer-501 | KLQRK | 78 |
| 202 | 5mer-202 | KWYRR | 65 | 502 | 5mer-502 | RRISR | 84 |
| 203 | 5mer-203 | RQLTR | 78 | 503 | 5mer-503 | KRVSK | 61 |
| 204 | 5mer-204 | RQFQR | 84 | 504 | 5mer-504 | KQIQR | 72 |
| 205 | 5mer-205 | RWYYR | 75 | 505 | 5mer-505 | RQIYR | 71 |
| 206 | 5mer-206 | KLQLR | 57 | 506 | 5mer-506 | RGRLR | 51 |
| 207 | 5mer-207 | KQVRR | 72 | 507 | 5mer-507 | KWVQK | 84 |
| 208 | 5mer-208 | KLICR | 80 | 508 | 5mer-508 | KGLQK | 67 |
| 209 | 5mer-209 | RGVRR | 78 | 509 | 5mer-509 | RVQRK | 61 |
| 210 | 5mer-210 | KVYYR | 67 | 510 | 5mer-510 | RQLSK | 71 |
| 211 | 5mer-211 | RLYYR | 73 | 511 | 5mer-511 | KSLSR | 56 |
| 212 | 5mer-212 | KQQYK | 64 | 512 | 5mer-512 | RLYLK | 70 |
| 213 | 5mer-213 | RQYTK | 83 | 513 | 5mer-513 | RWYYK | 83 |
| 214 | 5mer-214 | KQQCK | 79 | 514 | 5mer-514 | KLLLK | 52 |
| 215 | 5mer-215 | RQYRR | 79 | 515 | 5mer-515 | RGQRK | 56 |
| 216 | 5mer-216 | KWVRR | 56 | 516 | 5mer-516 | KWQTR | 83 |
| 217 | 5mer-217 | KLQRR | 72 | 517 | 5mer-517 | KGYTR | 70 |
| 218 | 5mer-218 | KVQYK | 80 | 518 | 5mer-518 | RVYYR | 87 |
| 219 | 5mer-219 | KQYTR | 55 | 519 | 5mer-519 | RLYSK | 72 |
| 220 | 5mer-220 | KRILR | 81 | 520 | 5mer-520 | RVLLK | 86 |
| 221 | 5mer-221 | RGRTR | 58 | 521 | 5mer-521 | KRFRK | 78 |
| 222 | 5mer-222 | KWLLR | 86 | 522 | 5mer-522 | RLVYR | 78 |
| 223 | 5mer-223 | KQVRK | 61 | 523 | 5mer-523 | KQFQK | 84 |
| 224 | 5mer-224 | RLQRR | 67 | 524 | 5mer-524 | RRICR | 83 |
| 225 | 5mer-225 | KRQQK | 51 | 525 | 5mer-525 | KQRYK | 51 |
| 226 | 5mer-226 | RRYTR | 60 | 526 | 5mer-526 | KQFSR | 54 |
| 227 | 5mer-227 | RRQQR | 84 | 527 | 5mer-527 | KSVQR | 70 |
| 228 | 5mer-228 | KRFLK | 53 | 528 | 5mer-528 | RVLLR | 50 |
| 229 | 5mer-229 | KGLTR | 84 | 529 | 5mer-529 | RVQSR | 84 |
| 230 | 5mer-230 | KVLCR | 67 | 530 | 5mer-530 | RWRRR | 80 |
| 231 | 5mer-231 | KGLLK | 60 | 531 | 5mer-531 | KRQSR | 65 |
| 232 | 5mer-232 | KWYTR | 71 | 532 | 5mer-532 | KQICK | 75 |
| 233 | 5mer-233 | RVYYK | 84 | 533 | 5mer-533 | RLVYK | 84 |
| 234 | 5mer-234 | RQYCR | 62 | 534 | 5mer-534 | RRQLR | 50 |
| 235 | 5mer-235 | KGYSK | 70 | 535 | 5mer-535 | KSLLR | 70 |
| 236 | 5mer-236 | KRRCK | 81 | 536 | 5mer-536 | RLITK | 78 |
| 237 | 5mer-237 | RGLRK | 77 | 537 | 5mer-537 | RLRLR | 59 |
| 238 | 5mer-238 | KQVLK | 58 | 538 | 5mer-538 | KQQQR | 72 |
| 239 | 5mer-239 | KRLYR | 61 | 539 | 5mer-539 | RVVTR | 70 |
| 240 | 5mer-240 | KWRYR | 51 | 540 | 5mer-540 | RQFYR | 74 |
| 241 | 5mer-241 | KVRCR | 51 | 541 | 5mer-541 | KVFLK | 80 |
| 242 | 5mer-242 | RGYTK | 64 | 542 | 5mer-542 | KRYTR | 85 |
| 243 | 5mer-243 | KLLSK | 51 | 543 | 5mer-543 | RLRLK | 65 |
| 244 | 5mer-244 | KSVSK | 78 | 544 | 5mer-544 | RLQLR | 63 |
| 245 | 5mer-245 | RQRSK | 62 | 545 | 5mer-545 | KGQYR | 80 |
| 246 | 5mer-246 | KSFTR | 60 | 546 | 5mer-546 | KWLK | 65 |
| 247 | 5mer-247 | RWYCR | 80 | 547 | 5mer-547 | KQVTK | 77 |
| 248 | 5mer-248 | RWYLK | 63 | 548 | 5mer-548 | KWLCK | 60 |
| 249 | 5mer-249 | RSYCR | 52 | 549 | 5mer-549 | KWITK | 78 |
| 250 | 5mer-250 | RQFSR | 76 | 550 | 5mer-550 | RQQSR | 83 |
| 251 | 5mer-251 | KRISK | 70 | 551 | 5mer-551 | RWQYR | 59 |
| 252 | 5mer-252 | KQYRK | 53 | 552 | 5mer-552 | RLYSR | 63 |
| 253 | 5mer-253 | RSLYR | 75 | 553 | 5mer-553 | KLLSR | 72 |
| 254 | 5mer-254 | KLLRK | 87 | 554 | 5mer-554 | RLLSK | 65 |
| 255 | 5mer-255 | KQRTR | 82 | 555 | 5mer-555 | KVQCK | 71 |
| 256 | 5mer-256 | RGVCK | 60 | 556 | 5mer-556 | RVIYK | 52 |
| 257 | 5mer-257 | RQFTR | 85 | 557 | 5mer-557 | KQRRK | 87 |
| 258 | 5mer-258 | KRQYK | 84 | 558 | 5mer-558 | RWRYK | 84 |
| 259 | 5mer-259 | RLIRK | 71 | 559 | 5mer-559 | KQQLK | 81 |
| 260 | 5mer-260 | KVYRR | 57 | 560 | 5mer-560 | KWYQR | 59 |
| 261 | 5mer-261 | RRVLR | 59 | 561 | 5mer-561 | KWRCK | 72 |
| 262 | 5mer-262 | KSRCR | 59 | 562 | 5mer-562 | RVRQR | 70 |
| 263 | 5mer-263 | KQVTR | 80 | 563 | 5mer-563 | RLVLK | 60 |
| 264 | 5mer-264 | KWYRK | 50 | 564 | 5mer-564 | RSRYR | 74 |
| 265 | 5mer-265 | RWQCR | 77 | 565 | 5mer-565 | RLVSK | 77 |
| 266 | 5mer-266 | KSYQK | 66 | 566 | 5mer-566 | KQLYK | 78 |
| 267 | 5mer-267 | KRLLK | 66 | 567 | 5mer-567 | RRYCK | 63 |
| 268 | 5mer-268 | KRYCK | 54 | 568 | 5mer-568 | KQITR | 64 |
| 269 | 5mer-269 | RLFLR | 85 | 569 | 5mer-569 | RQQRK | 79 |
| 270 | 5mer-270 | RSQQR | 52 | 570 | 5mer-570 | KLVRR | 56 |
| 271 | 5mer-271 | RGRYR | 68 | 571 | 5mer-571 | RRLTK | 51 |
| 272 | 5mer-272 | KGYQR | 83 | 572 | 5mer-572 | KLRTK | 58 |
| 273 | 5mer-273 | RVQSK | 82 | 573 | 5mer-573 | RSIYR | 84 |
| 274 | 5mer-274 | RQVRK | 62 | 574 | 5mer-574 | KGFTR | 59 |
| 275 | 5mer-275 | KVYTR | 64 | 575 | 5mer-575 | KGFYR | 69 |
| 276 | 5mer-276 | RSLRR | 70 | 576 | 5mer-576 | RLFRK | 62 |
| 277 | 5mer-277 | RVFTR | 82 | 577 | 5mer-577 | KLVTK | 77 |
| 278 | 5mer-278 | RWQRR | 66 | 578 | 5mer-578 | KSVLR | 65 |
| 279 | 5mer-279 | RVVTK | 56 | 579 | 5mer-579 | RSLYK | 53 |
| 280 | 5mer-280 | KWILK | 53 | 580 | 5mer-580 | RGYTR | 70 |
| 281 | 5mer-281 | RGFLK | 58 | 581 | 5mer-581 | KGVLR | 65 |
| 282 | 5mer-282 | RVLTR | 77 | 582 | 5mer-582 | RGICK | 82 |
| 283 | 5mer-283 | KWVYK | 51 | 583 | 5mer-583 | KVVYR | 75 |
| 284 | 5mer-284 | KQVQR | 52 | 584 | 5mer-584 | KQIQK | 76 |
| 285 | 5mer-285 | KGQLK | 75 | 585 | 5mer-585 | RSVSK | 74 |
| 286 | 5mer-286 | RWFQK | 71 | 586 | 5mer-586 | KGRRR | 78 |
| 287 | 5mer-287 | KRYRR | 68 | 587 | 5mer-587 | RVITR | 66 |
| 288 | 5mer-288 | KLILK | 73 | 588 | 5mer-588 | RSFTR | 60 |
| 289 | 5mer-289 | RRQYR | 87 | 589 | 5mer-589 | KGLCR | 58 |
| 290 | 5mer-290 | RGYYR | 57 | 590 | 5mer-590 | KQYTK | 81 |
| 291 | 5mer-291 | RWFTR | 72 | 591 | 5mer-591 | RVYTK | 61 |
| 292 | 5mer-292 | KLVCK | 59 | 592 | 5mer-592 | RWLCR | 56 |
| 293 | 5mer-293 | RQYYR | 83 | 593 | 5mer-593 | KQYLK | 77 |
| 294 | 5mer-294 | KQYYK | 50 | 594 | 5mer-594 | KQYSR | 50 |
| 295 | 5mer-295 | RGRCR | 52 | 595 | 5mer-595 | KWQQR | 58 |
| 296 | 5mer-296 | KWISK | 55 | 596 | 5mer-596 | KLQQR | 56 |
| 297 | 5mer-297 | RSLRK | 81 | 597 | 5mer-597 | KLVSR | 68 |
| 298 | 5mer-298 | KSIYR | 75 | 598 | 5mer-598 | RVRSK | 84 |
| 299 | 5mer-299 | KGRRK | 62 | 599 | 5mer-599 | KWVSR | 61 |
| 300 | 5mer-300 | RQYRK | 81 | 600 | 5mer-600 | KLYSK | 64 |
| Average Binding affinity | | | | | | | 68.5 |

**TABLE 9]**

| 5mer Bottom sequence | | | | | | | |
|---|---|---|---|---|---|---|---|
| SEQ ID NO: | Unique No . | Sequence | Binding affinity | SEQ ID NO: | Unique No. | Sequence | Binding affinity |
| 601 | 5mer-601 | RFCFK | 13 | 901 | 5mer-901 | KCTNR | 30 |
| 602 | 5mer-602 | KMSVR | 12 | 902 | 5mer-902 | REDPK | 23 |
| 603 | 5mer-603 | RKEPK | 27 | 903 | 5mer-903 | RHHKR | 21 |
| 604 | 5mer-604 | RKEGK | 13 | 904 | 5mer-904 | KPDPK | 23 |
| 605 | 5mer-605 | RECFK | 22 | 905 | 5mer-905 | RPSPR | 30 |
| 606 | 5mer-606 | RIHWK | 25 | 906 | 5mer-906 | RKHAK | 31 |
| 607 | 5mer-607 | RIEER | 25 | 907 | 5mer-907 | KDSVR | 18 |
| 608 | 5mer-608 | RDSER | 20 | 908 | 5mer-908 | RCHFK | 25 |
| 609 | 5mer-609 | RKTMK | 32 | 909 | 5mer-909 | RINVK | 33 |
| 610 | 5mer-610 | KCDDK | 16 | 910 | 5mer-910 | RPCFR | 17 |
| 611 | 5mer-611 | KDHIR | 33 | 911 | 5mer-911 | REHGK | 14 |
| 612 | 5mer-612 | KCTDK | 23 | 912 | 5mer-912 | KKMGR | 16 |
| 613 | 5mer-613 | RAE FR | 20 | 913 | 5mer-913 | KHTFK | 13 |
| 614 | 5mer-614 | RNTWK | 29 | 914 | 5mer-914 | RPTMR | 26 |
| 615 | 5mer-615 | KCCNR | 20 | 915 | 5mer-915 | KMTHR | 28 |
| 616 | 5mer-616 | KFCPR | 15 | 916 | 5mer-916 | RCHER | 12 |
| 617 | 5mer-617 | RYCAK | 28 | 917 | 5mer-917 | KCAPR | 11 |
| 618 | 5mer-618 | RMSWK | 19 | 918 | 5mer-918 | RNNVR | 12 |
| 619 | 5mer-619 | KCSFK | 19 | 919 | 5mer-919 | KNNIK | 32 |
| 620 | 5mer-620 | RCMHK | 23 | 920 | 5mer-920 | KPKIK | 15 |
| 621 | 5mer-621 | KKSMK | 13 | 921 | 5mer-921 | KKKFK | 21 |
| 622 | 5mer-622 | RKPKR | 22 | 922 | 5mer-922 | RMWKR | 14 |
| 623 | 5mer-623 | KNTGR | 19 | 923 | 5mer-923 | KCWMK | 19 |
| 624 | 5mer-624 | KEHER | 22 | 924 | 5mer-924 | RNEKK | 27 |
| 625 | 5mer-625 | KMCDR | 33 | 925 | 5mer-925 | KDCFK | 25 |
| 626 | 5mer-626 | KICMR | 12 | 926 | 5mer-926 | RNCWK | 22 |
| 627 | 5mer-627 | KMSPK | 30 | 927 | 5mer-927 | RDEDR | 19 |
| 628 | 5mer-628 | RYMDR | 19 | 928 | 5mer-928 | KHMVR | 27 |
| 629 | 5mer-629 | RPTNK | 32 | 929 | 5mer-929 | KYTFR | 28 |
| 630 | 5mer-630 | RITFR | 30 | 930 | 5mer-930 | RAWVR | 20 |
| 631 | 5mer-631 | RPANR | 17 | 931 | 5mer-931 | RITER | 11 |
| 632 | 5mer-632 | RFSHR | 33 | 932 | 5mer-932 | RTAWK | 27 |
| 633 | 5mer-633 | RAAKR | 20 | 933 | 5mer-933 | RYMKK | 27 |
| 634 | 5mer-634 | RMMMR | 26 | 934 | 5mer-934 | KMMMR | 17 |
| 635 | 5mer-635 | RMNGR | 21 | 935 | 5mer-935 | RETFK | 32 |
| 636 | 5mer-636 | KDKGR | 11 | 936 | 5mer-936 | RNHKR | 29 |
| 637 | 5mer-637 | KCDHK | 28 | 937 | 5mer-937 | KASNR | 21 |
| 638 | 5mer-638 | KHCIR | 33 | 938 | 5mer-938 | RIEFK | 18 |
| 639 | 5mer-639 | KETWK | 13 | 939 | 5mer-939 | KMHAR | 24 |
| 640 | 5mer-640 | KNPHK | 15 | 940 | 5mer-940 | KCMGK | 18 |
| 641 | 5mer-641 | RITWK | 22 | 941 | 5mer-941 | KNHHR | 21 |
| 642 | 5mer-642 | RAWDR | 22 | 942 | 5mer-942 | RPSDR | 11 |
| 643 | 5mer-643 | KHDGR | 28 | 943 | 5mer-943 | KDKGK | 12 |
| 644 | 5mer-644 | RMKWK | 24 | 944 | 5mer-944 | RYEFK | 25 |
| 645 | 5mer-645 | RHKDK | 20 | 945 | 5mer-945 | RYEKR | 23 |
| 646 | 5mer-646 | KDTKR | 16 | 946 | 5mer-946 | KYPWR | 32 |
| 647 | 5mer-647 | KHTVK | 29 | 947 | 5mer-947 | KDWIR | 27 |
| 648 | 5mer-648 | KNKKR | 15 | 948 | 5mer-948 | KDSFK | 18 |
| 649 | 5mer-649 | RYSFK | 20 | 949 | 5mer-949 | KMKMK | 26 |
| 650 | 5mer-650 | RPEDK | 22 | 950 | 5mer-950 | KDADR | 22 |
| 651 | 5mer-651 | RFTVR | 11 | 951 | 5mer-951 | RYMGR | 18 |
| 652 | 5mer-652 | RCAAR | 23 | 952 | 5mer-952 | KTMHK | 12 |
| 653 | 5mer-653 | KTTWK | 16 | 953 | 5mer-953 | RKMAR | 23 |
| 654 | 5mer-654 | KEWNR | 27 | 954 | 5mer-954 | KTEVK | 23 |
| 655 | 5mer-655 | RKCIR | 15 | 955 | 5mer-955 | KNADK | 14 |
| 656 | 5mer-656 | KATGR | 13 | 956 | 5mer-956 | KPTWK | 19 |
| 657 | 5mer-657 | KDNDR | 22 | 957 | 5mer-957 | RDCER | 30 |
| 658 | 5mer-658 | KKWGR | 31 | 958 | 5mer-958 | RKWKR | 11 |
| 659 | 5mer-659 | KIHEK | 22 | 959 | 5mer-959 | KPNMK | 29 |
| 660 | 5mer-660 | KFDVK | 11 | 960 | 5mer-960 | RNPGK | 14 |
| 661 | 5mer-661 | RCNMK | 30 | 961 | 5mer-961 | KIANR | 32 |
| 662 | 5mer-662 | KIEEK | 28 | 962 | 5mer-962 | RIWKR | 23 |
| 663 | 5mer-663 | RATPR | 31 | 963 | 5mer-963 | RAPNK | 24 |
| 664 | 5mer-664 | KPTWR | 17 | 964 | 5mer-964 | KYTDR | 22 |
| 665 | 5mer-665 | KTWEK | 26 | 965 | 5mer-965 | KMEIR | 30 |
| 666 | 5mer-666 | RHDWR | 18 | 966 | 5mer-966 | RPSAK | 32 |
| 667 | 5mer-667 | RDCHR | 16 | 967 | 5mer-967 | KKTHK | 26 |
| 668 | 5mer-668 | RPHAK | 27 | 968 | 5mer-968 | RIPHR | 11 |
| 669 | 5mer-669 | RNPER | 24 | 969 | 5mer-969 | RNTVR | 27 |
| 670 | 5mer-670 | KPCVK | 23 | 970 | 5mer-970 | KYMNR | 33 |
| 671 | 5mer-671 | KMNAK | 21 | 971 | 5mer-971 | KMTGK | 12 |
| 672 | 5mer-672 | KHAPK | 12 | 972 | 5mer-972 | KYAKR | 14 |
| 673 | 5mer-673 | RISPR | 14 | 973 | 5mer-973 | RYWAR | 12 |
| 674 | 5mer-674 | KETHR | 22 | 974 | 5mer-974 | RHMMR | 26 |
| 675 | 5mer-675 | RKPGR | 31 | 975 | 5mer-975 | RFAHK | 18 |
| 676 | 5mer-676 | RTMFR | 18 | 976 | 5mer-976 | RETNR | 16 |
| 677 | 5mer-677 | RCEMR | 15 | 977 | 5mer-977 | KNMIR | 16 |
| 678 | 5mer-678 | KYMGR | 23 | 978 | 5mer-978 | RYDHK | 30 |
| 679 | 5mer-679 | KKTVK | 21 | 979 | 5mer-979 | RHHMK | 23 |
| 680 | 5mer-680 | RFTER | 21 | 980 | 5mer-980 | RKPHR | 33 |
| 681 | 5mer-681 | KDTMR | 18 | 981 | 5mer-981 | KNDKK | 33 |
| 682 | 5mer-682 | KPNVK | 31 | 982 | 5mer-982 | KFMHK | 12 |
| 683 | 5mer-683 | KYPDK | 32 | 983 | 5mer-983 | RTTGR | 23 |
| 684 | 5mer-684 | KCAER | 27 | 984 | 5mer-984 | KNAHK | 11 |
| 685 | 5mer-685 | RNDIK | 21 | 985 | 5mer-985 | RDPKR | 17 |
| 686 | 5mer-686 | RFNIR | 18 | 986 | 5mer-986 | RPMDK | 12 |
| 687 | 5mer-687 | RMEIR | 22 | 987 | 5mer-987 | RAHWR | 22 |
| 688 | 5mer-688 | RPHFK | 31 | 988 | 5mer-988 | RCKWR | 17 |
| 689 | 5mer-689 | RFSDK | 20 | 989 | 5mer-989 | RCTAR | 31 |
| 690 | 5mer-690 | RAEIK | 12 | 990 | 5mer-990 | KEEPK | 12 |
| 691 | 5mer-691 | KEWEK | 16 | 991 | 5mer-991 | RPEFR | 24 |
| 692 | 5mer-692 | RDHWK | 15 | 992 | 5mer-992 | RNDKK | 15 |
| 693 | 5mer-693 | RPSKK | 17 | 993 | 5mer-993 | KEKFR | 27 |
| 694 | 5mer-694 | KNNAR | 24 | 994 | 5mer-994 | KAWVR | 26 |
| 695 | 5mer-695 | KKPDR | 26 | 995 | 5mer-995 | KFTDK | 18 |
| 696 | 5mer-696 | RCEHR | 11 | 996 | 5mer-996 | RMTKK | 32 |
| 697 | 5mer-697 | KASKK | 25 | 997 | 5mer-997 | RYPHR | 30 |
| 698 | 5mer-698 | KFDWK | 12 | 998 | 5mer-998 | RCTAK | 18 |
| 699 | 5mer-699 | KKNER | 12 | 999 | 5mer-999 | KTKFK | 19 |
| 700 | 5mer-700 | RAWER | 17 | 1000 | 5mer-1000 | KHCWR | 20 |
| 701 | 5mer-701 | RNHNK | 22 | 1001 | 5mer-1001 | KPCGK | 33 |
| 702 | 5mer-702 | KISHK | 30 | 1002 | 5mer-1002 | KEDNK | 12 |
| 703 | 5mer-703 | KPNWR | 22 | 1003 | 5mer-1003 | RMTVR | 11 |
| 704 | 5mer-704 | RFMGK | 28 | 1004 | 5mer-1004 | RDADR | 30 |
| 705 | 5mer-705 | RHAKK | 15 | 1005 | 5mer-1005 | RTHGR | 30 |
| 706 | 5mer-706 | RKMAK | 27 | 1006 | 5mer-1006 | KEPER | 17 |
| 707 | 5mer-707 | KIEAK | 32 | 1007 | 5mer-1007 | RDMMR | 30 |
| 708 | 5mer-708 | KPKAR | 20 | 1008 | 5mer-1008 | KDSNR | 11 |
| 709 | 5mer-709 | RHTIR | 27 | 1009 | 5mer-1009 | KMCAK | 29 |
| 710 | 5mer-710 | KEKAR | 21 | 1010 | 5mer-1010 | KCDNK | 16 |
| 711 | 5mer-711 | KECIK | 32 | 1011 | 5mer-1011 | KTDMK | 25 |
| 712 | 5mer-712 | RADFK | 18 | 1012 | 5mer-1012 | RHKVR | 22 |
| 713 | 5mer-713 | KYPKK | 16 | 1013 | 5mer-1013 | KCCVK | 19 |
| 714 | 5mer-714 | RPCAK | 32 | 1014 | 5mer-1014 | RKNKR | 23 |
| 715 | 5mer-715 | KCNGR | 22 | 1015 | 5mer-1015 | RCWMK | 30 |
| 716 | 5mer-716 | KINVR | 25 | 1016 | 5mer-1016 | KYTMR | 28 |
| 717 | 5mer-717 | KCKFR | 20 | 1017 | 5mer-1017 | RCTWK | 24 |
| 718 | 5mer-718 | RNKKK | 19 | 1018 | 5mer-1018 | KFWAK | 20 |
| 719 | 5mer-719 | KISER | 21 | 1019 | 5mer-1019 | RFKFK | 28 |
| 720 | 5mer-720 | KDPKR | 32 | 1020 | 5mer-1020 | KCKVK | 12 |
| 721 | 5mer-721 | RYSKK | 22 | 1021 | 5mer-1021 | KITHK | 26 |
| 722 | 5mer-722 | KNHKK | 33 | 1022 | 5mer-1022 | KIADK | 29 |
| 723 | 5mer-723 | RASPR | 22 | 1023 | 5mer-1023 | RYCKK | 13 |
| 724 | 5mer-724 | KTTAK | 14 | 1024 | 5mer-1024 | KIDNK | 26 |
| 725 | 5mer-725 | RNADR | 19 | 1025 | 5mer-1025 | RHKWK | 25 |
| 726 | 5mer-726 | KATMK | 33 | 1026 | 5mer-1026 | RNAVR | 20 |
| 727 | 5mer-727 | KMDAR | 31 | 1027 | 5mer-1027 | KNTNR | 28 |
| 728 | 5mer-728 | KPHIK | 19 | 1028 | 5mer-1028 | RMAMR | 28 |
| 729 | 5mer-729 | KEPDK | 23 | 1029 | 5mer-1029 | KADIK | 25 |
| 730 | 5mer-730 | KFCNK | 27 | 1030 | 5mer-1030 | RISDK | 18 |
| 731 | 5mer-731 | KADHK | 18 | 1031 | 5mer-1031 | RKEER | 13 |
| 732 | 5mer-732 | KIMWR | 22 | 1032 | 5mer-1032 | RHHMR | 23 |
| 733 | 5mer-733 | KTCDR | 23 | 1033 | 5mer-1033 | REWDK | 28 |
| 734 | 5mer-734 | KATER | 16 | 1034 | 5mer-1034 | RFAKK | 14 |
| 735 | 5mer-735 | RHCER | 11 | 1035 | 5mer-1035 | KEHVR | 19 |
| 736 | 5mer-736 | KKHHR | 24 | 1036 | 5mer-1036 | KTHMK | 23 |
| 737 | 5mer-737 | RINVR | 13 | 1037 | 5mer-1037 | KKS FR | 28 |
| 738 | 5mer-738 | KTPMK | 29 | 1038 | 5mer-1038 | KCCKR | 17 |
| 739 | 5mer-739 | KDCPR | 16 | 1039 | 5mer-1039 | KKDVK | 32 |
| 740 | 5mer-740 | RMMGK | 21 | 1040 | 5mer-1040 | KTPWR | 17 |
| 741 | 5mer-741 | KPTPR | 14 | 1041 | 5mer-1041 | RDWFK | 27 |
| 742 | 5mer-742 | RNSDK | 11 | 1042 | 5mer-1042 | RCCAK | 28 |
| 743 | 5mer-743 | KYAFR | 15 | 1043 | 5mer-1043 | KFEMK | 15 |
| 744 | 5mer-744 | KTEAK | 32 | 1044 | 5mer-1044 | RDCFK | 22 |
| 745 | 5mer-745 | RMEIK | 14 | 1045 | 5mer-1045 | KMDEK | 22 |
| 746 | 5mer-746 | RMTVK | 26 | 1046 | 5mer-1046 | KHTMR | 19 |
| 747 | 5mer-747 | RKTDK | 31 | 1047 | 5mer-1047 | KAHMR | 23 |
| 748 | 5mer-748 | REPMK | 12 | 1048 | 5mer-1048 | KYDNK | 12 |
| 749 | 5mer-749 | KHTWR | 24 | 1049 | 5mer-1049 | KPPGR | 29 |
| 750 | 5mer-750 | KEADK | 13 | 1050 | 5mer-1050 | KPEDR | 31 |
| 751 | 5mer-751 | RYMMR | 20 | 1051 | 5mer-1051 | RYAHR | 28 |
| 752 | 5mer-752 | KAWVK | 26 | 1052 | 5mer-1052 | RNDWR | 17 |
| 753 | 5mer-753 | RKTPK | 30 | 1053 | 5mer-1053 | KHEHK | 27 |
| 754 | 5mer-754 | RKNER | 29 | 1054 | 5mer-1054 | RNKVR | 21 |
| 755 | 5mer-755 | KNWDR | 15 | 1055 | 5mer-1055 | KHHEK | 15 |
| 756 | 5mer-756 | RCDWR | 18 | 1056 | 5mer-1056 | RCTPK | 11 |
| 757 | 5mer-757 | KYCMR | 17 | 1057 | 5mer-1057 | RFDPK | 13 |
| 758 | 5mer-758 | KCDFK | 30 | 1058 | 5mer-1058 | KIPWR | 25 |
| 759 | 5mer-759 | KHKMR | 17 | 1059 | 5mer-1059 | RMAGR | 29 |
| 760 | 5mer-760 | RIWAR | 19 | 1060 | 5mer-1060 | KTHIK | 29 |
| 761 | 5mer-761 | RMPWR | 31 | 1061 | 5mer-1061 | KDS FR | 24 |
| 762 | 5mer-762 | KMPER | 21 | 1062 | 5mer-1062 | RAPWR | 24 |
| 763 | 5mer-763 | RCHVR | 30 | 1063 | 5mer-1063 | RPHVK | 20 |
| 764 | 5mer-764 | KTWPK | 19 | 1064 | 5mer-1064 | KCMKR | 20 |
| 765 | 5mer-765 | RNE FR | 12 | 1065 | 5mer-1065 | KNPIR | 18 |
| 766 | 5mer-766 | RNMEK | 30 | 1066 | 5mer-1066 | RFWVK | 25 |
| 767 | 5mer-767 | RFHFK | 12 | 1067 | 5mer-1067 | KHENK | 23 |
| 768 | 5mer-768 | RDSWR | 11 | 1068 | 5mer-1068 | RMHKK | 11 |
| 769 | 5mer-769 | KTEWK | 32 | 1069 | 5mer-1069 | RPSFK | 31 |
| 770 | 5mer-770 | KKPFR | 11 | 1070 | 5mer-1070 | RETGK | 26 |
| 771 | 5mer-771 | RKWVK | 24 | 1071 | 5mer-1071 | RYEGR | 16 |
| 772 | 5mer-772 | KYWFK | 11 | 1072 | 5mer-1072 | KDWPK | 32 |
| 773 | 5mer-773 | RPNMR | 25 | 1073 | 5mer-1073 | RDEAK | 29 |
| 774 | 5mer-774 | RTCDR | 25 | 1074 | 5mer-1074 | RPCPR | 17 |
| 775 | 5mer-775 | RHTVK | 23 | 1075 | 5mer-1075 | RKHIK | 26 |
| 776 | 5mer-776 | RCDFR | 17 | 1076 | 5mer-1076 | KFEKK | 21 |
| 777 | 5mer-777 | RYNWK | 28 | 1077 | 5mer-1077 | KADMK | 12 |
| 778 | 5mer-778 | KKDHK | 24 | 1078 | 5mer-1078 | KEAWK | 19 |
| 779 | 5mer-779 | KYPPR | 21 | 1079 | 5mer-1079 | KYTGR | 31 |
| 780 | 5mer-780 | RDSHK | 27 | 1080 | 5mer-1080 | RYTHK | 25 |
| 781 | 5mer-781 | RIDPR | 28 | 1081 | 5mer-1081 | RPNVR | 13 |
| 782 | 5mer-782 | KANAR | 32 | 1082 | 5mer-1082 | RACER | 11 |
| 783 | 5mer-783 | KCKVR | 29 | 1083 | 5mer-1083 | KFSVR | 31 |
| 784 | 5mer-784 | RACEK | 21 | 1084 | 5mer-1084 | RTHDR | 21 |
| 785 | 5mer-785 | KPTIR | 31 | 1085 | 5mer-1085 | RTTER | 14 |
| 786 | 5mer-786 | RFNKK | 29 | 1086 | 5mer-1086 | RIWDR | 19 |
| 787 | 5mer-787 | KDNIR | 17 | 1087 | 5mer-1087 | KKKKR | 11 |
| 788 | 5mer-788 | KYHGK | 16 | 1088 | 5mer-1088 | KAKKK | 16 |
| 789 | 5mer-789 | KFWIR | 12 | 1089 | 5mer-1089 | KTHHR | 29 |
| 790 | 5mer-790 | REAGK | 33 | 1090 | 5mer-1090 | KISGK | 31 |
| 791 | 5mer-791 | KTKMR | 26 | 1091 | 5mer-1091 | KTEHR | 29 |
| 792 | 5mer-792 | RMEKK | 31 | 1092 | 5mer-1092 | KPMVK | 12 |
| 793 | 5mer-793 | KKPHR | 19 | 1093 | 5mer-1093 | REEDK | 32 |
| 794 | 5mer-794 | KEAPK | 20 | 1094 | 5mer-1094 | RYSMK | 15 |
| 795 | 5mer-795 | RTKKR | 20 | 1095 | 5mer-1095 | KAEKR | 27 |
| 796 | 5mer-796 | KNWVR | 17 | 1096 | 5mer-1096 | RPTDK | 15 |
| 797 | 5mer-797 | KETHK | 15 | 1097 | 5mer-1097 | KPDIR | 28 |
| 798 | 5mer-798 | RETMK | 13 | 1098 | 5mer-1098 | RFHGR | 31 |
| 799 | 5mer-799 | KDKPR | 24 | 1099 | 5mer-1099 | RATAR | 14 |
| 800 | 5mer-800 | KEAPR | 29 | 1100 | 5mer-1100 | RPWKR | 24 |
| 801 | 5mer-801 | KFHGR | 20 | 1101 | 5mer-1101 | KYKNR | 11 |
| 802 | 5mer-802 | RITVR | 27 | 1102 | 5mer-1102 | KTHKK | 20 |
| 803 | 5mer-803 | RFSDR | 11 | 1103 | 5mer-1103 | KKSHR | 18 |
| 804 | 5mer-804 | RNTIR | 20 | 1104 | 5mer-1104 | RETHK | 12 |
| 805 | 5mer-805 | RPTNR | 27 | 1105 | 5mer-1105 | RPTKR | 33 |
| 806 | 5mer-806 | RKMFR | 17 | 1106 | 5mer-1106 | RYTER | 18 |
| 807 | 5mer-807 | RKNKK | 20 | 1107 | 5mer-1107 | RPWHK | 21 |
| 808 | 5mer-808 | KFCIR | 13 | 1108 | 5mer-1108 | RNPIR | 19 |
| 809 | 5mer-809 | RKSGK | 15 | 1109 | 5mer-1109 | KCHPK | 14 |
| 810 | 5mer-810 | KFHKK | 11 | 1110 | 5mer-1110 | RMHMK | 13 |
| 811 | 5mer-811 | KMTMR | 32 | 1111 | 5mer-1111 | KAEHK | 12 |
| 812 | 5mer-812 | KTEGR | 27 | 1112 | 5mer-1112 | RTTFK | 29 |
| 813 | 5mer-813 | RAHVR | 21 | 1113 | 5mer-1113 | RHMER | 19 |
| 814 | 5mer-814 | RITMR | 11 | 1114 | 5mer-1114 | RKTFR | 28 |
| 815 | 5mer-815 | RKDHR | 27 | 1115 | 5mer-1115 | KEPEK | 27 |
| 816 | 5mer-816 | RKPIK | 26 | 1116 | 5mer-1116 | KECFR | 24 |
| 817 | 5mer-817 | RHKDR | 23 | 1117 | 5mer-1117 | RHKIR | 11 |
| 818 | 5mer-818 | KHWHR | 12 | 1118 | 5mer-1118 | RMDAR | 28 |
| 819 | 5mer-819 | RNTIK | 19 | 1119 | 5mer-1119 | KMNIK | 19 |
| 820 | 5mer-820 | RIMHK | 28 | 1120 | 5mer-1120 | RPPDR | 33 |
| 821 | 5mer-821 | RESDR | 12 | 1121 | 5mer-1121 | RFKVK | 29 |
| 822 | 5mer-822 | RAKGK | 16 | 1122 | 5mer-1122 | RNTDR | 14 |
| 823 | 5mer-823 | KECFK | 26 | 1123 | 5mer-1123 | KYEKR | 24 |
| 824 | 5mer-824 | KIDMK | 13 | 1124 | 5mer-1124 | KKPVK | 30 |
| 825 | 5mer-825 | RDEKK | 14 | 1125 | 5mer-1125 | RYPFK | 29 |
| 826 | 5mer-826 | KCKMK | 11 | 1126 | 5mer-1126 | KPTHK | 26 |
| 827 | 5mer-827 | KKAGR | 18 | 1127 | 5mer-1127 | RIADR | 16 |
| 828 | 5mer-828 | RFCWR | 27 | 1128 | 5mer-1128 | REWVR | 26 |
| 829 | 5mer-829 | KCWHR | 26 | 1129 | 5mer-1129 | KFDAR | 14 |
| 830 | 5mer-830 | RIDGK | 25 | 1130 | 5mer-1130 | RFMVR | 17 |
| 831 | 5mer-831 | RAAGK | 24 | 1131 | 5mer-1131 | KCDGK | 14 |
| 832 | 5mer-832 | RMHER | 18 | 1132 | 5mer-1132 | KFSMK | 19 |
| 833 | 5mer-833 | KATAK | 33 | 1133 | 5mer-1133 | KCSMK | 23 |
| 834 | 5mer-834 | RMNIK | 15 | 1134 | 5mer-1134 | RTCKR | 26 |
| 835 | 5mer-835 | RFMMR | 14 | 1135 | 5mer-1135 | RHTAR | 17 |
| 836 | 5mer-836 | REWNR | 15 | 1136 | 5mer-1136 | KNSAR | 25 |
| 837 | 5mer-837 | RPMDR | 28 | 1137 | 5mer-1137 | KEEEK | 22 |
| 838 | 5mer-838 | RHSMK | 13 | 1138 | 5mer-1138 | RENHK | 24 |
| 839 | 5mer-839 | RHHDK | 26 | 1139 | 5mer-1139 | KHCWK | 28 |
| 840 | 5mer-840 | KDTAR | 11 | 1140 | 5mer-1140 | RNEFK | 28 |
| 841 | 5mer-841 | KNEMR | 22 | 1141 | 5mer-1141 | REEVR | 26 |
| 842 | 5mer-842 | RHNVK | 20 | 1142 | 5mer-1142 | KTENK | 24 |
| 843 | 5mer-843 | KTTPK | 17 | 1143 | 5mer-1143 | RATGK | 19 |
| 844 | 5mer-844 | KTDPR | 32 | 1144 | 5mer-1144 | KAKNR | 33 |
| 845 | 5mer-845 | RYEDR | 15 | 1145 | 5mer-1145 | RMPAK | 25 |
| 846 | 5mer-846 | RPDWR | 32 | 1146 | 5mer-1146 | KPHGR | 23 |
| 847 | 5mer-847 | KAPEK | 28 | 1147 | 5mer-1147 | RDTGK | 16 |
| 848 | 5mer-848 | RYMVK | 12 | 1148 | 5mer-1148 | RDWFR | 24 |
| 849 | 5mer-849 | KTWMK | 15 | 1149 | 5mer-1149 | KETDR | 25 |
| 850 | 5mer-850 | KYDIR | 13 | 1150 | 5mer-1150 | KFTNR | 12 |
| 851 | 5mer-851 | KMCWK | 19 | 1151 | 5mer-1151 | KTNAR | 26 |
| 852 | 5mer-852 | KDSMK | 22 | 1152 | 5mer-1152 | KENFK | 18 |
| 853 | 5mer-853 | RYHIK | 13 | 1153 | 5mer-1153 | RDAPR | 27 |
| 854 | 5mer-854 | RNTER | 21 | 1154 | 5mer-1154 | KCWVR | 12 |
| 855 | 5mer-855 | RFMKK | 18 | 1155 | 5mer-1155 | RYDMK | 25 |
| 856 | 5mer-856 | KPPDR | 23 | 1156 | 5mer-1156 | KKMNK | 13 |
| 857 | 5mer-857 | KMENR | 23 | 1157 | 5mer-1157 | KTSHR | 21 |
| 858 | 5mer-858 | RIMGR | 33 | 1158 | 5mer-1158 | KYNPR | 23 |
| 859 | 5mer-859 | KHNAR | 26 | 1159 | 5mer-1159 | KKWKK | 17 |
| 860 | 5mer-860 | RCHAK | 31 | 1160 | 5mer-1160 | KETWR | 14 |
| 861 | 5mer-861 | KTKIR | 20 | 1161 | 5mer-1161 | KCHDR | 21 |
| 862 | 5mer-862 | KKNAK | 19 | 1162 | 5mer-1162 | KIKKK | 15 |
| 863 | 5mer-863 | REANR | 16 | 1163 | 5mer-1163 | KKSKR | 12 |
| 864 | 5mer-864 | KYHPR | 25 | 1164 | 5mer-1164 | RFPWR | 33 |
| 865 | 5mer-865 | RMMER | 21 | 1165 | 5mer-1165 | RNMNR | 21 |
| 866 | 5mer-866 | RINWR | 11 | 1166 | 5mer-1166 | KTHEK | 32 |
| 867 | 5mer-867 | KDSWK | 14 | 1167 | 5mer-1167 | KESWK | 12 |
| 868 | 5mer-868 | KMHFK | 18 | 1168 | 5mer-1168 | RHHWR | 31 |
| 869 | 5mer-869 | KKHPK | 31 | 1169 | 5mer-1169 | KDTPK | 29 |
| 870 | 5mer-870 | RCKVK | 25 | 1170 | 5mer-1170 | KHSEK | 28 |
| 871 | 5mer-871 | RHSHR | 21 | 1171 | 5mer-1171 | RCSWK | 21 |
| 872 | 5mer-872 | RKKWK | 29 | 1172 | 5mer-1172 | KIWWR | 25 |
| 873 | 5mer-873 | RYNFR | 32 | 1173 | 5mer-1173 | KECKR | 30 |
| 874 | 5mer-874 | RIHMR | 27 | 1174 | 5mer-1174 | KYSGK | 25 |
| 875 | 5mer-875 | KHCNK | 33 | 1175 | 5mer-1175 | RDMIR | 30 |
| 876 | 5mer-876 | KETAR | 16 | 1176 | 5mer-1176 | KIHFK | 25 |
| 877 | 5mer-877 | RFSMR | 17 | 1177 | 5mer-1177 | KIPVR | 20 |
| 878 | 5mer-878 | KHTER | 24 | 1178 | 5mer-1178 | RFDNR | 22 |
| 879 | 5mer-879 | KDCMK | 13 | 1179 | 5mer-1179 | RMNVR | 18 |
| 880 | 5mer-880 | RCDKK | 17 | 1180 | 5mer-1180 | KETAK | 27 |
| 881 | 5mer-881 | RNDAK | 31 | 1181 | 5mer-1181 | KKAKR | 22 |
| 882 | 5mer-882 | REEGR | 18 | 1182 | 5mer-1182 | REKWK | 15 |
| 883 | 5mer-883 | KMSAK | 16 | 1183 | 5mer-1183 | RHAPK | 13 |
| 884 | 5mer-884 | KIWEK | 28 | 1184 | 5mer-1184 | RDSMK | 14 |
| 885 | 5mer-885 | RHCNR | 19 | 1185 | 5mer-1185 | KPTGR | 30 |
| 886 | 5mer-886 | RMMFR | 21 | 1186 | 5mer-1186 | KMWVK | 12 |
| 887 | 5mer-887 | KTPVR | 30 | 1187 | 5mer-1187 | KHHIR | 27 |
| 888 | 5mer-888 | KKCIR | 31 | 1188 | 5mer-1188 | KPSGK | 30 |
| 889 | 5mer-889 | KTPGR | 25 | 1189 | 5mer-1189 | RKMDK | 11 |
| 890 | 5mer-890 | KKHFK | 33 | 1190 | 5mer-1190 | RFSEK | 32 |
| 891 | 5mer-891 | RKKMK | 24 | 1191 | 5mer-1191 | RMDWK | 30 |
| 892 | 5mer-892 | RKPMR | 29 | 1192 | 5mer-1192 | RYAKK | 28 |
| 893 | 5mer-893 | RIKIK | 18 | 1193 | 5mer-1193 | KHWFK | 22 |
| 894 | 5mer-894 | KMSPR | 18 | 1194 | 5mer-1194 | RDNGK | 18 |
| 895 | 5mer-895 | RFTKK | 18 | 1195 | 5mer-1195 | RHWEK | 19 |
| 896 | 5mer-896 | RYHWR | 27 | 1196 | 5mer-1196 | RICIR | 24 |
| 897 | 5mer-897 | KFCER | 30 | 1197 | 5mer-1197 | KDDAR | 24 |
| 898 | 5mer-898 | RYCMR | 20 | 1198 | 5mer-1198 | RAADK | 25 |
| 899 | 5mer-899 | RYTFR | 31 | 1199 | 5mer-1199 | KNNNR | 29 |
| 900 | 5mer-900 | RHSVK | 29 | 1200 | 5mer-1200 | RKNEK | 16 |
| Average Binding affinity | | | | | | | 21.8 |

**TABLE 10]**

| 6mer-1 Top sequence | | | | | | | |
|---|---|---|---|---|---|---|---|
| SEQ ID NO: | Unique No . | Sequence | Binding affinity | SEQ ID NO: | Unique No . | Sequence | Binding affinity |
| 1201 | 6mer-1-1 | KKVISK | 122 | 1501 | 6mer-1-301 | RRSILR | 154 |
| 1202 | 6mer-1-2 | RKQILR | 142 | 1502 | 6mer-1-302 | RRGQSR | 151 |
| 1203 | 6mer-1-3 | RKRRSR | 154 | 1503 | 6mer-1-303 | RKQYCK | 123 |
| 1204 | 6mer-1-4 | RKSFYK | 139 | 1504 | 6mer-1-304 | RKLYSR | 121 |
| 1205 | 6mer-1-5 | KRQIRK | 135 | 1505 | 6mer-1-305 | RKWISR | 153 |
| 1206 | 6mer-1-6 | RKSRCK | 153 | 1506 | 6mer-1-306 | KKLFYR | 135 |
| 1207 | 6mer-1-7 | KRQYSK | 126 | 1507 | 6mer-1-307 | KRWYRR | 153 |
| 1208 | 6mer-1-8 | RKRRRK | 125 | 1508 | 6mer-1-308 | KRRITR | 135 |
| 1209 | 6mer-1-9 | RKSFCK | 154 | 1509 | 6mer-1-309 | KKGICK | 153 |
| 1210 | 6mer-1-10 | KRWRLK | 139 | 1510 | 6mer-1-310 | RKRQLK | 127 |
| 1211 | 6mer-1-11 | KRQFTK | 139 | 1511 | 6mer-1-311 | RRGYQK | 127 |
| 1212 | 6mer-1-12 | KKQICK | 128 | 1512 | 6mer-1-312 | KKSRQK | 151 |
| 1213 | 6mer-1-13 | KRQIQR | 147 | 1513 | 6mer-1-313 | RRWFCK | 148 |
| 1214 | 6mer-1-14 | RKRFLK | 145 | 1514 | 6mer-1-314 | RRRILR | 141 |
| 1215 | 6mer-1-15 | KRQICK | 123 | 1515 | 6mer-1-315 | RKQQTK | 128 |
| 1216 | 6mer-1-16 | KKQYCR | 136 | 1516 | 6mer-1-316 | RKQIRK | 145 |
| 1217 | 6mer-1-17 | KRVQCR | 135 | 1517 | 6mer-1-317 | KRSFRR | 148 |
| 1218 | 6mer-1-18 | KKQFTR | 145 | 1518 | 6mer-1-318 | KKSRRK | 145 |
| 1219 | 6mer-1-19 | KRSFYR | 135 | 1519 | 6mer-1-319 | KRWVQK | 123 |
| 1220 | 6mer-1-20 | KKSRYK | 147 | 1520 | 6mer-1-320 | KRVQYR | 121 |
| 1221 | 6mer-1-21 | RKWYLK | 137 | 1521 | 6mer-1-321 | RKQVRR | 154 |
| 1222 | 6mer-1-22 | RKRLCK | 126 | 1522 | 6mer-1-322 | KRGVQR | 146 |
| 1223 | 6mer-1-23 | KRSVSR | 153 | 1523 | 6mer-1-323 | RRWLYR | 149 |
| 1224 | 6mer-1-24 | RKVIRK | 142 | 1524 | 6mer-1-324 | RRLQRR | 128 |
| 1225 | 6mer-1-25 | KKLIRR | 135 | 1525 | 6mer-1-325 | RRQIQR | 142 |
| 1226 | 6mer-1-26 | KKRVLR | 123 | 1526 | 6mer-1-326 | RKWIYK | 136 |
| 1227 | 6mer-1-27 | KRSFTK | 142 | 1527 | 6mer-1-327 | KRGITR | 139 |
| 1228 | 6mer-1-28 | RRWISK | 125 | 1528 | 6mer-1-328 | KRWLQK | 151 |
| 1229 | 6mer-1-29 | KRLISR | 124 | 1529 | 6mer-1-329 | RKWRQK | 123 |
| 1230 | 6mer-1-30 | RRQVRR | 154 | 1530 | 6mer-1-330 | RKVQRK | 148 |
| 1231 | 6mer-1-31 | KRLYCR | 121 | 1531 | 6mer-1-331 | RKRITR | 128 |
| 1232 | 6mer-1-32 | RKGQCR | 121 | 1532 | 6mer-1-332 | KKRVQK | 122 |
| 1233 | 6mer-1-33 | KKSYCR | 154 | 1533 | 6mer-1-333 | RKRQSR | 135 |
| 1234 | 6mer-1-34 | RKWLYK | 135 | 1534 | 6mer-1-334 | RRRRLR | 154 |
| 1235 | 6mer-1-35 | RRGRQR | 136 | 1535 | 6mer-1-335 | RRSQRR | 148 |
| 1236 | 6mer-1-36 | RKWQQK | 124 | 1536 | 6mer-1-336 | KRVVTK | 154 |
| 1237 | 6mer-1-37 | KKWVLR | 121 | 1537 | 6mer-1-337 | RKSRRR | 144 |
| 1238 | 6mer-1-38 | RRQQTK | 126 | 1538 | 6mer-1-338 | KKQVLK | 127 |
| 1239 | 6mer-1-39 | RKVYQK | 139 | 1539 | 6mer-1-339 | KKSIQK | 147 |
| 1240 | 6mer-1-40 | KRSQYK | 121 | 1540 | 6mer-1-340 | KRLLCK | 142 |
| 1241 | 6mer-1-41 | RKGITK | 122 | 1541 | 6mer-1-341 | RKQQQR | 128 |
| 1242 | 6mer-1-42 | KKRIQR | 139 | 1542 | 6mer-1-342 | RKRFCR | 131 |
| 1243 | 6mer-1-43 | RKRIRR | 548 | 1543 | 6mer-1-343 | KKLYTK | 135 |
| 1244 | 6mer-1-44 | KRQYCK | 148 | 1544 | 6mer-1-344 | KKSQCR | 125 |
| 1245 | 6mer-1-45 | RKGLTK | 145 | 1545 | 6mer-1-345 | KKLLCR | 147 |
| 1246 | 6mer-1-46 | KRLYQR | 142 | 1546 | 6mer-1-346 | KKRIYR | 123 |
| 1247 | 6mer-1-47 | KRRLLK | 149 | 1547 | 6mer-1-347 | KKQQSK | 126 |
| 1248 | 6mer-1-48 | RRWVRR | 124 | 1548 | 6mer-1-348 | RKQRLK | 142 |
| 1249 | 6mer-1-49 | RRWQTR | 149 | 1549 | 6mer-1-349 | KRLYSK | 123 |
| 1250 | 6mer-1-50 | KKVRTR | 139 | 1550 | 6mer-1-350 | KKLQTK | 153 |
| 1251 | 6mer-1-51 | RRVLYK | 127 | 1551 | 6mer-1-351 | RRGLRK | 141 |
| 1252 | 6mer-1-52 | RRRFLR | 131 | 1552 | 6mer-1-352 | RKVFRK | 142 |
| 1253 | 6mer-1-53 | KRVIQR | 123 | 1553 | 6mer-1-353 | KRLYLK | 126 |
| 1254 | 6mer-1-54 | RRRVCK | 144 | 1554 | 6mer-1-354 | KKVFLK | 153 |
| 1255 | 6mer-1-55 | KRSIYR | 133 | 1555 | 6mer-1-355 | RKVFYR | 148 |
| 1256 | 6mer-1-56 | RRGIQR | 137 | 1556 | 6mer-1-356 | KRGYSK | 153 |
| 1257 | 6mer-1-57 | RKQYYK | 139 | 1557 | 6mer-1-357 | KRRQTR | 148 |
| 1258 | 6mer-1-58 | RKSVTR | 126 | 1558 | 6mer-1-358 | KRRYTR | 124 |
| 1259 | 6mer-1-59 | KRLQCK | 145 | 1559 | 6mer-1-359 | KRQFLR | 131 |
| 1260 | 6mer-1-60 | RKSIQK | 139 | 1560 | 6mer-1-360 | KRVFLK | 148 |
| 1261 | 6mer-1-61 | KRSYCK | 121 | 1561 | 6mer-1-361 | RKSVQR | 127 |
| 1262 | 6mer-1-62 | RRWFTK | 133 | 1562 | 6mer-1-362 | KRWYK | 133 |
| 1263 | 6mer-1-63 | KKWVSR | 148 | 1563 | 6mer-1-363 | KRQIRR | 121 |
| 1264 | 6mer-1-64 | RKQFTK | 154 | 1564 | 6mer-1-364 | RKRQSK | 124 |
| 1265 | 6mer-1-65 | KKSRRR | 121 | 1565 | 6mer-1-365 | KKRICK | 142 |
| 1266 | 6mer-1-66 | KKSISR | 147 | 1566 | 6mer-1-366 | KRWFCR | 133 |
| 1267 | 6mer-1-67 | RRSYTR | 135 | 1567 | 6mer-1-367 | RKGFSK | 154 |
| 1268 | 6mer-1-68 | RKLQLK | 153 | 1568 | 6mer-1-368 | RRRFSK | 145 |
| 1269 | 6mer-1-69 | RKVRRR | 142 | 1569 | 6mer-1-369 | KKWYRK | 144 |
| 1270 | 6mer-1-70 | KKLLYR | 139 | 1570 | 6mer-1-370 | RKQLYK | 142 |
| 1271 | 6mer-1-71 | KRWFYK | 121 | 1571 | 6mer-1-371 | KRSLTR | 153 |
| 1272 | 6mer-1-72 | RKVQLK | 139 | 1572 | 6mer-1-372 | RKLRSK | 145 |
| 1273 | 6mer-1-73 | KKQQQR | 142 | 1573 | 6mer-1-373 | KRGVLK | 149 |
| 1274 | 6mer-1-74 | KKVRLR | 142 | 1574 | 6mer-1-374 | KKQFQK | 148 |
| 1275 | 6mer-1-75 | RRQYQK | 139 | 1575 | 6mer-1-375 | RRVFYR | 154 |
| 1276 | 6mer-1-76 | RRLQLK | 126 | 1576 | 6mer-1-376 | RRQLRK | 121 |
| 1277 | 6mer-1-77 | KKQLRR | 149 | 1577 | 6mer-1-377 | KRWITR | 148 |
| 1278 | 6mer-1-78 | KKRVSK | 145 | 1578 | 6mer-1-378 | KRSLQK | 153 |
| 1279 | 6mer-1-79 | KRLFCK | 137 | 1579 | 6mer-1-379 | KRRISK | 153 |
| 1280 | 6mer-1-80 | KRVQQR | 133 | 1580 | 6mer-1-380 | KRLQLR | 121 |
| 1281 | 6mer-1-81 | RRWQLR | 148 | 1581 | 6mer-1-381 | RKGFYR | 124 |
| 1282 | 6mer-1-82 | KKVLYR | 123 | 1582 | 6mer-1-382 | KRQYTR | 139 |
| 1283 | 6mer-1-83 | RRSFLR | 146 | 1583 | 6mer-1-383 | RKRRTK | 148 |
| 1284 | 6mer-1-84 | RRGQTK | 142 | 1584 | 6mer-1-384 | RKVICK | 148 |
| 1285 | 6mer-1-85 | RKRQCK | 121 | 1585 | 6mer-1-385 | KRQVLR | 127 |
| 1286 | 6mer-1-86 | KKWRQK | 142 | 1586 | 6mer-1-386 | RRSYQK | 127 |
| 1287 | 6mer-1-87 | KKQVYK | 139 | 1587 | 6mer-1-387 | KKGRLR | 148 |
| 1288 | 6mer-1-88 | KRWICK | 121 | 1588 | 6mer-1-388 | RRLLQR | 142 |
| 1289 | 6mer-1-89 | RKQRCK | 142 | 1589 | 6mer-1-389 | RKLFCK | 125 |
| 1290 | 6mer-1-90 | KKGISK | 125 | 1590 | 6mer-1-390 | RKLQQR | 121 |
| 1291 | 6mer-1-91 | RKQQCK | 131 | 1591 | 6mer-1-391 | RRQRCK | 131 |
| 1292 | 6mer-1-92 | RRWRLK | 153 | 1592 | 6mer-1-392 | RRVQTR | 135 |
| 1293 | 6mer-1-93 | KRWIRR | 136 | 1593 | 6mer-1-393 | KRLYYK | 148 |
| 1294 | 6mer-1-94 | RKVQQK | 142 | 1594 | 6mer-1-394 | KKVRRR | 149 |
| 1295 | 6mer-1-95 | KKWQTK | 142 | 1595 | 6mer-1-395 | KKRQTK | 125 |
| 1296 | 6mer-1-96 | KRGVRK | 125 | 1596 | 6mer-1-396 | KRQIQK | 148 |
| 1297 | 6mer-1-97 | KKVYLR | 139 | 1597 | 6mer-1-397 | KRLILR | 125 |
| 1298 | 6mer-1-98 | KRLQCR | 154 | 1598 | 6mer-1-398 | KKQYYR | 137 |
| 1299 | 6mer-1-99 | RKVVYR | 136 | 1599 | 6mer-1-399 | KRWILR | 125 |
| 1300 | 6mer-1-100 | RKWISK | 146 | 1600 | 6mer-1-400 | KRWQTK | 123 |
| 1301 | 6mer-1-101 | KRWLYR | 142 | 1601 | 6mer-1-401 | RRLYCR | 127 |
| 1302 | 6mer-1-102 | RRRYRR | 127 | 1602 | 6mer-1-402 | RRRVLR | 153 |
| 1303 | 6mer-1-103 | RKRICK | 122 | 1603 | 6mer-1-403 | KRSQTK | 153 |
| 1304 | 6mer-1-104 | KRGFSK | 121 | 1604 | 6mer-1-404 | KKQRQK | 137 |
| 1305 | 6mer-1-105 | KKSISK | 141 | 1605 | 6mer-1-405 | KKVQTR | 148 |
| 1306 | 6mer-1-106 | KRGQLK | 149 | 1606 | 6mer-1-406 | RKVLLR | 153 |
| 1307 | 6mer-1-107 | KKQRSR | 131 | 1607 | 6mer-1-407 | KRLRTR | 153 |
| 1308 | 6mer-1-108 | RRLLLR | 139 | 1608 | 6mer-1-408 | KRGRSR | 147 |
| 1309 | 6mer-1-109 | KKRYLR | 148 | 1609 | 6mer-1-409 | RRWISR | 144 |
| 1310 | 6mer-1-110 | KRQLLK | 121 | 1610 | 6mer-1-410 | KRVLRK | 148 |
| 1311 | 6mer-1-111 | RKVICR | 144 | 1611 | 6mer-1-411 | RRQRSR | 121 |
| 1312 | 6mer-1-112 | KKWVCR | 148 | 1612 | 6mer-1-412 | RKSILK | 139 |
| 1313 | 6mer-1-113 | KRGVQK | 148 | 1613 | 6mer-1-413 | RKQFCR | 148 |
| 1314 | 6mer-1-114 | RRQITK | 153 | 1614 | 6mer-1-414 | KRSFLR | 153 |
| 1315 | 6mer-1-115 | RRSLSR | 133 | 1615 | 6mer-1-415 | RRGVCR | 135 |
| 1316 | 6mer-1-116 | RKVRCK | 139 | 1616 | 6mer-1-416 | RKLIYR | 153 |
| 1317 | 6mer-1-117 | KRLFTR | 142 | 1617 | 6mer-1-417 | RKSVCK | 139 |
| 1318 | 6mer-1-118 | RKLQLR | 127 | 1618 | 6mer-1-418 | RRQVRK | 123 |
| 1319 | 6mer-1-119 | KRLQTR | 133 | 1619 | 6mer-1-419 | KRGFYR | 148 |
| 1320 | 6mer-1-120 | RRRYSR | 124 | 1620 | 6mer-1-420 | KKGQYK | 148 |
| 1321 | 6mer-1-121 | KRRYTK | 148 | 1621 | 6mer-1-421 | RKVLYR | 135 |
| 1322 | 6mer-1-122 | KKLIRK | 139 | 1622 | 6mer-1-422 | KRVRQR | 154 |
| 1323 | 6mer-1-123 | RRGVQK | 148 | 1623 | 6mer-1-423 | RRGYCK | 147 |
| 1324 | 6mer-1-124 | KRVQQK | 137 | 1624 | 6mer-1-424 | RRRIYR | 135 |
| 1325 | 6mer-1-125 | KRRLCR | 135 | 1625 | 6mer-1-425 | RKGVQK | 131 |
| 1326 | 6mer-1-126 | KKWIQR | 148 | 1626 | 6mer-1-426 | KKGQYR | 131 |
| 1327 | 6mer-1-127 | KRVIYR | 147 | 1627 | 6mer-1-427 | KRWVLR | 147 |
| 1328 | 6mer-1-128 | RKQFYR | 126 | 1628 | 6mer-1-428 | KRWLR | 139 |
| 1329 | 6mer-1-129 | RRRQSK | 121 | 1629 | 6mer-1-429 | RRQRRK | 135 |
| 1330 | 6mer-1-130 | RKQVLR | 142 | 1630 | 6mer-1-430 | KKQLQK | 144 |
| 1331 | 6mer-1-131 | KRGIQR | 142 | 1631 | 6mer-1-431 | RRRFRR | 144 |
| 1332 | 6mer-1-132 | RRQVYR | 153 | 1632 | 6mer-1-432 | RKGIQK | 139 |
| 1333 | 6mer-1-133 | RKRFLR | 154 | 1633 | 6mer-1-433 | RKQQYR | 154 |
| 1334 | 6mer-1-134 | KKSIYR | 128 | 1634 | 6mer-1-434 | KRWVCR | 137 |
| 1335 | 6mer-1-135 | KRQVSR | 123 | 1635 | 6mer-1-435 | RKWRCK | 135 |
| 1336 | 6mer-1-136 | RRWLYK | 148 | 1636 | 6mer-1-436 | KKLFYK | 148 |
| 1337 | 6mer-1-137 | RRLFRR | 121 | 1637 | 6mer-1-437 | RKWFLK | 131 |
| 1338 | 6mer-1-138 | RKWTR | 124 | 1638 | 6mer-1-438 | RRGFQK | 142 |
| 1339 | 6mer-1-139 | KRRFLR | 122 | 1639 | 6mer-1-439 | KRRQTK | 121 |
| 1340 | 6mer-1-140 | KRQVQR | 148 | 1640 | 6mer-1-440 | KRVYSK | 153 |
| 1341 | 6mer-1-141 | RKLYQK | 141 | 1641 | 6mer-1-441 | RRVYYR | 146 |
| 1342 | 6mer-1-142 | KKVYYK | 123 | 1642 | 6mer-1-442 | RKLQYK | 131 |
| 1343 | 6mer-1-143 | KRGVLR | 145 | 1643 | 6mer-1-443 | RKVLLK | 145 |
| 1344 | 6mer-1-144 | RKRYTR | 148 | 1644 | 6mer-1-444 | KKWILK | 144 |
| 1345 | 6mer-1-145 | RKVFQR | 141 | 1645 | 6mer-1-445 | KRVRLR | 145 |
| 1346 | 6mer-1-146 | KRGRRR | 139 | 1646 | 6mer-1-446 | RRVIRR | 124 |
| 1347 | 6mer-1-147 | KKQIQR | 125 | 1647 | 6mer-1-447 | RRRQQK | 146 |
| 1348 | 6mer-1-148 | RKLRCR | 146 | 1648 | 6mer-1-448 | RKRFSR | 141 |
| 1349 | 6mer-1-149 | RRWVCK | 131 | 1649 | 6mer-1-449 | KRGRCK | 135 |
| 1350 | 6mer-1-150 | RKRQCR | 142 | 1650 | 6mer-1-450 | RKWYK | 151 |
| 1351 | 6mer-1-151 | KRGVRR | 145 | 1651 | 6mer-1-451 | RKVRLR | 153 |
| 1352 | 6mer-1-152 | KRVIRR | 154 | 1652 | 6mer-1-452 | KRLYTR | 141 |
| 1353 | 6mer-1-153 | KKWQQR | 146 | 1653 | 6mer-1-453 | KKWQQK | 154 |
| 1354 | 6mer-1-154 | KKLYSR | 153 | 1654 | 6mer-1-454 | RKVLRR | 124 |
| 1355 | 6mer-1-155 | RRVRSR | 127 | 1655 | 6mer-1-455 | RRVFYK | 126 |
| 1356 | 6mer-1-156 | RRSVTK | 154 | 1656 | 6mer-1-456 | RRGQYR | 153 |
| 1357 | 6mer-1-157 | KKSLLR | 123 | 1657 | 6mer-1-457 | RKLLCR | 121 |
| 1358 | 6mer-1-158 | RRLIYR | 131 | 1658 | 6mer-1-458 | RKGQLK | 139 |
| 1359 | 6mer-1-159 | KRLRRK | 139 | 1659 | 6mer-1-459 | RRWVYK | 131 |
| 1360 | 6mer-1-160 | RRVIQR | 149 | 1660 | 6mer-1-460 | RRGFYR | 148 |
| 1361 | 6mer-1-161 | KRRYCK | 139 | 1661 | 6mer-1-461 | RRGYTR | 142 |
| 1362 | 6mer-1-162 | KKSLSK | 142 | 1662 | 6mer-1-462 | RKQILK | 126 |
| 1363 | 6mer-1-163 | KRLQTK | 147 | 1663 | 6mer-1-463 | KRSFRK | 153 |
| 1364 | 6mer-1-164 | KRGQYK | 145 | 1664 | 6mer-1-464 | RRQRLK | 131 |
| 1365 | 6mer-1-165 | KKWFTK | 148 | 1665 | 6mer-1-465 | RKQVYK | 154 |
| 1366 | 6mer-1-166 | RRWYSK | 148 | 1666 | 6mer-1-466 | KRSICR | 148 |
| 1367 | 6mer-1-167 | RRRYQR | 122 | 1667 | 6mer-1-467 | KRGYQK | 121 |
| 1368 | 6mer-1-168 | KRWFCK | 131 | 1668 | 6mer-1-468 | RRLYRR | 153 |
| 1369 | 6mer-1-169 | RRQQSK | 153 | 1669 | 6mer-1-469 | KKLIYR | 139 |
| 1370 | 6mer-1-170 | RKSFYR | 148 | 1670 | 6mer-1-470 | KKWVYR | 148 |
| 1371 | 6mer-1-171 | RKQFQK | 123 | 1671 | 6mer-1-471 | KKWRCK | 123 |
| 1372 | 6mer-1-172 | RRLIRK | 153 | 1672 | 6mer-1-472 | KRQRCK | 142 |
| 1373 | 6mer-1-173 | RKQYYR | 123 | 1673 | 6mer-1-473 | KKVLSK | 154 |
| 1374 | 6mer-1-174 | KKGVCR | 154 | 1674 | 6mer-1-474 | KRLFSK | 121 |
| 1375 | 6mer-1-175 | RRRIQK | 137 | 1675 | 6mer-1-475 | RKLQTK | 148 |
| 1376 | 6mer-1-176 | KKVYCK | 121 | 1676 | 6mer-1-476 | KRRRQK | 154 |
| 1377 | 6mer-1-177 | KRGYRR | 121 | 1677 | 6mer-1-477 | KRQQTK | 145 |
| 1378 | 6mer-1-178 | RKRYQK | 139 | 1678 | 6mer-1-478 | RKVRYK | 139 |
| 1379 | 6mer-1-179 | KRSQYR | 148 | 1679 | 6mer-1-479 | RRSLTR | 135 |
| 1380 | 6mer-1-180 | RKLVRK | 146 | 1680 | 6mer-1-480 | KKQFYK | 121 |
| 1381 | 6mer-1-181 | KKGVLR | 148 | 1681 | 6mer-1-481 | KRWRCK | 133 |
| 1382 | 6mer-1-182 | KRGICK | 147 | 1682 | 6mer-1-482 | RKQVRK | 133 |
| 1383 | 6mer-1-183 | KKGQTR | 121 | 1683 | 6mer-1-483 | KRQVTK | 151 |
| 1384 | 6mer-1-184 | KRQICR | 139 | 1684 | 6mer-1-484 | KKLYLR | 154 |
| 1385 | 6mer-1-185 | KRQLRK | 153 | 1685 | 6mer-1-485 | RRWYRR | 126 |
| 1386 | 6mer-1-186 | RRRVYK | 147 | 1686 | 6mer-1-486 | KRQVRK | 151 |
| 1387 | 6mer-1-187 | RKLFYK | 139 | 1687 | 6mer-1-487 | RKGRRK | 121 |
| 1388 | 6mer-1-188 | RKSLQK | 122 | 1688 | 6mer-1-488 | KKWYSK | 135 |
| 1389 | 6mer-1-189 | KKSYSK | 137 | 1689 | 6mer-1-489 | KKVITK | 148 |
| 1390 | 6mer-1-190 | RKLYYR | 153 | 1690 | 6mer-1-490 | KKLFRR | 133 |
| 1391 | 6mer-1-191 | KKQILK | 139 | 1691 | 6mer-1-491 | RKLRLR | 137 |
| 1392 | 6mer-1-192 | KRQYQR | 127 | 1692 | 6mer-1-492 | RRWRQK | 139 |
| 1393 | 6mer-1-193 | KRVQLK | 149 | 1693 | 6mer-1-493 | KRGYLR | 131 |
| 1394 | 6mer-1-194 | RKWQRR | 144 | 1694 | 6mer-1-494 | KKLFSR | 128 |
| 1395 | 6mer-1-195 | KRGISR | 122 | 1695 | 6mer-1-495 | RKGFQR | 126 |
| 1396 | 6mer-1-196 | RKWRRK | 142 | 1696 | 6mer-1-496 | RRVYQR | 148 |
| 1397 | 6mer-1-197 | RRSIRR | 139 | 1697 | 6mer-1-497 | KRRQLR | 133 |
| 1398 | 6mer-1-198 | KRLFCR | 148 | 1698 | 6mer-1-498 | RKRQQK | 139 |
| 1399 | 6mer-1-199 | KKQVQK | 151 | 1699 | 6mer-1-499 | RKLFYR | 149 |
| 1400 | 6mer-1-200 | RKLYQR | 142 | 1700 | 6mer-1-500 | RKLQSK | 151 |
| 1401 | 6mer-1-201 | RRRIQR | 151 | 1701 | 6mer-1-501 | KRVLYK | 154 |
| 1402 | 6mer-1-202 | RRQLTK | 154 | 1702 | 6mer-1-502 | RKLFSR | 146 |
| 1403 | 6mer-1-203 | RRQITR | 142 | 1703 | 6mer-1-503 | KRRLCK | 151 |
| 1404 | 6mer-1-204 | KKRQRR | 154 | 1704 | 6mer-1-504 | RRSITR | 122 |
| 1405 | 6mer-1-205 | KRWRLR | 133 | 1705 | 6mer-1-505 | RRRFTR | 148 |
| 1406 | 6mer-1-206 | RRLQTR | 121 | 1706 | 6mer-1-506 | RRRLSK | 151 |
| 1407 | 6mer-1-207 | KKSQQR | 151 | 1707 | 6mer-1-507 | RRSQSK | 139 |
| 1408 | 6mer-1-208 | KRSIQR | 122 | 1708 | 6mer-1-508 | RRQFSK | 135 |
| 1409 | 6mer-1-209 | RKGYTR | 121 | 1709 | 6mer-1-509 | RKWLRK | 128 |
| 1410 | 6mer-1-210 | RRGVRR | 184 | 1710 | 6mer-1-510 | KRRQSR | 122 |
| 1411 | 6mer-1-211 | KKQLLR | 123 | 1711 | 6mer-1-511 | RKVYRR | 136 |
| 1412 | 6mer-1-212 | RRRICR | 148 | 1712 | 6mer-1-512 | RKWICK | 154 |
| 1413 | 6mer-1-213 | KRRVRK | 127 | 1713 | 6mer-1-513 | RKQIRR | 154 |
| 1414 | 6mer-1-214 | RRSYCK | 139 | 1714 | 6mer-1-514 | KKGRSK | 128 |
| 1415 | 6mer-1-215 | RKRYSR | 136 | 1715 | 6mer-1-515 | RRGLSR | 139 |
| 1416 | 6mer-1-216 | RKSIQR | 148 | 1716 | 6mer-1-516 | KKRFSR | 139 |
| 1417 | 6mer-1-217 | KRVLQR | 135 | 1717 | 6mer-1-517 | KKGLCK | 139 |
| 1418 | 6mer-1-218 | KKQVTR | 126 | 1718 | 6mer-1-518 | KRWVLK | 153 |
| 1419 | 6mer-1-219 | RRLFCR | 121 | 1719 | 6mer-1-519 | KRRQCK | 147 |
| 1420 | 6mer-1-220 | KRQRQK | 148 | 1720 | 6mer-1-520 | KRQIYK | 127 |
| 1421 | 6mer-1-221 | KKWFQR | 148 | 1721 | 6mer-1-521 | RKVISR | 128 |
| 1422 | 6mer-1-222 | RRRVRR | 145 | 1722 | 6mer-1-522 | RKRRLR | 126 |
| 1423 | 6mer-1-223 | KKRRRK | 133 | 1723 | 6mer-1-523 | KRRRSK | 139 |
| 1424 | 6mer-1-224 | KRGFLR | 142 | 1724 | 6mer-1-524 | KKGRQK | 148 |
| 1425 | 6mer-1-225 | RKLICK | 142 | 1725 | 6mer-1-525 | RRQLRR | 139 |
| 1426 | 6mer-1-226 | RRWITR | 128 | 1726 | 6mer-1-526 | KRGYTR | 148 |
| 1427 | 6mer-1-227 | KRLVQK | 139 | 1727 | 6mer-1-527 | KKGIYR | 142 |
| 1428 | 6mer-1-228 | KRVIYK | 139 | 1728 | 6mer-1-528 | KRSYTR | 139 |
| 1429 | 6mer-1-229 | RRRLLR | 153 | 1729 | 6mer-1-529 | RRSLCK | 139 |
| 1430 | 6mer-1-230 | RKGYCK | 128 | 1730 | 6mer-1-530 | KRLVSK | 137 |
| 1431 | 6mer-1-231 | KRQRTK | 136 | 1731 | 6mer-1-531 | RKGQTK | 135 |
| 1432 | 6mer-1-232 | RRRICK | 146 | 1732 | 6mer-1-532 | KKSQLR | 121 |
| 1433 | 6mer-1-233 | KKWQSR | 128 | 1733 | 6mer-1-533 | RKWFSR | 145 |
| 1434 | 6mer-1-234 | RRGISR | 142 | 1734 | 6mer-1-534 | KKRLCR | 126 |
| 1435 | 6mer-1-235 | KKRYCR | 148 | 1735 | 6mer-1-535 | RRVILK | 139 |
| 1436 | 6mer-1-236 | RKLISR | 151 | 1736 | 6mer-1-536 | RRWFRK | 151 |
| 1437 | 6mer-1-237 | RKSRYK | 137 | 1737 | 6mer-1-537 | RRGLCK | 154 |
| 1438 | 6mer-1-238 | KKGQQK | 154 | 1738 | 6mer-1-538 | RRSQCK | 142 |
| 1439 | 6mer-1-239 | RKVLCR | 136 | 1739 | 6mer-1-539 | KRGIRK | 148 |
| 1440 | 6mer-1-240 | RRSRRR | 148 | 1740 | 6mer-1-540 | KRVQTR | 128 |
| 1441 | 6mer-1-241 | RRLYYK | 148 | 1741 | 6mer-1-541 | RKWFTR | 136 |
| 1442 | 6mer-1-242 | KRQYLK | 148 | 1742 | 6mer-1-542 | KRWFRR | 128 |
| 1443 | 6mer-1-243 | KKWYLR | 136 | 1743 | 6mer-1-543 | RRSVCK | 145 |
| 1444 | 6mer-1-244 | RRRVQK | 144 | 1744 | 6mer-1-544 | KRVICR | 149 |
| 1445 | 6mer-1-245 | RRGFRR | 142 | 1745 | 6mer-1-545 | RRSYRK | 127 |
| 1446 | 6mer-1-246 | RRLVSR | 122 | 1746 | 6mer-1-546 | RKLVQR | 123 |
| 1447 | 6mer-1-247 | KKVRQK | 122 | 1747 | 6mer-1-547 | RKVQQR | 148 |
| 1448 | 6mer-1-248 | RRLIYK | 151 | 1748 | 6mer-1-548 | RRVYSR | 123 |
| 1449 | 6mer-1-249 | RKGVSR | 142 | 1749 | 6mer-1-549 | KKSYQR | 142 |
| 1450 | 6mer-1-250 | RRVICR | 142 | 1750 | 6mer-1-550 | RRLICR | 148 |
| 1451 | 6mer-1-251 | RKVQSK | 133 | 1751 | 6mer-1-551 | KRGRLR | 142 |
| 1452 | 6mer-1-252 | RRWIRR | 131 | 1752 | 6mer-1-552 | RKRYRK | 144 |
| 1453 | 6mer-1-253 | RRVQLK | 145 | 1753 | 6mer-1-553 | RRGVRK | 128 |
| 1454 | 6mer-1-254 | RKSLSK | 137 | 1754 | 6mer-1-554 | KRSVTR | 131 |
| 1455 | 6mer-1-255 | KKGFQK | 139 | 1755 | 6mer-1-555 | KKWYLK | 128 |
| 1456 | 6mer-1-256 | KRVRTK | 125 | 1756 | 6mer-1-556 | KKGFTK | 145 |
| 1457 | 6mer-1-257 | RKWVTR | 124 | 1757 | 6mer-1-557 | KRQVQK | 128 |
| 1458 | 6mer-1-258 | KKLQYK | 137 | 1758 | 6mer-1-558 | RRLIQR | 139 |
| 1459 | 6mer-1-259 | KRRYRK | 141 | 1759 | 6mer-1-559 | RRRLTK | 139 |
| 1460 | 6mer-1-260 | RKQRTK | 141 | 1760 | 6mer-1-560 | RRSFSK | 127 |
| 1461 | 6mer-1-261 | KRWVTR | 121 | 1761 | 6mer-1-561 | RKVYLK | 131 |
| 1462 | 6mer-1-262 | RKQLQR | 139 | 1762 | 6mer-1-562 | KRSYQR | 148 |
| 1463 | 6mer-1-263 | RKSYTK | 146 | 1763 | 6mer-1-563 | RRSFTR | 139 |
| 1464 | 6mer-1-264 | RRQYQR | 128 | 1764 | 6mer-1-564 | KRSITK | 139 |
| 1465 | 6mer-1-265 | RKSVCR | 145 | 1765 | 6mer-1-565 | KRSIRR | 151 |
| 1466 | 6mer-1-266 | KKGRLK | 144 | 1766 | 6mer-1-566 | KKSFCR | 148 |
| 1467 | 6mer-1-267 | KRRLLR | 139 | 1767 | 6mer-1-567 | KKRVSR | 136 |
| 1468 | 6mer-1-268 | KRRQSK | 144 | 1768 | 6mer-1-568 | KRWQQK | 147 |
| 1469 | 6mer-1-269 | RKRRQR | 142 | 1769 | 6mer-1-569 | RRVITK | 148 |
| 1470 | 6mer-1-270 | KRGLRR | 149 | 1770 | 6mer-1-570 | RKRLRK | 122 |
| 1471 | 6mer-1-271 | RRQVSK | 122 | 1771 | 6mer-1-571 | RKLLCK | 154 |
| 1472 | 6mer-1-272 | KKQVSR | 139 | 1772 | 6mer-1-572 | KRVLLK | 126 |
| 1473 | 6mer-1-273 | KKQVSK | 137 | 1773 | 6mer-1-573 | KRVLLR | 126 |
| 1474 | 6mer-1-274 | KRLLYK | 135 | 1774 | 6mer-1-574 | RKSYLR | 148 |
| 1475 | 6mer-1-275 | RKRVLK | 123 | 1775 | 6mer-1-575 | KRWVRR | 148 |
| 1476 | 6mer-1-276 | RRVICK | 128 | 1776 | 6mer-1-576 | RKRQRR | 153 |
| 1477 | 6mer-1-277 | KKGLCR | 141 | 1777 | 6mer-1-577 | KKQYQR | 128 |
| 1478 | 6mer-1-278 | KRQQTR | 142 | 1778 | 6mer-1-578 | RKGQYK | 139 |
| 1479 | 6mer-1-279 | RRLRYR | 145 | 1779 | 6mer-1-579 | KRSVRK | 148 |
| 1480 | 6mer-1-280 | RKLQCK | 149 | 1780 | 6mer-1-580 | KKSLQK | 121 |
| 1481 | 6mer-1-281 | KRLLCR | 154 | 1781 | 6mer-1-581 | KRGLYK | 139 |
| 1482 | 6mer-1-282 | RRRRYR | 121 | 1782 | 6mer-1-582 | KRWFLK | 137 |
| 1483 | 6mer-1-283 | KKWVQK | 133 | 1783 | 6mer-1-583 | KKVLYK | 139 |
| 1484 | 6mer-1-284 | KRSRQK | 145 | 1784 | 6mer-1-584 | RKQVSK | 127 |
| 1485 | 6mer-1-285 | KRWVQR | 122 | 1785 | 6mer-1-585 | RKSRQK | 144 |
| 1486 | 6mer-1-286 | RKLISK | 139 | 1786 | 6mer-1-586 | KRVICK | 136 |
| 1487 | 6mer-1-287 | RKWLTR | 136 | 1787 | 6mer-1-587 | KKQLLK | 148 |
| 1488 | 6mer-1-288 | KKVQQR | 148 | 1788 | 6mer-1-588 | RRLVRK | 153 |
| 1489 | 6mer-1-289 | RKRYYR | 153 | 1789 | 6mer-1-589 | KKLQQK | 139 |
| 1490 | 6mer-1-290 | RKQLTK | 146 | 1790 | 6mer-1-590 | RKGVTK | 142 |
| 1491 | 6mer-1-291 | KKQIRK | 142 | 1791 | 6mer-1-591 | KKRLYR | 147 |
| 1492 | 6mer-1-292 | RKWQTK | 122 | 1792 | 6mer-1-592 | RRWLSK | 135 |
| 1493 | 6mer-1-293 | RKRVCK | 154 | 1793 | 6mer-1-593 | RKVYRK | 148 |
| 1494 | 6mer-1-294 | KKLYRR | 123 | 1794 | 6mer-1-594 | KRQLQR | 121 |
| 1495 | 6mer-1-295 | KRQRCR | 148 | 1795 | 6mer-1-595 | KKSLCK | 121 |
| 1496 | 6mer-1-296 | RKSIRR | 121 | 1796 | 6mer-1-596 | RKGVLR | 148 |
| 1497 | 6mer-1-297 | RRGFSK | 139 | 1797 | 6mer-1-597 | KRGFYK | 148 |
| 1498 | 6mer-1-298 | KKLYCK | 147 | 1798 | 6mer-1-598 | RKWLSK | 142 |
| 1499 | 6mer-1-299 | KRWLSK | 139 | 1799 | 6mer-1-599 | KKLYQR | 124 |
| 1500 | 6mer-1-300 | RRWQQK | 148 | 1800 | 6mer-1-600 | KRGYCR | 139 |
| Average Binding affinity | | | | | | | 138.66 |

**TABLE 11**

| 6mer-1 Bottom sequence | | | | | | | |
|---|---|---|---|---|---|---|---|
| SEQ ID NO: | Unique No . | Sequence | Binding affinity | SEQ ID NO: | Unique No . | Sequence | Binding affinity |
| 1801 | 6mer-1-601 | KKEWNK | 27 | 2101 | 6mer-1-901 | RKPNGR | 35 |
| 1802 | 6mer-1-602 | RKAEKR | 18 | 2102 | 6mer-1-902 | RRDKMK | 39 |
| 1803 | 6mer-1-603 | KRPHFK | 13 | 2103 | 6mer-1-903 | KKHKPR | 37 |
| 1804 | 6mer-1-604 | KKFTKK | 24 | 2104 | 6mer-1-904 | KKDTKR | 31 |
| 1805 | 6mer-1-605 | RKNADR | 20 | 2105 | 6mer-1-905 | RRICER | 36 |
| 1806 | 6mer-1-606 | RREWFR | 15 | 2106 | 6mer-1-906 | KKINGK | 30 |
| 1807 | 6mer-1-607 | KKDHFR | 22 | 2107 | 6mer-1-907 | RKPAWR | 42 |
| 1808 | 6mer-1-608 | KKACMR | 18 | 2108 | 6mer-1-908 | KKFMVR | 37 |
| 1809 | 6mer-1-609 | RKNPVR | 24 | 2109 | 6mer-1-909 | RKFCMR | 33 |
| 1810 | 6mer-1-610 | KKHHER | 27 | 2110 | 6mer-1-910 | KRPTIR | 42 |
| 1811 | 6mer-1-611 | KKNCHK | 23 | 2111 | 6mer-1-911 | KRCAAR | 28 |
| 1812 | 6mer-1-612 | KRIMER | 17 | 2112 | 6mer-1-912 | KKETFR | 33 |
| 1813 | 6mer-1-613 | KRIMKK | 14 | 2113 | 6mer-1-913 | KRFWWK | 27 |
| 1814 | 6mer-1-614 | KKEEER | 12 | 2114 | 6mer-1-914 | RRKCVR | 39 |
| 1815 | 6mer-1-615 | KKTSER | 18 | 2115 | 6mer-1-915 | RKDTWK | 27 |
| 1816 | 6mer-1-616 | KKNTIK | 31 | 2116 | 6mer-1-916 | RRNAAK | 33 |
| 1817 | 6mer-1-617 | KRHNVR | 31 | 2117 | 6mer-1-917 | KRADDK | 27 |
| 1818 | 6mer-1-618 | RKCHVK | 19 | 2118 | 6mer-1-918 | RRESMR | 33 |
| 1819 | 6mer-1-619 | KKIDKR | 23 | 2119 | 6mer-1-919 | RKDHIK | 26 |
| 1820 | 6mer-1-620 | RRKTAK | 11 | 2120 | 6mer-1-920 | RRPAMR | 40 |
| 1821 | 6mer-1-621 | RRMAGK | 30 | 2121 | 6mer-1-921 | RKFWHR | 36 |
| 1822 | 6mer-1-622 | KKCPNR | 18 | 2122 | 6mer-1-922 | RRDWGR | 34 |
| 1823 | 6mer-1-623 | RRTDWR | 21 | 2123 | 6mer-1-923 | RRASDR | 43 |
| 1824 | 6mer-1-624 | KKPPAK | 30 | 2124 | 6mer-1-924 | RKFNWR | 29 |
| 1825 | 6mer-1-625 | KREDKK | 33 | 2125 | 6mer-1-925 | KKAMHR | 42 |
| 1826 | 6mer-1-626 | KRPDNR | 27 | 2126 | 6mer-1-926 | KRESDR | 40 |
| 1827 | 6mer-1-627 | RRTWWR | 29 | 2127 | 6mer-1-927 | KRESHR | 37 |
| 1828 | 6mer-1-628 | RRENEK | 23 | 2128 | 6mer-1-928 | KRYCPK | 30 |
| 1829 | 6mer-1-629 | RKMDAK | 11 | 2129 | 6mer-1-929 | RRCDHK | 25 |
| 1830 | 6mer-1-630 | KRMEPR | 14 | 2130 | 6mer-1-930 | KKESNK | 43 |
| 1831 | 6mer-1-631 | RRYSDR | 13 | 2131 | 6mer-1-931 | KKNWPR | 35 |
| 1832 | 6mer-1-632 | RKDTAR | 17 | 2132 | 6mer-1-932 | KRFEFK | 38 |
| 1833 | 6mer-1-633 | RKIDAK | 16 | 2133 | 6mer-1-933 | RRPWNR | 28 |
| 1834 | 6mer-1-634 | KKNTMR | 12 | 2134 | 6mer-1-934 | KRPPGK | 39 |
| 1835 | 6mer-1-635 | KKTTWK | 33 | 2135 | 6mer-1-935 | RKKDIK | 40 |
| 1836 | 6mer-1-636 | RKYKDR | 27 | 2136 | 6mer-1-936 | RKETDR | 36 |
| 1837 | 6mer-1-637 | RRFEFK | 33 | 2137 | 6mer-1-937 | RKPTNR | 34 |
| 1838 | 6mer-1-638 | KKESHR | 20 | 2138 | 6mer-1-938 | KKIAWK | 28 |
| 1839 | 6mer-1-639 | KRPNVR | 24 | 2139 | 6mer-1-939 | KRNDFK | 41 |
| 1840 | 6mer-1-640 | RRNCWR | 25 | 2140 | 6mer-1-940 | RKPEDR | 29 |
| 1841 | 6mer-1-641 | RREMFK | 21 | 2141 | 6mer-1-941 | RKTPKK | 29 |
| 1842 | 6mer-1-642 | KKYNGK | 24 | 2142 | 6mer-1-942 | RRMMVR | 37 |
| 1843 | 6mer-1-643 | KRYSMR | 31 | 2143 | 6mer-1-943 | KKEHDK | 43 |
| 1844 | 6mer-1-644 | RKTWFR | 31 | 2144 | 6mer-1-944 | RKPAGK | 25 |
| 1845 | 6mer-1-645 | RRTTIR | 33 | 2145 | 6mer-1-945 | RKTSIK | 31 |
| 1846 | 6mer-1-646 | RRCTGK | 33 | 2146 | 6mer-1-946 | RRCEPR | 31 |
| 1847 | 6mer-1-647 | RKYNVR | 14 | 2147 | 6mer-1-947 | KRTKGR | 31 |
| 1848 | 6mer-1-648 | KKFWEK | 17 | 2148 | 6mer-1-948 | RKFTVR | 24 |
| 1849 | 6mer-1-649 | RRIAGK | 32 | 2149 | 6mer-1-949 | RKEMPR | 37 |
| 1850 | 6mer-1-650 | RRCNNR | 18 | 2150 | 6mer-1-950 | RKEDKR | 29 |
| 1851 | 6mer-1-651 | KRICWK | 27 | 2151 | 6mer-1-951 | RKAMDK | 29 |
| 1852 | 6mer-1-652 | RRPEVK | 26 | 2152 | 6mer-1-952 | KRHDKK | 28 |
| 1853 | 6mer-1-653 | KRMTHR | 15 | 2153 | 6mer-1-953 | KRTNGK | 26 |
| 1854 | 6mer-1-654 | KRMNDR | 23 | 2154 | 6mer-1-954 | KKAKGR | 41 |
| 1855 | 6mer-1-655 | RKNMFR | 12 | 2155 | 6mer-1-955 | RKECFK | 23 |
| 1856 | 6mer-1-656 | RRPTHK | 28 | 2156 | 6mer-1-956 | KRKSNK | 34 |
| 1857 | 6mer-1-657 | RRCTVR | 17 | 2157 | 6mer-1-957 | KKPPDK | 43 |
| 1858 | 6mer-1-658 | RKKEMK | 20 | 2158 | 6mer-1-958 | KRYAGR | 43 |
| 1859 | 6mer-1-659 | KKPSKR | 29 | 2159 | 6mer-1-959 | KKIWFR | 36 |
| 1860 | 6mer-1-660 | KKYHVK | 31 | 2160 | 6mer-1-960 | KKCPFR | 34 |
| 1861 | 6mer-1-661 | RKPKWR | 14 | 2161 | 6mer-1-961 | RRTWAR | 22 |
| 1862 | 6mer-1-662 | KKMTER | 20 | 2162 | 6mer-1-962 | KKNCDK | 42 |
| 1863 | 6mer-1-663 | KKFKDR | 26 | 2163 | 6mer-1-963 | RREEDK | 29 |
| 1864 | 6mer-1-664 | KRDHIK | 26 | 2164 | 6mer-1-964 | RKFKNK | 42 |
| 1865 | 6mer-1-665 | KKCDGK | 21 | 2165 | 6mer-1-965 | KRIWDK | 36 |
| 1866 | 6mer-1-666 | KKAANK | 18 | 2166 | 6mer-1-966 | RRNSWK | 22 |
| 1867 | 6mer-1-667 | KRDSPK | 32 | 2167 | 6mer-1-967 | RRTEDR | 24 |
| 1868 | 6mer-1-668 | RKATVK | 17 | 2168 | 6mer-1-968 | RREDFR | 39 |
| 1869 | 6mer-1-669 | KRNMAK | 23 | 2169 | 6mer-1-969 | RKDHWR | 33 |
| 1870 | 6mer-1-670 | KKDWNR | 11 | 2170 | 6mer-1-970 | RRCCAR | 38 |
| 1871 | 6mer-1-671 | KKNMVR | 11 | 2171 | 6mer-1-971 | RKIEMK | 32 |
| 1872 | 6mer-1-672 | RKMEIR | 17 | 2172 | 6mer-1-972 | RRHTHK | 43 |
| 1873 | 6mer-1-673 | RRAMPK | 16 | 2173 | 6mer-1-973 | RRDHVK | 25 |
| 1874 | 6mer-1-674 | RKHPKK | 32 | 2174 | 6mer-1-974 | RKPAHK | 27 |
| 1875 | 6mer-1-675 | KKIWKR | 21 | 2175 | 6mer-1-975 | RKASIK | 22 |
| 1876 | 6mer-1-676 | KRCPWR | 16 | 2176 | 6mer-1-976 | RKEMIK | 40 |
| 1877 | 6mer-1-677 | KKCCFR | 22 | 2177 | 6mer-1-977 | RRPCFR | 43 |
| 1878 | 6mer-1-678 | KRMAAK | 25 | 2178 | 6mer-1-978 | RRKSHK | 43 |
| 1879 | 6mer-1-679 | KKAEER | 30 | 2179 | 6mer-1-979 | RRYWEK | 42 |
| 1880 | 6mer-1-680 | KKDPPR | 27 | 2180 | 6mer-1-980 | RKICAK | 33 |
| 1881 | 6mer-1-681 | KKEWKK | 32 | 2181 | 6mer-1-981 | RKDDKK | 27 |
| 1882 | 6mer-1-682 | KKKDIR | 30 | 2182 | 6mer-1-982 | KRCPDR | 35 |
| 1883 | 6mer-1-683 | KRDSMR | 16 | 2183 | 6mer-1-983 | KRPHKK | 38 |
| 1884 | 6mer-1-684 | KRNNDR | 20 | 2184 | 6mer-1-984 | KKESDK | 33 |
| 1885 | 6mer-1-685 | RRESWK | 14 | 2185 | 6mer-1-985 | KRKSER | 22 |
| 1886 | 6mer-1-686 | KKYEVR | 25 | 2186 | 6mer-1-986 | KKAEEK | 22 |
| 1887 | 6mer-1-687 | RREWIK | 23 | 2187 | 6mer-1-987 | RRTEFK | 34 |
| 1888 | 6mer-1-688 | RKDEVR | 33 | 2188 | 6mer-1-988 | KRYPDR | 24 |
| 1889 | 6mer-1-689 | KRCPVR | 11 | 2189 | 6mer-1-989 | KRATVR | 27 |
| 1890 | 6mer-1-690 | KREAER | 21 | 2190 | 6mer-1-990 | RRFTER | 40 |
| 1891 | 6mer-1-691 | RKPKAR | 31 | 2191 | 6mer-1-991 | RKAHDK | 25 |
| 1892 | 6mer-1-692 | RKNTHK | 22 | 2192 | 6mer-1-992 | RRCTAK | 28 |
| 1893 | 6mer-1-693 | RKIDMK | 22 | 2193 | 6mer-1-993 | KRAHDK | 26 |
| 1894 | 6mer-1-694 | RKYTKR | 27 | 2194 | 6mer-1-994 | KKHEFR | 37 |
| 1895 | 6mer-1-695 | KRADVK | 13 | 2195 | 6mer-1-995 | KRCHHR | 33 |
| 1896 | 6mer-1-696 | KKHCNR | 18 | 2196 | 6mer-1-996 | RRPPWK | 39 |
| 1897 | 6mer-1-697 | RRPTNR | 13 | 2197 | 6mer-1-997 | KKPTFR | 35 |
| 1898 | 6mer-1-698 | KRFNHK | 27 | 2198 | 6mer-1-998 | KRNPVK | 41 |
| 1899 | 6mer-1-699 | RRTTVR | 28 | 2199 | 6mer-1-999 | RKATNR | 24 |
| 1900 | 6mer-1-700 | RKKMWK | 19 | 2200 | 6mer-1-1000 | RKMSHR | 28 |
| 1901 | 6mer-1-701 | RRKTWR | 13 | 2201 | 6mer-1-1001 | RRMPER | 23 |
| 1902 | 6mer-1-702 | KRDWVK | 31 | 2202 | 6mer-1-1002 | KRIHMK | 29 |
| 1903 | 6mer-1-703 | KKFHPR | 18 | 2203 | 6mer-1-1003 | RKYCHR | 24 |
| 1904 | 6mer-1-704 | RRMCER | 16 | 2204 | 6mer-1-1004 | RKHSHR | 41 |
| 1905 | 6mer-1-705 | KRTEWR | 12 | 2205 | 6mer-1-1005 | RKPPDR | 26 |
| 1906 | 6mer-1-706 | KRMKDR | 29 | 2206 | 6mer-1-1006 | KKDKIK | 25 |
| 1907 | 6mer-1-707 | KRFWPK | 26 | 2207 | 6mer-1-1007 | RKPTDR | 39 |
| 1908 | 6mer-1-708 | KKKHEK | 14 | 2208 | 6mer-1-1008 | RKPWAR | 21 |
| 1909 | 6mer-1-709 | KKFKPK | 29 | 2209 | 6mer-1-1009 | RKMSKK | 29 |
| 1910 | 6mer-1-710 | KKMHHR | 21 | 2210 | 6mer-1-1010 | KKPKHK | 33 |
| 1911 | 6mer-1-711 | RRATHR | 19 | 2211 | 6mer-1-1011 | RKKPEK | 35 |
| 1912 | 6mer-1-712 | KKPSNK | 24 | 2212 | 6mer-1-1012 | RRAWVR | 35 |
| 1913 | 6mer-1-713 | KKATNK | 20 | 2213 | 6mer-1-1013 | KKTTNK | 29 |
| 1914 | 6mer-1-714 | KRTSFK | 25 | 2214 | 6mer-1-1014 | KKNAWR | 25 |
| 1915 | 6mer-1-715 | KRMMVK | 18 | 2215 | 6mer-1-1015 | KREEWR | 26 |
| 1916 | 6mer-1-716 | RRMPFK | 11 | 2216 | 6mer-1-1016 | KRHTHR | 21 |
| 1917 | 6mer-1-717 | KRHEDR | 22 | 2217 | 6mer-1-1017 | KRANMK | 37 |
| 1918 | 6mer-1-718 | RKFKAR | 23 | 2218 | 6mer-1-1018 | KKTAFK | 28 |
| 1919 | 6mer-1-719 | KKDAEK | 27 | 2219 | 6mer-1-1019 | KRDAWR | 37 |
| 1920 | 6mer-1-720 | RRKHKR | 11 | 2220 | 6mer-1-1020 | KKMEAK | 38 |
| 1921 | 6mer-1-721 | RKAMEK | 22 | 2221 | 6mer-1-1021 | KKHDIK | 41 |
| 1922 | 6mer-1-722 | RRNKVR | 12 | 2222 | 6mer-1-1022 | RKDTVR | 22 |
| 1923 | 6mer-1-723 | RKMPAK | 33 | 2223 | 6mer-1-1023 | KRIMHK | 39 |
| 1924 | 6mer-1-724 | KRPKNK | 19 | 2224 | 6mer-1-1024 | RKTEIR | 36 |
| 1925 | 6mer-1-725 | KKDKPK | 32 | 2225 | 6mer-1-1025 | KRFAKR | 33 |
| 1926 | 6mer-1-726 | KKHSMK | 33 | 2226 | 6mer-1-1026 | RKKCER | 30 |
| 1927 | 6mer-1-727 | RKANGK | 29 | 2227 | 6mer-1-1027 | KKPWHK | 22 |
| 1928 | 6mer-1-728 | RRDNAR | 23 | 2228 | 6mer-1-1028 | RRHCWR | 36 |
| 1929 | 6mer-1-729 | KKKSGK | 23 | 2229 | 6mer-1-1029 | RRCMAK | 40 |
| 1930 | 6mer-1-730 | KRATFR | 13 | 2230 | 6mer-1-1030 | KKFCFR | 30 |
| 1931 | 6mer-1-731 | RRIKAR | 18 | 2231 | 6mer-1-1031 | RKDTHK | 38 |
| 1932 | 6mer-1-732 | RRAAVR | 26 | 2232 | 6mer-1-1032 | KKTSMR | 27 |
| 1933 | 6mer-1-733 | KKKMFK | 21 | 2233 | 6mer-1-1033 | KRHMVK | 30 |
| 1934 | 6mer-1-734 | RRTHER | 21 | 2234 | 6mer-1-1034 | KKASDR | 24 |
| 1935 | 6mer-1-735 | RKDKER | 19 | 2235 | 6mer-1-1035 | RRCHHR | 34 |
| 1936 | 6mer-1-736 | KKATDK | 15 | 2236 | 6mer-1-1036 | KKITFR | 21 |
| 1937 | 6mer-1-737 | RRKNAR | 27 | 2237 | 6mer-1-1037 | RRKNNR | 36 |
| 1938 | 6mer-1-738 | RKYHHR | 25 | 2238 | 6mer-1-1038 | RRKKIR | 21 |
| 1939 | 6mer-1-739 | RKCCEK | 32 | 2239 | 6mer-1-1039 | KRADIK | 40 |
| 1940 | 6mer-1-740 | RKDNKR | 26 | 2240 | 6mer-1-1040 | KRIEFR | 28 |
| 1941 | 6mer-1-741 | RKEAGR | 24 | 2241 | 6mer-1-1041 | RKEHGK | 23 |
| 1942 | 6mer-1-742 | RRFHWR | 14 | 2242 | 6mer-1-1042 | KKITAR | 40 |
| 1943 | 6mer-1-743 | KKTMVR | 14 | 2243 | 6mer-1-1043 | KRAAHR | 30 |
| 1944 | 6mer-1-744 | RKHEIK | 31 | 2244 | 6mer-1-1044 | KKHHIR | 24 |
| 1945 | 6mer-1-745 | KKTMEK | 11 | 2245 | 6mer-1-1045 | KKTPGR | 27 |
| 1946 | 6mer-1-746 | RKMSVK | 24 | 2246 | 6mer-1-1046 | RKDPHR | 38 |
| 1947 | 6mer-1-747 | KKTMDR | 24 | 2247 | 6mer-1-1047 | RKATDK | 30 |
| 1948 | 6mer-1-748 | RKHNGR | 24 | 2248 | 6mer-1-1048 | RRCMMK | 36 |
| 1949 | 6mer-1-749 | RKPHWR | 33 | 2249 | 6mer-1-1049 | KKDHKK | 31 |
| 1950 | 6mer-1-750 | KRENIK | 33 | 2250 | 6mer-1-1050 | KKMEDR | 38 |
| 1951 | 6mer-1-751 | RRTNFK | 27 | 2251 | 6mer-1-1051 | KKKSFK | 43 |
| 1952 | 6mer-1-752 | KRPTEK | 16 | 2252 | 6mer-1-1052 | KRHWIR | 33 |
| 1953 | 6mer-1-753 | KKDTHK | 12 | 2253 | 6mer-1-1053 | RKYTPK | 27 |
| 1954 | 6mer-1-754 | RKKKMK | 24 | 2254 | 6mer-1-1054 | RRMKVK | 37 |
| 1955 | 6mer-1-755 | RRANMR | 25 | 2255 | 6mer-1-1055 | KRCNDK | 40 |
| 1956 | 6mer-1-756 | RKAKMK | 33 | 2256 | 6mer-1-1056 | RRNTAK | 27 |
| 1957 | 6mer-1-757 | RKDNEK | 26 | 2257 | 6mer-1-1057 | KRKPVR | 43 |
| 1958 | 6mer-1-758 | KKYHHR | 33 | 2258 | 6mer-1-1058 | KRFPWR | 21 |
| 1959 | 6mer-1-759 | RKMTWR | 23 | 2259 | 6mer-1-1059 | RRETVK | 39 |
| 1960 | 6mer-1-760 | KRMMVR | 21 | 2260 | 6mer-1-1060 | KRYNVR | 21 |
| 1961 | 6mer-1-761 | RKETFR | 33 | 2261 | 6mer-1-1061 | RRISIR | 41 |
| 1962 | 6mer-1-762 | KKTKAR | 13 | 2262 | 6mer-1-1062 | RRICMR | 27 |
| 1963 | 6mer-1-763 | RRDCAK | 12 | 2263 | 6mer-1-1063 | KKIDHK | 25 |
| 1964 | 6mer-1-764 | RRNPFK | 24 | 2264 | 6mer-1-1064 | RKPTAK | 41 |
| 1965 | 6mer-1-765 | KRASPK | 25 | 2265 | 6mer-1-1065 | KRDKAK | 23 |
| 1966 | 6mer-1-766 | RKCTIR | 15 | 2266 | 6mer-1-1066 | RKEDVK | 23 |
| 1967 | 6mer-1-767 | KRTPDK | 18 | 2267 | 6mer-1-1067 | RRKHWK | 38 |
| 1968 | 6mer-1-768 | RKFEWK | 32 | 2268 | 6mer-1-1068 | KRYHIR | 31 |
| 1969 | 6mer-1-769 | RKMPVK | 14 | 2269 | 6mer-1-1069 | KKIWAR | 23 |
| 1970 | 6mer-1-770 | RKTNAR | 29 | 2270 | 6mer-1-1070 | KKTCDK | 24 |
| 1971 | 6mer-1-771 | KKMDIK | 17 | 2271 | 6mer-1-1071 | RKTKAK | 38 |
| 1972 | 6mer-1-772 | KRNCAK | 12 | 2272 | 6mer-1-1072 | KKCTPR | 36 |
| 1973 | 6mer-1-773 | KRTEIR | 29 | 2273 | 6mer-1-1073 | RKYMWK | 27 |
| 1974 | 6mer-1-774 | KKEMER | 21 | 2274 | 6mer-1-1074 | KRTCGR | 24 |
| 1975 | 6mer-1-775 | KKYPWR | 12 | 2275 | 6mer-1-1075 | KKPSVK | 38 |
| 1976 | 6mer-1-776 | KKTSIK | 13 | 2276 | 6mer-1-1076 | RKKEPK | 43 |
| 1977 | 6mer-1-777 | RRHNHR | 16 | 2277 | 6mer-1-1077 | RRKWGR | 24 |
| 1978 | 6mer-1-778 | RRDDFR | 28 | 2278 | 6mer-1-1078 | RRPDIK | 25 |
| 1979 | 6mer-1-779 | RRPDAR | 30 | 2279 | 6mer-1-1079 | RKHPVK | 25 |
| 1980 | 6mer-1-780 | KRKCWR | 24 | 2280 | 6mer-1-1080 | KKHNMR | 38 |
| 1981 | 6mer-1-781 | RRHWDK | 16 | 2281 | 6mer-1-1081 | KRPSNK | 23 |
| 1982 | 6mer-1-782 | KKNAWK | 30 | 2282 | 6mer-1-1082 | KKTCDR | 37 |
| 1983 | 6mer-1-783 | KKNEFK | 29 | 2283 | 6mer-1-1083 | KKNAVR | 36 |
| 1984 | 6mer-1-784 | KKFTPK | 33 | 2284 | 6mer-1-1084 | KKCCPK | 25 |
| 1985 | 6mer-1-785 | KKTCER | 19 | 2285 | 6mer-1-1085 | KREAMK | 33 |
| 1986 | 6mer-1-786 | KRIKEK | 26 | 2286 | 6mer-1-1086 | RKEANK | 22 |
| 1987 | 6mer-1-787 | KKESIK | 16 | 2287 | 6mer-1-1087 | KKPWKR | 27 |
| 1988 | 6mer-1-788 | RKIPKK | 22 | 2288 | 6mer-1-1088 | RRETHK | 38 |
| 1989 | 6mer-1-789 | KRMMPK | 19 | 2289 | 6mer-1-1089 | RKFTKK | 31 |
| 1990 | 6mer-1-790 | KKPNPK | 12 | 2290 | 6mer-1-1090 | KKTMWR | 27 |
| 1991 | 6mer-1-791 | RKPDDK | 13 | 2291 | 6mer-1-1091 | KKDDGK | 32 |
| 1992 | 6mer-1-792 | KRKDKK | 30 | 2292 | 6mer-1-1092 | RKYHDK | 43 |
| 1993 | 6mer-1-793 | KKTPNR | 31 | 2293 | 6mer-1-1093 | RRKSDR | 37 |
| 1994 | 6mer-1-794 | RKYEPK | 11 | 2294 | 6mer-1-1094 | RRMAIK | 42 |
| 1995 | 6mer-1-795 | KKCHMR | 12 | 2295 | 6mer-1-1095 | RRCHVK | 27 |
| 1996 | 6mer-1-796 | RRHAFK | 28 | 2296 | 6mer-1-1096 | RKPEPR | 23 |
| 1997 | 6mer-1-797 | RRAWNK | 22 | 2297 | 6mer-1-1097 | RKNCNK | 39 |
| 1998 | 6mer-1-798 | RRDCFR | 18 | 2298 | 6mer-1-1098 | RKKDMR | 42 |
| 1999 | 6mer-1-799 | RRKKKR | 30 | 2299 | 6mer-1-1099 | RRKTDR | 24 |
| 2000 | 6mer-1-800 | RREHIR | 15 | 2300 | 6mer-1-1100 | KRMSHK | 43 |
| 2001 | 6mer-1-801 | KKCAVK | 18 | 2301 | 6mer-1-1101 | RRECDK | 37 |
| 2002 | 6mer-1-802 | RKYHGR | 32 | 2302 | 6mer-1-1102 | KRFTKK | 35 |
| 2003 | 6mer-1-803 | RKMKHK | 31 | 2303 | 6mer-1-1103 | KREDNK | 27 |
| 2004 | 6mer-1-804 | KRDPFR | 31 | 2304 | 6mer-1-1104 | KRNSAR | 28 |
| 2005 | 6mer-1-805 | KRDTMK | 24 | 2305 | 6mer-1-1105 | RRCTAR | 40 |
| 2006 | 6mer-1-806 | RKKTIR | 12 | 2306 | 6mer-1-1106 | KRFMNK | 39 |
| 2007 | 6mer-1-807 | RRDKNR | 13 | 2307 | 6mer-1-1107 | KRCPPR | 36 |
| 2008 | 6mer-1-808 | RRFEKK | 24 | 2308 | 6mer-1-1108 | RRAHNK | 43 |
| 2009 | 6mer-1-809 | KKKSNR | 12 | 2309 | 6mer-1-1109 | KRCCVR | 41 |
| 2010 | 6mer-1-810 | RKCNAR | 12 | 2310 | 6mer-1-1110 | RKFTER | 24 |
| 2011 | 6mer-1-811 | KRHCHK | 16 | 2311 | 6mer-1-1111 | RRASPR | 38 |
| 2012 | 6mer-1-812 | RKDHMK | 26 | 2312 | 6mer-1-1112 | KKDPWK | 27 |
| 2013 | 6mer-1-813 | RREDFK | 15 | 2313 | 6mer-1-1113 | RRCTMK | 28 |
| 2014 | 6mer-1-814 | KKCNAK | 15 | 2314 | 6mer-1-1114 | KKFNFK | 38 |
| 2015 | 6mer-1-815 | RRFEMR | 23 | 2315 | 6mer-1-1115 | RKCAFR | 43 |
| 2016 | 6mer-1-816 | RKIDFK | 14 | 2316 | 6mer-1-1116 | KRHPFR | 35 |
| 2017 | 6mer-1-817 | RRIMKR | 16 | 2317 | 6mer-1-1117 | KKEWFR | 40 |
| 2018 | 6mer-1-818 | RKIKAR | 14 | 2318 | 6mer-1-1118 | KKDTIK | 33 |
| 2019 | 6mer-1-819 | KRMHAK | 17 | 2319 | 6mer-1-1119 | KRIWVR | 31 |
| 2020 | 6mer-1-820 | KRNDGK | 12 | 2320 | 6mer-1-1120 | RRKEAK | 22 |
| 2021 | 6mer-1-821 | RRTTNR | 22 | 2321 | 6mer-1-1121 | KKTWWR | 28 |
| 2022 | 6mer-1-822 | KREPIK | 17 | 2322 | 6mer-1-1122 | RRTMGR | 33 |
| 2023 | 6mer-1-823 | KRHTGK | 12 | 2323 | 6mer-1-1123 | RRDNVK | 41 |
| 2024 | 6mer-1-824 | KKMNIK | 12 | 2324 | 6mer-1-1124 | KKDEIR | 21 |
| 2025 | 6mer-1-825 | RKEKWK | 30 | 2325 | 6mer-1-1125 | KKPTDR | 26 |
| 2026 | 6mer-1-826 | KKAHMR | 31 | 2326 | 6mer-1-1126 | RRHCDK | 41 |
| 2027 | 6mer-1-827 | RKKKFR | 30 | 2327 | 6mer-1-1127 | RKMMMK | 34 |
| 2028 | 6mer-1-828 | RRPNMK | 26 | 2328 | 6mer-1-1128 | KKKKPK | 39 |
| 2029 | 6mer-1-829 | RRIHKK | 25 | 2329 | 6mer-1-1129 | RKDHAR | 39 |
| 2030 | 6mer-1-830 | KKTTMK | 33 | 2330 | 6mer-1-1130 | RKATFR | 31 |
| 2031 | 6mer-1-831 | KKNPWK | 15 | 2331 | 6mer-1-1131 | RKDNIK | 42 |
| 2032 | 6mer-1-832 | RKYWGK | 12 | 2332 | 6mer-1-1132 | RRKAER | 31 |
| 2033 | 6mer-1-833 | RRYKNR | 19 | 2333 | 6mer-1-1133 | KKPNIK | 24 |
| 2034 | 6mer-1-834 | KRNTIR | 17 | 2334 | 6mer-1-1134 | KRMSWK | 22 |
| 2035 | 6mer-1-835 | KRTADK | 26 | 2335 | 6mer-1-1135 | KKHTER | 26 |
| 2036 | 6mer-1-836 | RRCMIK | 22 | 2336 | 6mer-1-1136 | KKIEIK | 34 |
| 2037 | 6mer-1-837 | KRDHAK | 19 | 2337 | 6mer-1-1137 | KRFTDK | 33 |
| 2038 | 6mer-1-838 | KRNTWK | 17 | 2338 | 6mer-1-1138 | KRDCFR | 21 |
| 2039 | 6mer-1-839 | KRNWAK | 29 | 2339 | 6mer-1-1139 | KKMENR | 36 |
| 2040 | 6mer-1-840 | RKICNK | 12 | 2340 | 6mer-1-1140 | KRATMK | 43 |
| 2041 | 6mer-1-841 | KKDAPR | 30 | 2341 | 6mer-1-1141 | KRNMHK | 40 |
| 2042 | 6mer-1-842 | RRFANK | 11 | 2342 | 6mer-1-1142 | KKETPR | 24 |
| 2043 | 6mer-1-843 | KKKTIK | 28 | 2343 | 6mer-1-1143 | KRHEFK | 32 |
| 2044 | 6mer-1-844 | KKPMNR | 28 | 2344 | 6mer-1-1144 | KKCMGR | 25 |
| 2045 | 6mer-1-845 | KKIDKK | 23 | 2345 | 6mer-1-1145 | RRDCKK | 40 |
| 2046 | 6mer-1-846 | RKDAEK | 28 | 2346 | 6mer-1-1146 | KKMMPK | 34 |
| 2047 | 6mer-1-847 | KKYNHK | 20 | 2347 | 6mer-1-1147 | RKAPHR | 35 |
| 2048 | 6mer-1-848 | RKTMFK | 19 | 2348 | 6mer-1-1148 | RRCSHR | 31 |
| 2049 | 6mer-1-849 | KKFNWK | 21 | 2349 | 6mer-1-1149 | RKMTPK | 30 |
| 2050 | 6mer-1-850 | RRNPEK | 29 | 2350 | 6mer-1-1150 | KRKWWR | 34 |
| 2051 | 6mer-1-851 | RRYMEK | 30 | 2351 | 6mer-1-1151 | KKKEAK | 34 |
| 2052 | 6mer-1-852 | KRASVK | 19 | 2352 | 6mer-1-1152 | RKYKEK | 23 |
| 2053 | 6mer-1-853 | KRCNGR | 30 | 2353 | 6mer-1-1153 | RKHNKK | 23 |
| 2054 | 6mer-1-854 | KKEAVR | 16 | 2354 | 6mer-1-1154 | RKCEKR | 41 |
| 2055 | 6mer-1-855 | RKCDVR | 15 | 2355 | 6mer-1-1155 | RKKEKR | 28 |
| 2056 | 6mer-1-856 | KRMWFK | 30 | 2356 | 6mer-1-1156 | KRYTVR | 37 |
| 2057 | 6mer-1-857 | KRPAFK | 14 | 2357 | 6mer-1-1157 | RKESVR | 36 |
| 2058 | 6mer-1-858 | KKKHKR | 27 | 2358 | 6mer-1-1158 | RKPMMK | 33 |
| 2059 | 6mer-1-859 | RKCMVK | 27 | 2359 | 6mer-1-1159 | RRAWPR | 39 |
| 2060 | 6mer-1-860 | RKHWWR | 22 | 2360 | 6mer-1-1160 | RRDWAK | 29 |
| 2061 | 6mer-1-861 | KRINIR | 21 | 2361 | 6mer-1-1161 | KRHWVK | 40 |
| 2062 | 6mer-1-862 | KKHSEK | 32 | 2362 | 6mer-1-1162 | KKCDHR | 41 |
| 2063 | 6mer-1-863 | RRCTER | 27 | 2363 | 6mer-1-1163 | RKCAFK | 21 |
| 2064 | 6mer-1-864 | RRTWGR | 32 | 2364 | 6mer-1-1164 | KKHHAK | 41 |
| 2065 | 6mer-1-865 | KKMTAR | 21 | 2365 | 6mer-1-1165 | RRATWR | 29 |
| 2066 | 6mer-1-866 | KRCHFR | 26 | 2366 | 6mer-1-1166 | KKEEKR | 22 |
| 2067 | 6mer-1-867 | KRIPMR | 25 | 2367 | 6mer-1-1167 | KRHMMK | 42 |
| 2068 | 6mer-1-868 | KKHHAR | 14 | 2368 | 6mer-1-1168 | KKEKFR | 40 |
| 2069 | 6mer-1-869 | KRKDER | 22 | 2369 | 6mer-1-1169 | RKMDIR | 32 |
| 2070 | 6mer-1-870 | KRMSHR | 25 | 2370 | 6mer-1-1170 | KKYNPK | 25 |
| 2071 | 6mer-1-871 | KREWDR | 30 | 2371 | 6mer-1-1171 | RKIAFK | 40 |
| 2072 | 6mer-1-872 | KRPCHR | 23 | 2372 | 6mer-1-1172 | RRFNFR | 23 |
| 2073 | 6mer-1-873 | KRIWAR | 23 | 2373 | 6mer-1-1173 | KKIPMK | 26 |
| 2074 | 6mer-1-874 | KRANHK | 18 | 2374 | 6mer-1-1174 | RKDAIR | 30 |
| 2075 | 6mer-1-875 | KKTNGR | 33 | 2375 | 6mer-1-1175 | RKTWEK | 23 |
| 2076 | 6mer-1-876 | RKYWAR | 19 | 2376 | 6mer-1-1176 | KKADHK | 21 |
| 2077 | 6mer-1-877 | KKPEWR | 19 | 2377 | 6mer-1-1177 | RRHTMR | 21 |
| 2078 | 6mer-1-878 | RRKDNR | 27 | 2378 | 6mer-1-1178 | RKKMAK | 29 |
| 2079 | 6mer-1-879 | RRYNNR | 27 | 2379 | 6mer-1-1179 | KRMTPR | 40 |
| 2080 | 6mer-1-880 | KRPMDK | 21 | 2380 | 6mer-1-1180 | KRPPPK | 34 |
| 2081 | 6mer-1-881 | KKHMER | 20 | 2381 | 6mer-1-1181 | RRMDAK | 21 |
| 2082 | 6mer-1-882 | KKPAMR | 25 | 2382 | 6mer-1-1182 | KKTCHR | 35 |
| 2083 | 6mer-1-883 | KKIDDK | 21 | 2383 | 6mer-1-1183 | RKIHNK | 35 |
| 2084 | 6mer-1-884 | KRYTKR | 26 | 2384 | 6mer-1-1184 | RRDTVK | 30 |
| 2085 | 6mer-1-885 | RRKTPR | 17 | 2385 | 6mer-1-1185 | KKEMMK | 29 |
| 2086 | 6mer-1-886 | RKFSNK | 17 | 2386 | 6mer-1-1186 | KKNNAK | 37 |
| 2087 | 6mer-1-887 | KRINHR | 30 | 2387 | 6mer-1-1187 | RRHAHK | 26 |
| 2088 | 6mer-1-888 | KKTANK | 33 | 2388 | 6mer-1-1188 | RRMNER | 41 |
| 2089 | 6mer-1-889 | KRIMEK | 25 | 2389 | 6mer-1-1189 | KKCHWR | 40 |
| 2090 | 6mer-1-890 | RRTSNK | 18 | 2390 | 6mer-1-1190 | KKCMKR | 29 |
| 2091 | 6mer-1-891 | RKMAFR | 13 | 2391 | 6mer-1-1191 | RKYTKK | 39 |
| 2092 | 6mer-1-892 | RKTEKR | 20 | 2392 | 6mer-1-1192 | RKDDDR | 36 |
| 2093 | 6mer-1-893 | RRIPHR | 22 | 2393 | 6mer-1-1193 | KKPTNK | 33 |
| 2094 | 6mer-1-894 | RKCWWR | 18 | 2394 | 6mer-1-1194 | KKTHAK | 22 |
| 2095 | 6mer-1-895 | KKDSKK | 28 | 2395 | 6mer-1-1195 | KRDEDK | 25 |
| 2096 | 6mer-1-896 | KRDWIK | 11 | 2396 | 6mer-1-1196 | KKTTGR | 25 |
| 2097 | 6mer-1-897 | RRPSIK | 24 | 2397 | 6mer-1-1197 | KRPMAK | 21 |
| 2098 | 6mer-1-898 | RKATKR | 31 | 2398 | 6mer-1-1198 | RRATER | 28 |
| 2099 | 6mer-1-899 | KRMKWK | 31 | 2399 | 6mer-1-1199 | KKTNKR | 39 |
| 2100 | 6mer-1-900 | RRDTGR | 24 | 2400 | 6mer-1-1200 | RKACEK | 21 |
| Average Binding affinity | | | | | | | 27.17 |

**TABLE 12**

| 6mer-2 Top sequence | | | | | | | |
|---|---|---|---|---|---|---|---|
| SEQ ID NO: | Unique No. | Sequence | Binding affinity | SEQ ID NO: | Unique No. | Sequence | Binding affinity |
| 2401 | 6mer-2-1 | RRRLKK | 131 | 2701 | 6mer-2-301 | RQVRKR | 122 |
| 2402 | 6mer-2-2 | RWYLKR | 139 | 2702 | 6mer-2-302 | KQICKK | 148 |
| 2403 | 6mer-2-3 | KVYSRK | 148 | 2703 | 6mer-2-303 | KVFTKR | 126 |
| 2404 | 6mer-2-4 | RSYYRR | 125 | 2704 | 6mer-2-304 | KWFYRK | 144 |
| 2405 | 6mer-2-5 | RWVSKR | 124 | 2705 | 6mer-2-305 | KQFYKK | 154 |
| 2406 | 6mer-2-6 | KSICRR | 122 | 2706 | 6mer-2-306 | RQFQKR | 141 |
| 2407 | 6mer-2-7 | KSRCRK | 126 | 2707 | 6mer-2-307 | RLRSKK | 141 |
| 2408 | 6mer-2-8 | KSLQKK | 135 | 2708 | 6mer-2-308 | KGISKR | 148 |
| 2409 | 6mer-2-9 | KLICRK | 139 | 2709 | 6mer-2-309 | KRRRRR | 151 |
| 2410 | 6mer-2-10 | KVFSKK | 124 | 2710 | 6mer-2-310 | KLFQRK | 149 |
| 2411 | 6mer-2-11 | RQYQRR | 121 | 2711 | 6mer-2-311 | KWTRK | 147 |
| 2412 | 6mer-2-12 | RQFQRK | 147 | 2712 | 6mer-2-312 | KWRTKR | 121 |
| 2413 | 6mer-2-13 | RQYTRK | 123 | 2713 | 6mer-2-313 | RWRTKR | 154 |
| 2414 | 6mer-2-14 | RGRQKK | 153 | 2714 | 6mer-2-314 | KQYCKK | 127 |
| 2415 | 6mer-2-15 | KQVRKR | 133 | 2715 | 6mer-2-315 | RRQRRR | 139 |
| 2416 | 6mer-2-16 | KGVCRR | 121 | 2716 | 6mer-2-316 | KGIRRR | 139 |
| 2417 | 6mer-2-17 | RWVTRR | 142 | 2717 | 6mer-2-317 | RGLYKR | 135 |
| 2418 | 6mer-2-18 | RSILRR | 124 | 2718 | 6mer-2-318 | RGIQKK | 145 |
| 2419 | 6mer-2-19 | RSFSKK | 135 | 2719 | 6mer-2-319 | RSRQRK | 137 |
| 2420 | 6mer-2-20 | RVQTRR | 137 | 2720 | 6mer-2-320 | RQYRRK | 148 |
| 2421 | 6mer-2-21 | RLFTRK | 141 | 2721 | 6mer-2-321 | KLVSRK | 139 |
| 2422 | 6mer-2-22 | RQIRKK | 124 | 2722 | 6mer-2-322 | RRYSRR | 148 |
| 2423 | 6mer-2-23 | RWVRKK | 148 | 2723 | 6mer-2-323 | KVLLRK | 145 |
| 2424 | 6mer-2-24 | KGIQRK | 141 | 2724 | 6mer-2-324 | KLQSRK | 139 |
| 2425 | 6mer-2-25 | KQVQKR | 148 | 2725 | 6mer-2-325 | RVFSRK | 142 |
| 2426 | 6mer-2-26 | RWRRKK | 121 | 2726 | 6mer-2-326 | RGFYKK | 142 |
| 2427 | 6mer-2-27 | KVIRRK | 128 | 2727 | 6mer-2-327 | KGLRKR | 153 |
| 2428 | 6mer-2-28 | KVLSRK | 142 | 2728 | 6mer-2-328 | KRYQRK | 131 |
| 2429 | 6mer-2-29 | RSQRKR | 142 | 2729 | 6mer-2-329 | RVYTKR | 142 |
| 2430 | 6mer-2-30 | RQFYKK | 139 | 2730 | 6mer-2-330 | KQLRKK | 139 |
| 2431 | 6mer-2-31 | KSYQRK | 121 | 2731 | 6mer-2-331 | RWVSKK | 148 |
| 2432 | 6mer-2-32 | RRLCRK | 139 | 2732 | 6mer-2-332 | RRQYKK | 145 |
| 2433 | 6mer-2-33 | RQFRKK | 142 | 2733 | 6mer-2-333 | KRFRRK | 139 |
| 2434 | 6mer-2-34 | KVFRRK | 124 | 2734 | 6mer-2-334 | KVILRR | 146 |
| 2435 | 6mer-2-35 | RWISRK | 148 | 2735 | 6mer-2-335 | KWRSRR | 153 |
| 2436 | 6mer-2-36 | RRLQKK | 131 | 2736 | 6mer-2-336 | KLITKR | 135 |
| 2437 | 6mer-2-37 | KLLYRK | 148 | 2737 | 6mer-2-337 | KQFQKK | 142 |
| 2438 | 6mer-2-38 | RSYYKR | 136 | 2738 | 6mer-2-338 | RSVQKR | 145 |
| 2439 | 6mer-2-39 | RWRYRK | 137 | 2739 | 6mer-2-339 | KQVYKK | 151 |
| 2440 | 6mer-2-40 | RRYRKK | 153 | 2740 | 6mer-2-340 | RRLTRR | 122 |
| 2441 | 6mer-2-41 | KQLCKR | 142 | 2741 | 6mer-2-341 | KWTKR | 144 |
| 2442 | 6mer-2-42 | KLLSKR | 126 | 2742 | 6mer-2-342 | KRYRKK | 145 |
| 2443 | 6mer-2-43 | RVITKR | 146 | 2743 | 6mer-2-343 | KQISKK | 146 |
| 2444 | 6mer-2-44 | KSVRRK | 145 | 2744 | 6mer-2-344 | KGIRRK | 148 |
| 2445 | 6mer-2-45 | RSLTKK | 121 | 2745 | 6mer-2-345 | KSRQKK | 148 |
| 2446 | 6mer-2-46 | KLYLKR | 153 | 2746 | 6mer-2-346 | KRFSKK | 153 |
| 2447 | 6mer-2-47 | KLQSRR | 148 | 2747 | 6mer-2-347 | RRYSKK | 148 |
| 2448 | 6mer-2-48 | RRFRKK | 153 | 2748 | 6mer-2-348 | KSQLRR | 153 |
| 2449 | 6mer-2-49 | RLRCRK | 154 | 2749 | 6mer-2-349 | RRLSRR | 137 |
| 2450 | 6mer-2-50 | RSQSKR | 125 | 2750 | 6mer-2-350 | RGQTRK | 148 |
| 2451 | 6mer-2-51 | KQFLKK | 139 | 2751 | 6mer-2-351 | KLYTKR | 135 |
| 2452 | 6mer-2-52 | KRQRKK | 136 | 2752 | 6mer-2-352 | RWFYRR | 137 |
| 2453 | 6mer-2-53 | KSLSRR | 125 | 2753 | 6mer-2-353 | RLQTKK | 137 |
| 2454 | 6mer-2-54 | KGRRRR | 124 | 2754 | 6mer-2-354 | RWISKK | 135 |
| 2455 | 6mer-2-55 | RLFYKK | 125 | 2755 | 6mer-2-355 | RGIRKK | 124 |
| 2456 | 6mer-2-56 | RSQLRR | 149 | 2756 | 6mer-2-356 | RLLQRR | 145 |
| 2457 | 6mer-2-57 | KQRCRR | 139 | 2757 | 6mer-2-357 | RGQRKK | 151 |
| 2458 | 6mer-2-58 | KRLQRK | 122 | 2758 | 6mer-2-358 | RQIYKK | 154 |
| 2459 | 6mer-2-59 | RVLSRK | 123 | 2759 | 6mer-2-359 | RLFCKK | 136 |
| 2460 | 6mer-2-60 | RRLSKK | 148 | 2760 | 6mer-2-360 | KGVRRR | 141 |
| 2461 | 6mer-2-61 | RGFQRK | 148 | 2761 | 6mer-2-361 | RSFQRR | 153 |
| 2462 | 6mer-2-62 | RLVTKR | 147 | 2762 | 6mer-2-362 | RGRQKR | 154 |
| 2463 | 6mer-2-63 | KSFRKR | 121 | 2763 | 6mer-2-363 | KSIYRK | 147 |
| 2464 | 6mer-2-64 | KSVSKK | 136 | 2764 | 6mer-2-364 | KVFTKK | 154 |
| 2465 | 6mer-2-65 | KGVCKR | 148 | 2765 | 6mer-2-365 | RWRYRR | 121 |
| 2466 | 6mer-2-66 | RVIRRR | 123 | 2766 | 6mer-2-366 | KQYYRR | 142 |
| 2467 | 6mer-2-67 | KLFCKK | 124 | 2767 | 6mer-2-367 | RWRSRR | 142 |
| 2468 | 6mer-2-68 | KGRRRK | 142 | 2768 | 6mer-2-368 | RLRTRR | 142 |
| 2469 | 6mer-2-69 | KWFLRK | 139 | 2769 | 6mer-2-369 | KVVRRR | 145 |
| 2470 | 6mer-2-70 | KRLLRK | 126 | 2770 | 6mer-2-370 | KSLLRR | 142 |
| 2471 | 6mer-2-71 | KQVYKR | 141 | 2771 | 6mer-2-371 | KRLSRR | 148 |
| 2472 | 6mer-2-72 | KRIQKK | 144 | 2772 | 6mer-2-372 | KLVQRR | 141 |
| 2473 | 6mer-2-73 | RLYTKR | 139 | 2773 | 6mer-2-373 | KLIYRK | 139 |
| 2474 | 6mer-2-74 | RWIYKR | 136 | 2774 | 6mer-2-374 | KRQLKK | 125 |
| 2475 | 6mer-2-75 | KLYTRK | 133 | 2775 | 6mer-2-375 | RWQKK | 131 |
| 2476 | 6mer-2-76 | RRQSRK | 142 | 2776 | 6mer-2-376 | KQQCKR | 141 |
| 2477 | 6mer-2-77 | KQQTRR | 123 | 2777 | 6mer-2-377 | RRYCRR | 142 |
| 2478 | 6mer-2-78 | KRYLKR | 135 | 2778 | 6mer-2-378 | KRFQRK | 131 |
| 2479 | 6mer-2-79 | RQLSKR | 135 | 2779 | 6mer-2-379 | RGVQKR | 127 |
| 2480 | 6mer-2-80 | RRQYKR | 148 | 2780 | 6mer-2-380 | RLVYRK | 141 |
| 2481 | 6mer-2-81 | RRVSRR | 121 | 2781 | 6mer-2-381 | KVYRRR | 139 |
| 2482 | 6mer-2-82 | RGVQKK | 124 | 2782 | 6mer-2-382 | KSVSRR | 148 |
| 2483 | 6mer-2-83 | KQYQRR | 135 | 2783 | 6mer-2-383 | KLVRRK | 128 |
| 2484 | 6mer-2-84 | KQRYKK | 139 | 2784 | 6mer-2-384 | RSFQKK | 142 |
| 2485 | 6mer-2-85 | KSQYKR | 137 | 2785 | 6mer-2-385 | RGVCKR | 154 |
| 2486 | 6mer-2-86 | KQFRRK | 126 | 2786 | 6mer-2-386 | KWQSKR | 136 |
| 2487 | 6mer-2-87 | RRLTKR | 142 | 2787 | 6mer-2-387 | KWLCKK | 146 |
| 2488 | 6mer-2-88 | KSFQRR | 141 | 2788 | 6mer-2-388 | KRFQKK | 139 |
| 2489 | 6mer-2-89 | KSRSKK | 141 | 2789 | 6mer-2-389 | KSLYKK | 153 |
| 2490 | 6mer-2-90 | KGRTKR | 133 | 2790 | 6mer-2-390 | KWLRRR | 145 |
| 2491 | 6mer-2-91 | KWIYKR | 121 | 2791 | 6mer-2-391 | KQYQKR | 141 |
| 2492 | 6mer-2-92 | KWCKR | 131 | 2792 | 6mer-2-392 | RQILKR | 149 |
| 2493 | 6mer-2-93 | RWQTRR | 133 | 2793 | 6mer-2-393 | RGLQRK | 145 |
| 2494 | 6mer-2-94 | RLYQRK | 126 | 2794 | 6mer-2-394 | KLVLKR | 141 |
| 2495 | 6mer-2-95 | RRIYRK | 142 | 2795 | 6mer-2-395 | KWRYRR | 148 |
| 2496 | 6mer-2-96 | KWRQRK | 154 | 2796 | 6mer-2-396 | KWLQRR | 122 |
| 2497 | 6mer-2-97 | RQFYRK | 154 | 2797 | 6mer-2-397 | RSIYRK | 154 |
| 2498 | 6mer-2-98 | RRQQRR | 148 | 2798 | 6mer-2-398 | RQQRRR | 141 |
| 2499 | 6mer-2-99 | RQRSRR | 151 | 2799 | 6mer-2-399 | RWLTRR | 148 |
| 2500 | 6mer-2-100 | RRRCRR | 142 | 2800 | 6mer-2-400 | RQIRRR | 154 |
| 2501 | 6mer-2-101 | RLICRK | 154 | 2801 | 6mer-2-401 | RSILRK | 153 |
| 2502 | 6mer-2-102 | KVYQKK | 139 | 2802 | 6mer-2-402 | KRYSRR | 145 |
| 2503 | 6mer-2-103 | RWTKK | 136 | 2803 | 6mer-2-403 | KGVLRK | 123 |
| 2504 | 6mer-2-104 | RGVCRK | 145 | 2804 | 6mer-2-404 | RVLSKR | 121 |
| 2505 | 6mer-2-105 | RGFTRR | 151 | 2805 | 6mer-2-405 | KGYRKK | 137 |
| 2506 | 6mer-2-106 | RWVQRK | 123 | 2806 | 6mer-2-406 | KGIYRR | 139 |
| 2507 | 6mer-2-107 | RGVYRK | 144 | 2807 | 6mer-2-407 | KQVSKR | 122 |
| 2508 | 6mer-2-108 | RWYKK | 153 | 2808 | 6mer-2-408 | KQIYRR | 139 |
| 2509 | 6mer-2-109 | RRVYRK | 144 | 2809 | 6mer-2-409 | KRRRKR | 139 |
| 2510 | 6mer-2-110 | KSQLKK | 153 | 2810 | 6mer-2-410 | KRFRKK | 153 |
| 2511 | 6mer-2-111 | KLIRRK | 123 | 2811 | 6mer-2-411 | RSRQKK | 121 |
| 2512 | 6mer-2-112 | RLQQKR | 142 | 2812 | 6mer-2-412 | KRFLKK | 139 |
| 2513 | 6mer-2-113 | KRYSRK | 145 | 2813 | 6mer-2-413 | RLILKR | 136 |
| 2514 | 6mer-2-114 | KLQYRK | 121 | 2814 | 6mer-2-414 | KWRQKK | 135 |
| 2515 | 6mer-2-115 | KWQYRK | 144 | 2815 | 6mer-2-415 | KGRCKK | 149 |
| 2516 | 6mer-2-116 | KLQRKK | 126 | 2816 | 6mer-2-416 | RWRLRR | 131 |
| 2517 | 6mer-2-117 | KRYTRK | 148 | 2817 | 6mer-2-417 | RWVYKK | 151 |
| 2518 | 6mer-2-118 | KWFSRR | 135 | 2818 | 6mer-2-418 | RVQYKK | 121 |
| 2519 | 6mer-2-119 | RSISKK | 139 | 2819 | 6mer-2-419 | RSRYKK | 154 |
| 2520 | 6mer-2-120 | RRQCRK | 146 | 2820 | 6mer-2-420 | RRQYRK | 128 |
| 2521 | 6mer-2-121 | KRVYRK | 137 | 2821 | 6mer-2-421 | KVICRK | 124 |
| 2522 | 6mer-2-122 | RVITKK | 125 | 2822 | 6mer-2-422 | RLITRR | 154 |
| 2523 | 6mer-2-123 | RQFTKR | 146 | 2823 | 6mer-2-423 | RLQYRK | 154 |
| 2524 | 6mer-2-124 | KWQLKR | 126 | 2824 | 6mer-2-424 | RLYCKK | 139 |
| 2525 | 6mer-2-125 | KVQLRR | 142 | 2825 | 6mer-2-425 | KLQTKK | 145 |
| 2526 | 6mer-2-126 | KWLYRR | 148 | 2826 | 6mer-2-426 | KGQRRK | 141 |
| 2527 | 6mer-2-127 | RLYLRK | 136 | 2827 | 6mer-2-427 | RLQRRR | 147 |
| 2528 | 6mer-2-128 | KSYYRR | 147 | 2828 | 6mer-2-428 | KVFLKK | 142 |
| 2529 | 6mer-2-129 | KSVLRK | 139 | 2829 | 6mer-2-429 | RWRQRK | 123 |
| 2530 | 6mer-2-130 | RGVCRR | 122 | 2830 | 6mer-2-430 | RWFRRR | 139 |
| 2531 | 6mer-2-131 | KQQTKR | 148 | 2831 | 6mer-2-431 | KLILKR | 135 |
| 2532 | 6mer-2-132 | KLYCKK | 139 | 2832 | 6mer-2-432 | KVVRKR | 148 |
| 2533 | 6mer-2-133 | KRRLKK | 145 | 2833 | 6mer-2-433 | RWIQKR | 139 |
| 2534 | 6mer-2-134 | RLFQRK | 148 | 2834 | 6mer-2-434 | RVIRRK | 154 |
| 2535 | 6mer-2-135 | RWFYKK | 124 | 2835 | 6mer-2-435 | RWVYKR | 154 |
| 2536 | 6mer-2-136 | RWRYKR | 194 | 2836 | 6mer-2-436 | KVLYKR | 148 |
| 2537 | 6mer-2-137 | KVFCKK | 135 | 2837 | 6mer-2-437 | KQVCKK | 121 |
| 2538 | 6mer-2-138 | KQRRRK | 153 | 2838 | 6mer-2-438 | RLLCRK | 131 |
| 2539 | 6mer-2-139 | KLFRKK | 149 | 2839 | 6mer-2-439 | KWFSKR | 153 |
| 2540 | 6mer-2-140 | KGRLKK | 142 | 2840 | 6mer-2-440 | KQLSKK | 153 |
| 2541 | 6mer-2-141 | KWYQKK | 154 | 2841 | 6mer-2-441 | KSQRKR | 139 |
| 2542 | 6mer-2-142 | RWVLRR | 121 | 2842 | 6mer-2-442 | RLVQKR | 139 |
| 2543 | 6mer-2-143 | RRVCRR | 128 | 2843 | 6mer-2-443 | RQYCRR | 122 |
| 2544 | 6mer-2-144 | KSIRRK | 133 | 2844 | 6mer-2-444 | RQRLKK | 141 |
| 2545 | 6mer-2-145 | RQYYKK | 128 | 2845 | 6mer-2-445 | RLVCKR | 145 |
| 2546 | 6mer-2-146 | KQLLKR | 135 | 2846 | 6mer-2-446 | RQLQKK | 153 |
| 2547 | 6mer-2-147 | RQYTKR | 133 | 2847 | 6mer-2-447 | RVYTKK | 139 |
| 2548 | 6mer-2-148 | KGQYKK | 145 | 2848 | 6mer-2-448 | RSFRRR | 128 |
| 2549 | 6mer-2-149 | KGQQRK | 127 | 2849 | 6mer-2-449 | RGRCRR | 154 |
| 2550 | 6mer-2-150 | KRILKR | 136 | 2850 | 6mer-2-450 | RQRYRK | 123 |
| 2551 | 6mer-2-151 | KQLRKR | 142 | 2851 | 6mer-2-451 | RGRCKK | 148 |
| 2552 | 6mer-2-152 | RQFYKR | 139 | 2852 | 6mer-2-452 | KGLYKR | 146 |
| 2553 | 6mer-2-153 | RVYSRR | 124 | 2853 | 6mer-2-453 | RLVRKK | 141 |
| 2554 | 6mer-2-154 | KRVTKR | 148 | 2854 | 6mer-2-454 | RSVQKK | 133 |
| 2555 | 6mer-2-155 | KGVYKK | 139 | 2855 | 6mer-2-455 | RWYSKR | 135 |
| 2556 | 6mer-2-156 | KGLQRR | 127 | 2856 | 6mer-2-456 | KSVTKR | 147 |
| 2557 | 6mer-2-157 | RSYRKK | 153 | 2857 | 6mer-2-457 | RRVYKR | 146 |
| 2558 | 6mer-2-158 | RSFLRR | 145 | 2858 | 6mer-2-458 | RRFQRR | 122 |
| 2559 | 6mer-2-159 | RQYYRK | 127 | 2859 | 6mer-2-459 | KLFSRK | 139 |
| 2560 | 6mer-2-160 | RVRRKK | 121 | 2860 | 6mer-2-460 | RSQCRK | 139 |
| 2561 | 6mer-2-161 | KWRRKK | 154 | 2861 | 6mer-2-461 | KSVCRR | 149 |
| 2562 | 6mer-2-162 | KVISKR | 136 | 2862 | 6mer-2-462 | KQICKR | 145 |
| 2563 | 6mer-2-163 | KWFYRR | 149 | 2863 | 6mer-2-463 | KLLTRR | 124 |
| 2564 | 6mer-2-164 | KRQRRK | 135 | 2864 | 6mer-2-464 | KSFRKK | 135 |
| 2565 | 6mer-2-165 | KWFTRR | 139 | 2865 | 6mer-2-465 | KGVSKR | 122 |
| 2566 | 6mer-2-166 | RWLRKR | 125 | 2866 | 6mer-2-466 | RLLTRR | 142 |
| 2567 | 6mer-2-167 | KLFQKR | 145 | 2867 | 6mer-2-467 | KQYSRR | 142 |
| 2568 | 6mer-2-168 | RVFLKR | 135 | 2868 | 6mer-2-468 | RSFSKR | 133 |
| 2569 | 6mer-2-169 | KRQSRR | 123 | 2869 | 6mer-2-469 | RLRTKK | 153 |
| 2570 | 6mer-2-170 | RQYQKK | 128 | 2870 | 6mer-2-470 | RLRRRK | 153 |
| 2571 | 6mer-2-171 | RQQTRR | 139 | 2871 | 6mer-2-471 | KWISRR | 136 |
| 2572 | 6mer-2-172 | KVIRKK | 128 | 2872 | 6mer-2-472 | RSRLRR | 148 |
| 2573 | 6mer-2-173 | RQQCKK | 127 | 2873 | 6mer-2-473 | RGRLRK | 131 |
| 2574 | 6mer-2-174 | KLLRKR | 142 | 2874 | 6mer-2-474 | RWQQRR | 128 |
| 2575 | 6mer-2-175 | KVYLRK | 137 | 2875 | 6mer-2-475 | KRFCRR | 139 |
| 2576 | 6mer-2-176 | RWIYRR | 144 | 2876 | 6mer-2-476 | KGIRKK | 148 |
| 2577 | 6mer-2-177 | RSVTKK | 136 | 2877 | 6mer-2-477 | RSRTRR | 135 |
| 2578 | 6mer-2-178 | KWLLRR | 133 | 2878 | 6mer-2-478 | KWYTRK | 145 |
| 2579 | 6mer-2-179 | RRVYRR | 121 | 2879 | 6mer-2-479 | KWRLKK | 154 |
| 2580 | 6mer-2-180 | RLRLKR | 142 | 2880 | 6mer-2-480 | RRFTKK | 147 |
| 2581 | 6mer-2-181 | RWQQKK | 139 | 2881 | 6mer-2-481 | RQRYKK | 131 |
| 2582 | 6mer-2-182 | KLFYKK | 139 | 2882 | 6mer-2-482 | KGQTRR | 154 |
| 2583 | 6mer-2-183 | RLQLRR | 154 | 2883 | 6mer-2-483 | RRYLKK | 121 |
| 2584 | 6mer-2-184 | KWVSRR | 136 | 2884 | 6mer-2-484 | RWYTKR | 148 |
| 2585 | 6mer-2-185 | RGYQKR | 137 | 2885 | 6mer-2-485 | RQIQKR | 148 |
| 2586 | 6mer-2-186 | KQICRK | 127 | 2886 | 6mer-2-486 | KQQSKK | 121 |
| 2587 | 6mer-2-187 | KRRRRK | 145 | 2887 | 6mer-2-487 | KSYTKR | 121 |
| 2588 | 6mer-2-188 | RGYTKR | 142 | 2888 | 6mer-2-488 | KVYRRK | 146 |
| 2589 | 6mer-2-189 | RWQCKK | 121 | 2889 | 6mer-2-489 | RVISKK | 142 |
| 2590 | 6mer-2-190 | KVYLKR | 148 | 2890 | 6mer-2-490 | RRYQKK | 145 |
| 2591 | 6mer-2-191 | RQLLKR | 154 | 2891 | 6mer-2-491 | KRICKR | 135 |
| 2592 | 6mer-2-192 | RWFSKK | 133 | 2892 | 6mer-2-492 | RLIQKK | 148 |
| 2593 | 6mer-2-193 | KWISKR | 127 | 2893 | 6mer-2-493 | KGVRRK | 123 |
| 2594 | 6mer-2-194 | KLRLRK | 154 | 2894 | 6mer-2-494 | KGLLRR | 144 |
| 2595 | 6mer-2-195 | KSRQKR | 154 | 2895 | 6mer-2-495 | KLLCKK | 121 |
| 2596 | 6mer-2-196 | KSQRKK | 121 | 2896 | 6mer-2-496 | RVRYRK | 144 |
| 2597 | 6mer-2-197 | RGQYRR | 126 | 2897 | 6mer-2-497 | KSYTRR | 148 |
| 2598 | 6mer-2-198 | KWYYRK | 139 | 2898 | 6mer-2-498 | KGLCRR | 127 |
| 2599 | 6mer-2-199 | RGQYKR | 137 | 2899 | 6mer-2-499 | RLFTKR | 145 |
| 2600 | 6mer-2-200 | RGVQRK | 121 | 2900 | 6mer-2-500 | KRICRR | 146 |
| 2601 | 6mer-2-201 | KLFLRR | 148 | 2901 | 6mer-2-501 | KSYSKK | 144 |
| 2602 | 6mer-2-202 | RLIYKK | 122 | 2902 | 6mer-2-502 | KSFCKR | 144 |
| 2603 | 6mer-2-203 | KSRTKR | 135 | 2903 | 6mer-2-503 | KVQTKK | 154 |
| 2604 | 6mer-2-204 | RRVQKR | 142 | 2904 | 6mer-2-504 | RGLTRK | 154 |
| 2605 | 6mer-2-205 | KSFQRK | 147 | 2905 | 6mer-2-505 | KRQCKK | 148 |
| 2606 | 6mer-2-206 | KWQYKK | 148 | 2906 | 6mer-2-506 | KSLLKK | 135 |
| 2607 | 6mer-2-207 | KWVSKR | 153 | 2907 | 6mer-2-507 | RGICKR | 145 |
| 2608 | 6mer-2-208 | RSQQRK | 128 | 2908 | 6mer-2-508 | RVFLKK | 121 |
| 2609 | 6mer-2-209 | RLYYRK | 148 | 2909 | 6mer-2-509 | RGFYRK | 136 |
| 2610 | 6mer-2-210 | RSQSRR | 128 | 2910 | 6mer-2-510 | KRVLKK | 146 |
| 2611 | 6mer-2-211 | RGLSRR | 135 | 2911 | 6mer-2-511 | RVFCRR | 126 |
| 2612 | 6mer-2-212 | KWQLRR | 148 | 2912 | 6mer-2-512 | RWFTRR | 148 |
| 2613 | 6mer-2-213 | KSILRR | 142 | 2913 | 6mer-2-513 | RRLTRK | 121 |
| 2614 | 6mer-2-214 | RWRSRK | 139 | 2914 | 6mer-2-514 | KGLQKK | 124 |
| 2615 | 6mer-2-215 | RLYYRR | 123 | 2915 | 6mer-2-515 | RWYCRK | 123 |
| 2616 | 6mer-2-216 | KGILKK | 126 | 2916 | 6mer-2-516 | RQQQKK | 146 |
| 2617 | 6mer-2-217 | KLFQKK | 135 | 2917 | 6mer-2-517 | KLRTKK | 153 |
| 2618 | 6mer-2-218 | KGVSKK | 147 | 2918 | 6mer-2-518 | KLVTKK | 151 |
| 2619 | 6mer-2-219 | RGVSKR | 153 | 2919 | 6mer-2-519 | KQIQRR | 135 |
| 2620 | 6mer-2-220 | KVYSKR | 135 | 2920 | 6mer-2-520 | KSYCKR | 144 |
| 2621 | 6mer-2-221 | KQVRKK | 153 | 2921 | 6mer-2-521 | KQYTRK | 144 |
| 2622 | 6mer-2-222 | RVVYRK | 127 | 2922 | 6mer-2-522 | RWLRK | 124 |
| 2623 | 6mer-2-223 | KQLTRR | 142 | 2923 | 6mer-2-523 | RGRYKR | 146 |
| 2624 | 6mer-2-224 | RLFLKR | 148 | 2924 | 6mer-2-524 | RGIQKR | 128 |
| 2625 | 6mer-2-225 | KRLLRR | 145 | 2925 | 6mer-2-525 | KWVRKK | 145 |
| 2626 | 6mer-2-226 | RLLCKR | 154 | 2926 | 6mer-2-526 | RVLLRK | 144 |
| 2627 | 6mer-2-227 | KLQRRK | 147 | 2927 | 6mer-2-527 | RGQTRR | 145 |
| 2628 | 6mer-2-228 | KGQCKK | 141 | 2928 | 6mer-2-528 | KGFRKK | 151 |
| 2629 | 6mer-2-229 | RWQSKK | 127 | 2929 | 6mer-2-529 | KQQRKR | 124 |
| 2630 | 6mer-2-230 | RWIQRR | 135 | 2930 | 6mer-2-530 | RSYSKR | 139 |
| 2631 | 6mer-2-231 | KGISKK | 144 | 2931 | 6mer-2-531 | KVIYKR | 148 |
| 2632 | 6mer-2-232 | KQFTRK | 135 | 2932 | 6mer-2-532 | KSITKR | 147 |
| 2633 | 6mer-2-233 | KWRQKR | 190 | 2933 | 6mer-2-533 | KRLLKR | 142 |
| 2634 | 6mer-2-234 | RSQTKR | 142 | 2934 | 6mer-2-534 | RVQLRR | 139 |
| 2635 | 6mer-2-235 | KLVRKK | 136 | 2935 | 6mer-2-535 | KLFCRR | 133 |
| 2636 | 6mer-2-236 | KWQQKK | 131 | 2936 | 6mer-2-536 | RWQRKK | 125 |
| 2637 | 6mer-2-237 | RLVYKR | 123 | 2937 | 6mer-2-537 | KVLCKR | 153 |
| 2638 | 6mer-2-238 | RRRRRK | 142 | 2938 | 6mer-2-538 | KQFCKK | 125 |
| 2639 | 6mer-2-239 | RGQYKK | 125 | 2939 | 6mer-2-539 | KWYTKK | 127 |
| 2640 | 6mer-2-240 | RRRCRK | 142 | 2940 | 6mer-2-540 | KRLTRR | 142 |
| 2641 | 6mer-2-241 | KQLTKR | 135 | 2941 | 6mer-2-541 | KGQCRK | 126 |
| 2642 | 6mer-2-242 | KSQRRR | 135 | 2942 | 6mer-2-542 | RQIYRR | 148 |
| 2643 | 6mer-2-243 | KRVLKR | 148 | 2943 | 6mer-2-543 | RRQYRR | 144 |
| 2644 | 6mer-2-244 | RRFSKR | 139 | 2944 | 6mer-2-544 | KVIQKR | 147 |
| 2645 | 6mer-2-245 | RSLSKR | 135 | 2945 | 6mer-2-545 | KLYCRK | 125 |
| 2646 | 6mer-2-246 | KWISKK | 123 | 2946 | 6mer-2-546 | RVFTKK | 127 |
| 2647 | 6mer-2-247 | KGYQKK | 146 | 2947 | 6mer-2-547 | RWQCKR | 135 |
| 2648 | 6mer-2-248 | RSYTRR | 154 | 2948 | 6mer-2-548 | RLQCRR | 142 |
| 2649 | 6mer-2-249 | RGLRKR | 136 | 2949 | 6mer-2-549 | KQLCKK | 142 |
| 2650 | 6mer-2-250 | KRRRKK | 145 | 2950 | 6mer-2-550 | RSFTKK | 148 |
| 2651 | 6mer-2-251 | KSQTKR | 142 | 2951 | 6mer-2-551 | KWYTKR | 141 |
| 2652 | 6mer-2-252 | KVFRKK | 148 | 2952 | 6mer-2-552 | RGLLRR | 148 |
| 2653 | 6mer-2-253 | KLRSRR | 145 | 2953 | 6mer-2-553 | RSLQKK | 124 |
| 2654 | 6mer-2-254 | KRIQRR | 148 | 2954 | 6mer-2-554 | RWRCRR | 121 |
| 2655 | 6mer-2-255 | RLRRKK | 139 | 2955 | 6mer-2-555 | RSVSKK | 154 |
| 2656 | 6mer-2-256 | KSQQKR | 142 | 2956 | 6mer-2-556 | RSICKK | 148 |
| 2657 | 6mer-2-257 | KRFCRK | 144 | 2957 | 6mer-2-557 | KWVLRK | 123 |
| 2658 | 6mer-2-258 | KRIYRR | 125 | 2958 | 6mer-2-558 | KSVCKK | 154 |
| 2659 | 6mer-2-259 | RVQSKR | 128 | 2959 | 6mer-2-559 | KGQLRR | 121 |
| 2660 | 6mer-2-260 | RRFCKR | 142 | 2960 | 6mer-2-560 | RQYTRR | 153 |
| 2661 | 6mer-2-261 | RWYTKK | 121 | 2961 | 6mer-2-561 | RRVLKK | 142 |
| 2662 | 6mer-2-262 | KWILKR | 142 | 2962 | 6mer-2-562 | RQQRKR | 136 |
| 2663 | 6mer-2-263 | RRICKR | 145 | 2963 | 6mer-2-563 | KGYQKR | 131 |
| 2664 | 6mer-2-264 | KSQLKR | 127 | 2964 | 6mer-2-564 | KQVYRK | 148 |
| 2665 | 6mer-2-265 | RRQTRK | 135 | 2965 | 6mer-2-565 | KSRRRR | 147 |
| 2666 | 6mer-2-266 | RWQTKK | 131 | 2966 | 6mer-2-566 | KRLSKK | 139 |
| 2667 | 6mer-2-267 | KLYSRR | 141 | 2967 | 6mer-2-567 | KLYYRK | 146 |
| 2668 | 6mer-2-268 | RQICKK | 139 | 2968 | 6mer-2-568 | KGFQKK | 142 |
| 2669 | 6mer-2-269 | KWVQRK | 123 | 2969 | 6mer-2-569 | RWQLRK | 135 |
| 2670 | 6mer-2-270 | KSRLRR | 148 | 2970 | 6mer-2-570 | RVYYKR | 148 |
| 2671 | 6mer-2-271 | RLFQKK | 131 | 2971 | 6mer-2-571 | RGYQRR | 135 |
| 2672 | 6mer-2-272 | KWRSRK | 154 | 2972 | 6mer-2-572 | KSQRRK | 148 |
| 2673 | 6mer-2-273 | KLRQRR | 131 | 2973 | 6mer-2-573 | KLYLRR | 121 |
| 2674 | 6mer-2-274 | KLRTKR | 148 | 2974 | 6mer-2-574 | KGVYRK | 153 |
| 2675 | 6mer-2-275 | KLITRR | 135 | 2975 | 6mer-2-575 | RGYSRK | 139 |
| 2676 | 6mer-2-276 | RLVCRK | 153 | 2976 | 6mer-2-576 | KGVSRR | 139 |
| 2677 | 6mer-2-277 | RLRYRK | 128 | 2977 | 6mer-2-577 | KRVQRR | 139 |
| 2678 | 6mer-2-278 | RQIQKK | 135 | 2978 | 6mer-2-578 | RSVLRK | 135 |
| 2679 | 6mer-2-279 | KSLQRK | 148 | 2979 | 6mer-2-579 | RWQYRK | 135 |
| 2680 | 6mer-2-280 | KSIYRR | 153 | 2980 | 6mer-2-580 | KWQQRR | 121 |
| 2681 | 6mer-2-281 | KGQRKK | 149 | 2981 | 6mer-2-581 | RVQQKR | 139 |
| 2682 | 6mer-2-282 | RQQYRK | 151 | 2982 | 6mer-2-582 | RWQTRK | 148 |
| 2683 | 6mer-2-283 | KLISKR | 148 | 2983 | 6mer-2-583 | KSVTKK | 154 |
| 2684 | 6mer-2-284 | KLRSKR | 151 | 2984 | 6mer-2-584 | KRLTRK | 148 |
| 2685 | 6mer-2-285 | RRIQRR | 142 | 2985 | 6mer-2-585 | RSFRRK | 136 |
| 2686 | 6mer-2-286 | KRRYKK | 139 | 2986 | 6mer-2-586 | RWLSRK | 121 |
| 2687 | 6mer-2-287 | RLYLKR | 124 | 2987 | 6mer-2-587 | KRISKR | 148 |
| 2688 | 6mer-2-288 | KSRLRK | 122 | 2988 | 6mer-2-588 | KWFYKK | 149 |
| 2689 | 6mer-2-289 | RLLQKK | 146 | 2989 | 6mer-2-589 | RWQQRK | 153 |
| 2690 | 6mer-2-290 | RQLYRK | 135 | 2990 | 6mer-2-590 | KGRLRK | 148 |
| 2691 | 6mer-2-291 | RRYRKR | 154 | 2991 | 6mer-2-591 | KGILRR | 146 |
| 2692 | 6mer-2-292 | RQLCKR | 121 | 2992 | 6mer-2-592 | RWVLRK | 122 |
| 2693 | 6mer-2-293 | KQQSKR | 153 | 2993 | 6mer-2-593 | RSQLKR | 139 |
| 2694 | 6mer-2-294 | KLYYRR | 135 | 2994 | 6mer-2-594 | RWYRRR | 139 |
| 2695 | 6mer-2-295 | KVRYKK | 145 | 2995 | 6mer-2-595 | KLRCKK | 154 |
| 2696 | 6mer-2-296 | KWYRRK | 135 | 2996 | 6mer-2-596 | KGIQKK | 153 |
| 2697 | 6mer-2-297 | RSLSRR | 139 | 2997 | 6mer-2-597 | KQFLRK | 154 |
| 2698 | 6mer-2-298 | KWICKK | 149 | 2998 | 6mer-2-598 | KVFRRR | 146 |
| 2699 | 6mer-2-299 | RWYYRK | 141 | 2999 | 6mer-2-599 | RRRYRK | 121 |
| 2700 | 6mer-2-300 | RWRLKK | 153 | 3000 | 6mer-2-600 | RSQLKK | 139 |
| Average Binding affinity | | | | | | | 139.09 |

**TABLE 13**

| 6mer-2 Bottom sequence | | | | | | | |
|---|---|---|---|---|---|---|---|
| SEQ ID NO: | Unique No. | Sequence | Binding affinity | SEQ ID NO: | Unique No. | Sequence | Binding affinity |
| 3001 | 6mer-2-601 | KNSHKK | 25 | 3301 | 6mer-2-901 | RYWDRK | 34 |
| 3002 | 6mer-2-602 | RNTPKR | 24 | 3302 | 6mer-2-902 | KPDVRK | 26 |
| 3003 | 6mer-2-603 | KFHARR | 32 | 3303 | 6mer-2-903 | KKNPKK | 40 |
| 3004 | 6mer-2-604 | KEPIRK | 16 | 3304 | 6mer-2-904 | KCCFKK | 30 |
| 3005 | 6mer-2-605 | KNCGKK | 17 | 3305 | 6mer-2-905 | RPKERR | 26 |
| 3006 | 6mer-2-606 | RKPFRR | 15 | 3306 | 6mer-2-906 | KYCDKK | 40 |
| 3007 | 6mer-2-607 | KKWVKR | 31 | 3307 | 6mer-2-907 | RTMNRR | 25 |
| 3008 | 6mer-2-608 | RCNHKK | 32 | 3308 | 6mer-2-908 | KYKKKR | 33 |
| 3009 | 6mer-2-609 | RDSDRR | 24 | 3309 | 6mer-2-909 | RCNPRR | 42 |
| 3010 | 6mer-2-610 | RKNPKR | 32 | 3310 | 6mer-2-910 | RHEHRR | 21 |
| 3011 | 6mer-2-611 | RIKMRR | 22 | 3311 | 6mer-2-911 | KMSGKR | 21 |
| 3012 | 6mer-2-612 | RDCVRK | 17 | 3312 | 6mer-2-912 | RAPIRR | 27 |
| 3013 | 6mer-2-613 | KAANRR | 18 | 3313 | 6mer-2-913 | KFKNRR | 25 |
| 3014 | 6mer-2-614 | KAWIRR | 19 | 3314 | 6mer-2-914 | KDDIKK | 25 |
| 3015 | 6mer-2-615 | KDNGRK | 27 | 3315 | 6mer-2-915 | KNCWKR | 29 |
| 3016 | 6mer-2-616 | REPHRK | 26 | 3316 | 6mer-2-916 | KTTEKR | 22 |
| 3017 | 6mer-2-617 | RCNGRR | 32 | 3317 | 6mer-2-917 | RFCWRK | 23 |
| 3018 | 6mer-2-618 | KHHKKR | 12 | 3318 | 6mer-2-918 | KNWMRK | 35 |
| 3019 | 6mer-2-619 | RPPDRR | 26 | 3319 | 6mer-2-919 | KAKHKR | 27 |
| 3020 | 6mer-2-620 | RHTGRR | 33 | 3320 | 6mer-2-920 | RMTIKK | 26 |
| 3021 | 6mer-2-621 | RHEPKR | 32 | 3321 | 6mer-2-921 | RPKAKK | 22 |
| 3022 | 6mer-2-622 | RYTNKR | 13 | 3322 | 6mer-2-922 | KDNVRR | 41 |
| 3023 | 6mer-2-623 | KHAHRR | 13 | 3323 | 6mer-2-923 | RCCAKK | 33 |
| 3024 | 6mer-2-624 | KPPIRK | 33 | 3324 | 6mer-2-924 | RFDHKK | 28 |
| 3025 | 6mer-2-625 | RFPDKK | 33 | 3325 | 6mer-2-925 | RASVKR | 23 |
| 3026 | 6mer-2-626 | KNSEKK | 33 | 3326 | 6mer-2-926 | RPTVKR | 31 |
| 3027 | 6mer-2-627 | KKTARR | 33 | 3327 | 6mer-2-927 | RFNPRK | 39 |
| 3028 | 6mer-2-628 | RKSFKR | 30 | 3328 | 6mer-2-928 | KFCIRR | 28 |
| 3029 | 6mer-2-629 | RFCWKK | 32 | 3329 | 6mer-2-929 | KNHMRK | 26 |
| 3030 | 6mer-2-630 | RYAFRR | 27 | 3330 | 6mer-2-930 | RTWIKR | 37 |
| 3031 | 6mer-2-631 | KYCMKR | 16 | 3331 | 6mer-2-931 | RYCFKR | 29 |
| 3032 | 6mer-2-632 | KYDIKR | 12 | 3332 | 6mer-2-932 | RIPWRR | 35 |
| 3033 | 6mer-2-633 | KYAVKR | 17 | 3333 | 6mer-2-933 | RYSMKR | 34 |
| 3034 | 6mer-2-634 | RYEHRK | 33 | 3334 | 6mer-2-934 | KYKDRR | 26 |
| 3035 | 6mer-2-635 | KECVRR | 11 | 3335 | 6mer-2-935 | KKCNKK | 22 |
| 3036 | 6mer-2-636 | KMAPKR | 21 | 3336 | 6mer-2-936 | KPPAKK | 21 |
| 3037 | 6mer-2-637 | RPPVRK | 32 | 3337 | 6mer-2-937 | RASHRR | 35 |
| 3038 | 6mer-2-638 | RCWAKR | 30 | 3338 | 6mer-2-938 | RIEHKR | 40 |
| 3039 | 6mer-2-639 | KHTVKK | 23 | 3339 | 6mer-2-939 | KKCARR | 22 |
| 3040 | 6mer-2-640 | KFKKKR | 21 | 3340 | 6mer-2-940 | RNENKK | 41 |
| 3041 | 6mer-2-641 | REEFRR | 12 | 3341 | 6mer-2-941 | RDEDKR | 33 |
| 3042 | 6mer-2-642 | KTENKK | 16 | 3342 | 6mer-2-942 | KTDKRK | 22 |
| 3043 | 6mer-2-643 | KANPKK | 12 | 3343 | 6mer-2-943 | KIDHRR | 41 |
| 3044 | 6mer-2-644 | REPNRR | 12 | 3344 | 6mer-2-944 | KPEDKK | 35 |
| 3045 | 6mer-2-645 | RIKVKK | 15 | 3345 | 6mer-2-945 | KKCERK | 42 |
| 3046 | 6mer-2-646 | RAAPRR | 32 | 3346 | 6mer-2-946 | KPCEKK | 21 |
| 3047 | 6mer-2-647 | KTEPKR | 19 | 3347 | 6mer-2-947 | KTNWRK | 41 |
| 3048 | 6mer-2-648 | RYPDRK | 23 | 3348 | 6mer-2-948 | RPPFRR | 28 |
| 3049 | 6mer-2-649 | KFDVRK | 31 | 3349 | 6mer-2-949 | RPNARR | 39 |
| 3050 | 6mer-2-650 | RFKARK | 18 | 3350 | 6mer-2-950 | KYNKRR | 26 |
| 3051 | 6mer-2-651 | KNADRR | 21 | 3351 | 6mer-2-951 | RADEKK | 42 |
| 3052 | 6mer-2-652 | RTTMRK | 16 | 3352 | 6mer-2-952 | KYWGRR | 24 |
| 3053 | 6mer-2-653 | KIEMKR | 20 | 3353 | 6mer-2-953 | KESVKK | 25 |
| 3054 | 6mer-2-654 | RMPEKR | 20 | 3354 | 6mer-2-954 | KCTIRK | 32 |
| 3055 | 6mer-2-655 | KIWFRR | 31 | 3355 | 6mer-2-955 | RMPERR | 29 |
| 3056 | 6mer-2-656 | KADPRR | 11 | 3356 | 6mer-2-956 | KFCWRR | 28 |
| 3057 | 6mer-2-657 | RKHERK | 32 | 3357 | 6mer-2-957 | RDTHRR | 40 |
| 3058 | 6mer-2-658 | RYWVKK | 23 | 3358 | 6mer-2-958 | KFKARK | 28 |
| 3059 | 6mer-2-659 | KIPNKR | 22 | 3359 | 6mer-2-959 | RTNARK | 35 |
| 3060 | 6mer-2-660 | RHKDRK | 16 | 3360 | 6mer-2-960 | RDCVKR | 29 |
| 3061 | 6mer-2-661 | RMKAKK | 29 | 3361 | 6mer-2-961 | KNTKKR | 21 |
| 3062 | 6mer-2-662 | KAHPKK | 11 | 3362 | 6mer-2-962 | KKSNRK | 30 |
| 3063 | 6mer-2-663 | KFAGRR | 24 | 3363 | 6mer-2-963 | KAKHRR | 37 |
| 3064 | 6mer-2-664 | KCCWKK | 11 | 3364 | 6mer-2-964 | KCEDRK | 41 |
| 3065 | 6mer-2-665 | KCAERR | 18 | 3365 | 6mer-2-965 | KPWFKR | 36 |
| 3066 | 6mer-2-666 | KPMDRR | 25 | 3366 | 6mer-2-966 | KEDAKR | 41 |
| 3067 | 6mer-2-667 | KHDMRR | 25 | 3367 | 6mer-2-967 | RTSNKK | 35 |
| 3068 | 6mer-2-668 | RFCNKK | 13 | 3368 | 6mer-2-968 | RHWEKK | 39 |
| 3069 | 6mer-2-669 | KNMWRK | 15 | 3369 | 6mer-2-969 | KATHKR | 33 |
| 3070 | 6mer-2-670 | RKWHKR | 33 | 3370 | 6mer-2-970 | KDDVRR | 27 |
| 3071 | 6mer-2-671 | RDWIRR | 27 | 3371 | 6mer-2-971 | KEMFRK | 29 |
| 3072 | 6mer-2-672 | KIDDRK | 20 | 3372 | 6mer-2-972 | KAMFRR | 38 |
| 3073 | 6mer-2-673 | RFCGRK | 23 | 3373 | 6mer-2-973 | KCPIKK | 41 |
| 3074 | 6mer-2-674 | KKWERK | 26 | 3374 | 6mer-2-974 | RFDDRR | 38 |
| 3075 | 6mer-2-675 | RMKWRK | 14 | 3375 | 6mer-2-975 | KKTMKK | 38 |
| 3076 | 6mer-2-676 | KCTAKK | 14 | 3376 | 6mer-2-976 | RTTGKK | 26 |
| 3077 | 6mer-2-677 | RENWKK | 28 | 3377 | 6mer-2-977 | REMKRK | 30 |
| 3078 | 6mer-2-678 | KEWWRR | 32 | 3378 | 6mer-2-978 | KEPHRR | 28 |
| 3079 | 6mer-2-679 | RYSAKK | 20 | 3379 | 6mer-2-979 | KKAFKK | 22 |
| 3080 | 6mer-2-680 | KNHGKR | 15 | 3380 | 6mer-2-980 | KESGKR | 34 |
| 3081 | 6mer-2-681 | RDTWRR | 33 | 3381 | 6mer-2-981 | RCEIRK | 32 |
| 3082 | 6mer-2-682 | RNMERR | 33 | 3382 | 6mer-2-982 | RMHAKK | 38 |
| 3083 | 6mer-2-683 | KTEIRK | 33 | 3383 | 6mer-2-983 | RTWHRR | 37 |
| 3084 | 6mer-2-684 | RYTMKK | 14 | 3384 | 6mer-2-984 | KDMWKK | 43 |
| 3085 | 6mer-2-685 | KFHNRK | 15 | 3385 | 6mer-2-985 | REMNKR | 34 |
| 3086 | 6mer-2-686 | KTWEKK | 21 | 3386 | 6mer-2-986 | KNWARR | 27 |
| 3087 | 6mer-2-687 | KADGRK | 16 | 3387 | 6mer-2-987 | RCHPKK | 22 |
| 3088 | 6mer-2-688 | RPMFRK | 13 | 3388 | 6mer-2-988 | KMCEKK | 41 |
| 3089 | 6mer-2-689 | RDKERR | 24 | 3389 | 6mer-2-989 | KAHNKR | 22 |
| 3090 | 6mer-2-690 | KFTMRK | 13 | 3390 | 6mer-2-990 | RKKMKR | 41 |
| 3091 | 6mer-2-691 | RHKMRK | 15 | 3391 | 6mer-2-991 | KCHFRR | 30 |
| 3092 | 6mer-2-692 | KYTNRR | 29 | 3392 | 6mer-2-992 | RMHFRR | 33 |
| 3093 | 6mer-2-693 | KNTIRR | 16 | 3393 | 6mer-2-993 | RHDVKK | 29 |
| 3094 | 6mer-2-694 | KDSIRK | 22 | 3394 | 6mer-2-994 | KYCIKK | 41 |
| 3095 | 6mer-2-695 | KCWWRR | 28 | 3395 | 6mer-2-995 | KFWEKK | 38 |
| 3096 | 6mer-2-696 | KFTKKK | 20 | 3396 | 6mer-2-996 | RHDARK | 40 |
| 3097 | 6mer-2-697 | KCAVKK | 19 | 3397 | 6mer-2-997 | KISFKK | 30 |
| 3098 | 6mer-2-698 | KCEDKK | 27 | 3398 | 6mer-2-998 | KEMWKK | 43 |
| 3099 | 6mer-2-699 | KNAGKR | 23 | 3399 | 6mer-2-999 | RATIKR | 31 |
| 3100 | 6mer-2-700 | RPWHKK | 15 | 3400 | 6mer-2-1000 | KCHDKK | 41 |
| 3101 | 6mer-2-701 | KTWAKR | 23 | 3401 | 6mer-2-1001 | RKKFKK | 37 |
| 3102 | 6mer-2-702 | KEMIKR | 18 | 3402 | 6mer-2-1002 | RCNDKR | 42 |
| 3103 | 6mer-2-703 | RTMHKR | 14 | 3403 | 6mer-2-1003 | KPEWKR | 42 |
| 3104 | 6mer-2-704 | KDWHRK | 20 | 3404 | 6mer-2-1004 | RDTHKR | 38 |
| 3105 | 6mer-2-705 | RENFKK | 11 | 3405 | 6mer-2-1005 | RFKARR | 23 |
| 3106 | 6mer-2-706 | KFTDRK | 23 | 3406 | 6mer-2-1006 | RATMRR | 30 |
| 3107 | 6mer-2-707 | RTWKRK | 21 | 3407 | 6mer-2-1007 | RHTNKK | 42 |
| 3108 | 6mer-2-708 | RAWVKK | 29 | 3408 | 6mer-2-1008 | RAWKKR | 38 |
| 3109 | 6mer-2-709 | RIWPKR | 31 | 3409 | 6mer-2-1009 | KINPRK | 34 |
| 3110 | 6mer-2-710 | RNKKKK | 14 | 3410 | 6mer-2-1010 | RANHKK | 36 |
| 3111 | 6mer-2-711 | KECGRR | 29 | 3411 | 6mer-2-1011 | RAEFRR | 22 |
| 3112 | 6mer-2-712 | KIPHRR | 29 | 3412 | 6mer-2-1012 | KASAKK | 30 |
| 3113 | 6mer-2-713 | RASWRR | 24 | 3413 | 6mer-2-1013 | KPPDKR | 23 |
| 3114 | 6mer-2-714 | KIDVRK | 30 | 3414 | 6mer-2-1014 | RPPMRR | 34 |
| 3115 | 6mer-2-715 | RATHRK | 32 | 3415 | 6mer-2-1015 | RDEMKR | 29 |
| 3116 | 6mer-2-716 | RTWKKR | 18 | 3416 | 6mer-2-1016 | RNEEKR | 41 |
| 3117 | 6mer-2-717 | KCPGKR | 15 | 3417 | 6mer-2-1017 | RAPVRR | 40 |
| 3118 | 6mer-2-718 | KYTFKR | 12 | 3418 | 6mer-2-1018 | RPTKKK | 24 |
| 3119 | 6mer-2-719 | KIKPRR | 29 | 3419 | 6mer-2-1019 | RNTWKK | 23 |
| 3120 | 6mer-2-720 | RATNRK | 29 | 3420 | 6mer-2-1020 | KHDKKR | 21 |
| 3121 | 6mer-2-721 | RHSKKK | 26 | 3421 | 6mer-2-1021 | RYTPKR | 39 |
| 3122 | 6mer-2-722 | RNEHRR | 21 | 3422 | 6mer-2-1022 | KCNPRR | 22 |
| 3123 | 6mer-2-723 | KEDMKR | 23 | 3423 | 6mer-2-1023 | KAEFKR | 26 |
| 3124 | 6mer-2-724 | KYTMKK | 24 | 3424 | 6mer-2-1024 | RFHNKR | 37 |
| 3125 | 6mer-2-725 | KAHMKK | 25 | 3425 | 6mer-2-1025 | RCSHRR | 39 |
| 3126 | 6mer-2-726 | KHHKKK | 25 | 3426 | 6mer-2-1026 | KENHRK | 33 |
| 3127 | 6mer-2-727 | RDMIKR | 11 | 3427 | 6mer-2-1027 | KTPVKK | 32 |
| 3128 | 6mer-2-728 | RMTMKK | 23 | 3428 | 6mer-2-1028 | KPEHRR | 41 |
| 3129 | 6mer-2-729 | RCPDKR | 30 | 3429 | 6mer-2-1029 | KEEFKK | 40 |
| 3130 | 6mer-2-730 | RNTHKR | 26 | 3430 | 6mer-2-1030 | RAPFKK | 28 |
| 3131 | 6mer-2-731 | KASWKK | 19 | 3431 | 6mer-2-1031 | RAWERK | 33 |
| 3132 | 6mer-2-732 | KPTWRK | 24 | 3432 | 6mer-2-1032 | KETVKR | 31 |
| 3133 | 6mer-2-733 | RTPARK | 29 | 3433 | 6mer-2-1033 | KMDEKK | 28 |
| 3134 | 6mer-2-734 | KHCHRR | 17 | 3434 | 6mer-2-1034 | KHSWRR | 38 |
| 3135 | 6mer-2-735 | KMNAKK | 21 | 3435 | 6mer-2-1035 | RPPVKK | 39 |
| 3136 | 6mer-2-736 | KNNHKK | 29 | 3436 | 6mer-2-1036 | KAKNKR | 25 |
| 3137 | 6mer-2-737 | KDSVRR | 19 | 3437 | 6mer-2-1037 | KIHWKK | 26 |
| 3138 | 6mer-2-738 | KPWFKK | 29 | 3438 | 6mer-2-1038 | KHWERK | 33 |
| 3139 | 6mer-2-739 | RIHAKK | 17 | 3439 | 6mer-2-1039 | RNHFRK | 26 |
| 3140 | 6mer-2-740 | KIDIRR | 12 | 3440 | 6mer-2-1040 | KYAWRK | 36 |
| 3141 | 6mer-2-741 | KNEKKR | 30 | 3441 | 6mer-2-1041 | RACDKR | 22 |
| 3142 | 6mer-2-742 | KNNWRR | 32 | 3442 | 6mer-2-1042 | KKMFRK | 36 |
| 3143 | 6mer-2-743 | KHTMKR | 18 | 3443 | 6mer-2-1043 | RDKGKK | 40 |
| 3144 | 6mer-2-744 | KDHPRK | 19 | 3444 | 6mer-2-1044 | KHTDKK | 41 |
| 3145 | 6mer-2-745 | KNTERR | 17 | 3445 | 6mer-2-1045 | RHAHRR | 41 |
| 3146 | 6mer-2-746 | RIKVRK | 26 | 3446 | 6mer-2-1046 | RNSEKR | 39 |
| 3147 | 6mer-2-747 | RANFRR | 29 | 3447 | 6mer-2-1047 | RHDDKR | 28 |
| 3148 | 6mer-2-748 | RFDKKR | 28 | 3448 | 6mer-2-1048 | RIHNRK | 22 |
| 3149 | 6mer-2-749 | KMWKRK | 20 | 3449 | 6mer-2-1049 | KMSPRR | 26 |
| 3150 | 6mer-2-750 | RKCVKR | 29 | 3450 | 6mer-2-1050 | KPPPRK | 26 |
| 3151 | 6mer-2-751 | RPHWKR | 12 | 3451 | 6mer-2-1051 | RKHMRR | 24 |
| 3152 | 6mer-2-752 | KINWRR | 29 | 3452 | 6mer-2-1052 | KAPARK | 23 |
| 3153 | 6mer-2-753 | RYEHRR | 18 | 3453 | 6mer-2-1053 | KEMGKK | 34 |
| 3154 | 6mer-2-754 | KADGRR | 12 | 3454 | 6mer-2-1054 | KKNKKR | 43 |
| 3155 | 6mer-2-755 | RYNDKK | 18 | 3455 | 6mer-2-1055 | KPDFKR | 30 |
| 3156 | 6mer-2-756 | KPTNRK | 26 | 3456 | 6mer-2-1056 | RNDERK | 22 |
| 3157 | 6mer-2-757 | RIPNKR | 19 | 3457 | 6mer-2-1057 | RKKKKK | 25 |
| 3158 | 6mer-2-758 | RFSFKK | 18 | 3458 | 6mer-2-1058 | RMPWKK | 24 |
| 3159 | 6mer-2-759 | RFDEKK | 18 | 3459 | 6mer-2-1059 | RFADRR | 35 |
| 3160 | 6mer-2-760 | KIPVKK | 15 | 3460 | 6mer-2-1060 | KTNERK | 35 |
| 3161 | 6mer-2-761 | RTEPRK | 29 | 3461 | 6mer-2-1061 | KPPIKK | 38 |
| 3162 | 6mer-2-762 | KFEERK | 24 | 3462 | 6mer-2-1062 | KYNIRK | 35 |
| 3163 | 6mer-2-763 | KMKERR | 19 | 3463 | 6mer-2-1063 | RFNVRK | 24 |
| 3164 | 6mer-2-764 | RNAVKK | 31 | 3464 | 6mer-2-1064 | RCMKRR | 39 |
| 3165 | 6mer-2-765 | KHSPRK | 23 | 3465 | 6mer-2-1065 | RIANRK | 27 |
| 3166 | 6mer-2-766 | RPTFRK | 23 | 3466 | 6mer-2-1066 | KPEHKR | 29 |
| 3167 | 6mer-2-767 | RICGRK | 13 | 3467 | 6mer-2-1067 | RDEIRK | 29 |
| 3168 | 6mer-2-768 | KYMGRK | 22 | 3468 | 6mer-2-1068 | RIHVKK | 26 |
| 3169 | 6mer-2-769 | RCPFRK | 13 | 3469 | 6mer-2-1069 | KHDMKK | 36 |
| 3170 | 6mer-2-770 | KPNMKK | 24 | 3470 | 6mer-2-1070 | RPHVRR | 31 |
| 3171 | 6mer-2-771 | KITARR | 26 | 3471 | 6mer-2-1071 | RTTWRK | 25 |
| 3172 | 6mer-2-772 | RHPAKK | 15 | 3472 | 6mer-2-1072 | KPHWKR | 29 |
| 3173 | 6mer-2-773 | KPDARR | 11 | 3473 | 6mer-2-1073 | KYSWRK | 24 |
| 3174 | 6mer-2-774 | KFDARK | 17 | 3474 | 6mer-2-1074 | RPMGRK | 33 |
| 3175 | 6mer-2-775 | KMSMKK | 26 | 3475 | 6mer-2-1075 | RHMERK | 31 |
| 3176 | 6mer-2-776 | RFPWRK | 12 | 3476 | 6mer-2-1076 | RYCIRR | 34 |
| 3177 | 6mer-2-777 | KNDVRR | 31 | 3477 | 6mer-2-1077 | KMTIKK | 34 |
| 3178 | 6mer-2-778 | RNAPKK | 21 | 3478 | 6mer-2-1078 | KDTFRR | 40 |
| 3179 | 6mer-2-779 | KAAHRR | 13 | 3479 | 6mer-2-1079 | KCNEKK | 40 |
| 3180 | 6mer-2-780 | KITIRK | 31 | 3480 | 6mer-2-1080 | RKHKKK | 23 |
| 3181 | 6mer-2-781 | KTMNRK | 28 | 3481 | 6mer-2-1081 | KYKMRR | 32 |
| 3182 | 6mer-2-782 | KPWPKR | 18 | 3482 | 6mer-2-1082 | KYTERR | 31 |
| 3183 | 6mer-2-783 | RDMVRK | 32 | 3483 | 6mer-2-1083 | KTKGKR | 39 |
| 3184 | 6mer-2-784 | KAEWRK | 26 | 3484 | 6mer-2-1084 | REKPRK | 29 |
| 3185 | 6mer-2-785 | RHCHRR | 31 | 3485 | 6mer-2-1085 | RPEIRK | 42 |
| 3186 | 6mer-2-786 | RMEMRK | 30 | 3486 | 6mer-2-1086 | KNDWRK | 21 |
| 3187 | 6mer-2-787 | RIWHKK | 19 | 3487 | 6mer-2-1087 | KEEMRR | 21 |
| 3188 | 6mer-2-788 | KKEKKK | 30 | 3488 | 6mer-2-1088 | RPCEKK | 39 |
| 3189 | 6mer-2-789 | KNENRK | 24 | 3489 | 6mer-2-1089 | RKTDRR | 43 |
| 3190 | 6mer-2-790 | RCAGRR | 23 | 3490 | 6mer-2-1090 | KEMHKK | 33 |
| 3191 | 6mer-2-791 | KNMNRK | 27 | 3491 | 6mer-2-1091 | RFDNKR | 31 |
| 3192 | 6mer-2-792 | RAEDKR | 13 | 3492 | 6mer-2-1092 | RAKFRR | 21 |
| 3193 | 6mer-2-793 | RMHKKR | 29 | 3493 | 6mer-2-1093 | KTTDKK | 43 |
| 3194 | 6mer-2-794 | KAEMRR | 19 | 3494 | 6mer-2-1094 | KAWERK | 43 |
| 3195 | 6mer-2-795 | RTHNKR | 16 | 3495 | 6mer-2-1095 | KYSMRR | 39 |
| 3196 | 6mer-2-796 | RMMARR | 32 | 3496 | 6mer-2-1096 | RANVKR | 39 |
| 3197 | 6mer-2-797 | RHCIKR | 19 | 3497 | 6mer-2-1097 | RFWEKK | 23 |
| 3198 | 6mer-2-798 | RDTPKK | 17 | 3498 | 6mer-2-1098 | KHPKRR | 28 |
| 3199 | 6mer-2-799 | KCSAKK | 13 | 3499 | 6mer-2-1099 | REDWRR | 21 |
| 3200 | 6mer-2-800 | RTAWKK | 11 | 3500 | 6mer-2-1100 | RTAPKK | 24 |
| 3201 | 6mer-2-801 | RPNIRK | 24 | 3501 | 6mer-2-1101 | RTKMRR | 38 |
| 3202 | 6mer-2-802 | KETKRR | 24 | 3502 | 6mer-2-1102 | KHKGKR | 32 |
| 3203 | 6mer-2-803 | RNKERK | 18 | 3503 | 6mer-2-1103 | RDAWRK | 42 |
| 3204 | 6mer-2-804 | RNCGKR | 21 | 3504 | 6mer-2-1104 | RYTKKR | 38 |
| 3205 | 6mer-2-805 | RDTMKK | 12 | 3505 | 6mer-2-1105 | RNNAKK | 28 |
| 3206 | 6mer-2-806 | KHCVRK | 30 | 3506 | 6mer-2-1106 | KDKVKR | 28 |
| 3207 | 6mer-2-807 | KIWNRK | 28 | 3507 | 6mer-2-1107 | RAHERK | 29 |
| 3208 | 6mer-2-808 | RKKFKK | 21 | 3508 | 6mer-2-1108 | KKKVRR | 36 |
| 3209 | 6mer-2-809 | REPWRR | 18 | 3509 | 6mer-2-1109 | KEDMRK | 26 |
| 3210 | 6mer-2-810 | KHAKKK | 18 | 3510 | 6mer-2-1110 | RMTKKK | 37 |
| 3211 | 6mer-2-811 | KAMKRK | 16 | 3511 | 6mer-2-1111 | REDEKK | 23 |
| 3212 | 6mer-2-812 | KPPEKR | 33 | 3512 | 6mer-2-1112 | RASWRK | 26 |
| 3213 | 6mer-2-813 | KKMDRR | 29 | 3513 | 6mer-2-1113 | KPNFRK | 43 |
| 3214 | 6mer-2-814 | RYMERK | 28 | 3514 | 6mer-2-1114 | RKKFRK | 34 |
| 3215 | 6mer-2-815 | KICGRK | 30 | 3515 | 6mer-2-1115 | KYSPRK | 39 |
| 3216 | 6mer-2-816 | RKMGKR | 15 | 3516 | 6mer-2-1116 | KMEVKK | 31 |
| 3217 | 6mer-2-817 | KCNDKK | 22 | 3517 | 6mer-2-1117 | RMHVKR | 21 |
| 3218 | 6mer-2-818 | KTPERK | 13 | 3518 | 6mer-2-1118 | RFDGRR | 42 |
| 3219 | 6mer-2-819 | KFSVRR | 23 | 3519 | 6mer-2-1119 | KCTVRK | 39 |
| 3220 | 6mer-2-820 | RPNNKK | 24 | 3520 | 6mer-2-1120 | RDMMKK | 28 |
| 3221 | 6mer-2-821 | RPTMKR | 33 | 3521 | 6mer-2-1121 | KPSKRR | 21 |
| 3222 | 6mer-2-822 | RCTAKK | 13 | 3522 | 6mer-2-1122 | RCNFKK | 22 |
| 3223 | 6mer-2-823 | REDWRK | 25 | 3523 | 6mer-2-1123 | RITARR | 36 |
| 3224 | 6mer-2-824 | RITNKR | 26 | 3524 | 6mer-2-1124 | RKTWRK | 39 |
| 3225 | 6mer-2-825 | KIHARK | 11 | 3525 | 6mer-2-1125 | RDAFKK | 30 |
| 3226 | 6mer-2-826 | RNTPRK | 11 | 3526 | 6mer-2-1126 | RHEVRR | 23 |
| 3227 | 6mer-2-827 | KYAEKK | 13 | 3527 | 6mer-2-1127 | KAAEKK | 42 |
| 3228 | 6mer-2-828 | RMKKKR | 32 | 3528 | 6mer-2-1128 | KNCMRK | 36 |
| 3229 | 6mer-2-829 | KKKIRR | 22 | 3529 | 6mer-2-1129 | RITGKR | 42 |
| 3230 | 6mer-2-830 | RMKFKR | 21 | 3530 | 6mer-2-1130 | RHSFRK | 29 |
| 3231 | 6mer-2-831 | RMTWRK | 29 | 3531 | 6mer-2-1131 | RTNAKR | 26 |
| 3232 | 6mer-2-832 | KYHNRR | 32 | 3532 | 6mer-2-1132 | RPWERR | 32 |
| 3233 | 6mer-2-833 | RKTWRR | 14 | 3533 | 6mer-2-1133 | KMKMKK | 22 |
| 3234 | 6mer-2-834 | RFWWKR | 28 | 3534 | 6mer-2-1134 | KYENRK | 30 |
| 3235 | 6mer-2-835 | RHCGKR | 20 | 3535 | 6mer-2-1135 | RANWKR | 29 |
| 3236 | 6mer-2-836 | KNTMKR | 26 | 3536 | 6mer-2-1136 | KMKKKR | 36 |
| 3237 | 6mer-2-837 | REDFRR | 32 | 3537 | 6mer-2-1137 | RTDFKR | 22 |
| 3238 | 6mer-2-838 | RCTVRK | 13 | 3538 | 6mer-2-1138 | KAHFKK | 42 |
| 3239 | 6mer-2-839 | KNHNRR | 32 | 3539 | 6mer-2-1139 | KAKAKK | 39 |
| 3240 | 6mer-2-840 | KDWDRK | 15 | 3540 | 6mer-2-1140 | KMNKKR | 29 |
| 3241 | 6mer-2-841 | KPNHRK | 33 | 3541 | 6mer-2-1141 | KCTGKR | 32 |
| 3242 | 6mer-2-842 | KPKMRR | 22 | 3542 | 6mer-2-1142 | KKAMKR | 30 |
| 3243 | 6mer-2-843 | KAPDRK | 33 | 3543 | 6mer-2-1143 | KACIKK | 42 |
| 3244 | 6mer-2-844 | RDPKKK | 22 | 3544 | 6mer-2-1144 | RFEDRK | 39 |
| 3245 | 6mer-2-845 | KMKEKR | 14 | 3545 | 6mer-2-1145 | RIKNRR | 23 |
| 3246 | 6mer-2-846 | RFSFKR | 30 | 3546 | 6mer-2-1146 | REWGRK | 32 |
| 3247 | 6mer-2-847 | RYCHKR | 22 | 3547 | 6mer-2-1147 | KFMKKK | 35 |
| 3248 | 6mer-2-848 | RMTERK | 29 | 3548 | 6mer-2-1148 | KEKGKR | 34 |
| 3249 | 6mer-2-849 | RNTAKK | 13 | 3549 | 6mer-2-1149 | KIDFRR | 21 |
| 3250 | 6mer-2-850 | KTSMKR | 16 | 3550 | 6mer-2-1150 | KTDVKK | 42 |
| 3251 | 6mer-2-851 | KFWWRR | 19 | 3551 | 6mer-2-1151 | RYPVRK | 43 |
| 3252 | 6mer-2-852 | RNCERR | 28 | 3552 | 6mer-2-1152 | RNWAKK | 37 |
| 3253 | 6mer-2-853 | KHTGRR | 29 | 3553 | 6mer-2-1153 | KHKPKR | 27 |
| 3254 | 6mer-2-854 | RCNWKR | 23 | 3554 | 6mer-2-1154 | RYEARR | 30 |
| 3255 | 6mer-2-855 | REMIKR | 19 | 3555 | 6mer-2-1155 | RNTNRK | 25 |
| 3256 | 6mer-2-856 | KEPMRR | 14 | 3556 | 6mer-2-1156 | KMNARR | 35 |
| 3257 | 6mer-2-857 | KHTEKK | 20 | 3557 | 6mer-2-1157 | RNTDRK | 37 |
| 3258 | 6mer-2-858 | KEEHRR | 30 | 3558 | 6mer-2-1158 | RDNIRK | 37 |
| 3259 | 6mer-2-859 | RMDFRK | 21 | 3559 | 6mer-2-1159 | KDMGKR | 40 |
| 3260 | 6mer-2-860 | KPNFRR | 31 | 3560 | 6mer-2-1160 | RITVKK | 23 |
| 3261 | 6mer-2-861 | KDTDRK | 21 | 3561 | 6mer-2-1161 | RHSFKK | 27 |
| 3262 | 6mer-2-862 | RHTFKK | 29 | 3562 | 6mer-2-1162 | KMSERR | 34 |
| 3263 | 6mer-2-863 | RKHWRR | 17 | 3563 | 6mer-2-1163 | KCHFKK | 37 |
| 3264 | 6mer-2-864 | RKCAKK | 12 | 3564 | 6mer-2-1164 | RYNHKR | 33 |
| 3265 | 6mer-2-865 | RHCNRK | 16 | 3565 | 6mer-2-1165 | RIKAKK | 30 |
| 3266 | 6mer-2-866 | RDNPKK | 24 | 3566 | 6mer-2-1166 | KCSIKK | 26 |
| 3267 | 6mer-2-867 | KYWEKK | 32 | 3567 | 6mer-2-1167 | RCAPKR | 27 |
| 3268 | 6mer-2-868 | RTPNRR | 30 | 3568 | 6mer-2-1168 | KMKPKK | 41 |
| 3269 | 6mer-2-869 | RHDHKR | 18 | 3569 | 6mer-2-1169 | KICERK | 21 |
| 3270 | 6mer-2-870 | KMTEKK | 21 | 3570 | 6mer-2-1170 | KFTIRR | 25 |
| 3271 | 6mer-2-871 | REMPKR | 31 | 3571 | 6mer-2-1171 | KMPARR | 40 |
| 3272 | 6mer-2-872 | KTHFRK | 28 | 3572 | 6mer-2-1172 | KNCERR | 36 |
| 3273 | 6mer-2-873 | KNWGRR | 11 | 3573 | 6mer-2-1173 | KCCNKK | 38 |
| 3274 | 6mer-2-874 | KIHFRK | 33 | 3574 | 6mer-2-1174 | RCEPRK | 21 |
| 3275 | 6mer-2-875 | RIDHRK | 13 | 3575 | 6mer-2-1175 | RYSKRR | 34 |
| 3276 | 6mer-2-876 | KEDNKR | 17 | 3576 | 6mer-2-1176 | RYTEKK | 43 |
| 3277 | 6mer-2-877 | KPPERR | 17 | 3577 | 6mer-2-1177 | RASEKR | 41 |
| 3278 | 6mer-2-878 | KETVRR | 24 | 3578 | 6mer-2-1178 | KTTVRK | 30 |
| 3279 | 6mer-2-879 | RTMDRR | 19 | 3579 | 6mer-2-1179 | KACVKK | 34 |
| 3280 | 6mer-2-880 | RTHHKK | 12 | 3580 | 6mer-2-1180 | RAANRR | 34 |
| 3281 | 6mer-2-881 | KDTEKK | 33 | 3581 | 6mer-2-1181 | KACWKR | 32 |
| 3282 | 6mer-2-882 | RTPAKR | 15 | 3582 | 6mer-2-1182 | RHNDKR | 30 |
| 3283 | 6mer-2-883 | RTNEKR | 22 | 3583 | 6mer-2-1183 | RENNRK | 35 |
| 3284 | 6mer-2-884 | KMTMKK | 11 | 3584 | 6mer-2-1184 | KFWVRR | 22 |
| 3285 | 6mer-2-885 | KCMVRR | 17 | 3585 | 6mer-2-1185 | RIDAKK | 42 |
| 3286 | 6mer-2-886 | KTWVRR | 13 | 3586 | 6mer-2-1186 | KEKFRR | 27 |
| 3287 | 6mer-2-887 | RCPKKR | 18 | 3587 | 6mer-2-1187 | RTADRK | 36 |
| 3288 | 6mer-2-888 | RIAKRK | 17 | 3588 | 6mer-2-1188 | RAPARR | 22 |
| 3289 | 6mer-2-889 | REWDKK | 12 | 3589 | 6mer-2-1189 | KTAPKK | 40 |
| 3290 | 6mer-2-890 | RDNPKR | 30 | 3590 | 6mer-2-1190 | KACIRR | 32 |
| 3291 | 6mer-2-891 | RTSIRK | 28 | 3591 | 6mer-2-1191 | KKADRK | 35 |
| 3292 | 6mer-2-892 | KDENRK | 27 | 3592 | 6mer-2-1192 | KHKNKR | 40 |
| 3293 | 6mer-2-893 | REHVRR | 17 | 3593 | 6mer-2-1193 | REKIRR | 43 |
| 3294 | 6mer-2-894 | RFHWRR | 22 | 3594 | 6mer-2-1194 | KYCDRK | 35 |
| 3295 | 6mer-2-895 | KYMAKK | 28 | 3595 | 6mer-2-1195 | KMTERK | 25 |
| 3296 | 6mer-2-896 | RTCARR | 30 | 3596 | 6mer-2-1196 | RESKKR | 39 |
| 3297 | 6mer-2-897 | RPAHKK | 17 | 3597 | 6mer-2-1197 | KNCIKK | 23 |
| 3298 | 6mer-2-898 | KKKDKK | 16 | 3598 | 6mer-2-1198 | RMSHRR | 27 |
| 3299 | 6mer-2-899 | KKMVKR | 13 | 3599 | 6mer-2-1199 | RCTNKR | 27 |
| 3300 | 6mer-2-900 | RFSAKK | 11 | 3600 | 6mer-2-1200 | RHHNRR | 30 |
| Average Binding affinity | | | | | | | 27.07 |

According to Table 8, the binding affinity for acetylcholine receptor of SEQ ID NO: 73, 5mer-73, with the sequence RRQRR, was significantly higher than other 5mers (binding affinity of 179). Consequently, extended 6mer sequences XRRQRR and RRQRRX were created based on SEQ ID NO: 73 to check the binding affinity for acetylcholine receptor, and the results are presented as relative value to the RRQRR sequence in Table 14.

**TABLE 14**

| XRRQRR-6mer | | | RRQRRX-6mer | | |
|---|---|---|---|---|---|
| SEQ ID NO: | Sequence | Binding affinity | SEQ ID NO: | Sequence | Binding affinity |
| 3601 | GRRQRR | 132 | 3 621 | RRQRRG | 109 |
| 3602 | ARRQRR | 129 | 3622 | RRQRRA | 113 |
| 3603 | VRRQRR | 135 | 3623 | RRQRRV | 120 |
| 3604 | CRRQRR | 123 | 3624 | RRQRRC | 126 |
| 3605 | PRRQRR | 133 | 3625 | RRQRRP | 137 |
| 3606 | LRRQRR | 144 | 3626 | RRQRRL | 141 |
| 3607 | IRRQRR | 121 | 3627 | RRQRRI | 133 |
| 3608 | MRRQRR | 119 | 3628 | RRQRRM | 122 |
| 3609 | WRRQRR | 138 | 3629 | RRQRRW | 129 |
| 3610 | FRRQRR | 143 | 3630 | RRQRRF | 132 |
| 3611 | SRRQRR | 121 | 3631 | RRQRRS | 113 |
| 3612 | TRRQRR | 103 | 3632 | RRQRRT | 117 |
| 3613 | YRRQRR | 130 | 3633 | RRQRRY | 105 |
| 3614 | NRRQRR | 113 | 3634 | RRQRRN | 109 |
| 3615 | QRRQRR | 105 | 3635 | RRQRRQ | 101 |
| 3616 | KRRQRR | 173 | 3636 | RRQRRK | 175 |
| 3617 | RRRQRR | 189 | 3637 | RRQRRR | 192 |
| 3618 | HRRQRR | 164 | 3638 | RRQRRH | 158 |
| 3619 | DRRQRR | 63 | 3639 | RRQRRD | 65 |
| 3620 | ERRQRR | 47 | 3640 | RRQRRE | 37 |

As shown in Table 14, most sequences of XRRQRR and RRQRRX, which are both extended sequences of RRQRR, had high binding affinity for acetylcholine receptor.

Furthermore, the binding affinity of SEQ ID NO: 1410, 6mer-1-210, with the sequence RRGVRR for the acetylcholine receptor was very high compared to other 6mers (binding affinity of 184). Therefore, the extended 7mer sequences XRRGVRR and RRGVRRX were created based on SEQ ID NO: 1410 to check binding affinity for acetylcholine receptor, and the results are presented as values relative to the RRGVRR sequence in Table 15.

**TABLE 15**

| XRRGVRR-7mer | | | RRGVRRX-7mer | | |
|---|---|---|---|---|---|
| SEQ ID NO: | Sequence | Binding affinity | SEQ ID NO: | Sequence | Binding affinity |
| 3641 | GRRGVRR | 137 | 3661 | RRGVRRG | 105 |
| 3642 | ARRGVRR | 134 | 3662 | RRGVRRA | 118 |
| 3643 | VRRGVRR | 140 | 3663 | RRGVRRV | 149 |
| 3644 | CRRGVRR | 128 | 3664 | RRGVRRC | 131 |
| 3645 | PRRGVRR | 138 | 3665 | RRGVRRP | 142 |
| 3646 | LRRGVRR | 149 | 3666 | RRGVRRL | 146 |
| 3647 | IRRGVRR | 126 | 3667 | RRGVRRI | 138 |
| 3648 | WRRGVRR | 124 | 3668 | RRGVRRM | 127 |
| 3649 | WRRGVRR | 134 | 3669 | RRGVRRW | 134 |
| 3650 | FRRGVRR | 139 | 3670 | RRGVRRF | 137 |
| 3651 | SRRGVRR | 117 | 3671 | RRGVRRS | 118 |
| 3652 | TRRGVRR | 105 | 3672 | RRGVRRT | 113 |
| 3653 | YRRGVRR | 145 | 3673 | RRGVRRY | 101 |
| 3654 | NRRGVRR | 109 | 3674 | RRGVRRN | 105 |
| 3655 | QRRGVRR | 101 | 3675 | RRGVRRQ | 105 |
| 3656 | KRRGVRR | 169 | 3676 | RRGVRRK | 171 |
| 3657 | RRRGVRR | 185 | 3677 | RRGVRRR | 188 |
| 3658 | HRRGVRR | 160 | 3678 | RRGVRRH | 154 |
| 3659 | DRRGVRR | 59 | 3679 | RRGVRRD | 61 |
| 3660 | ERRGVRR | 43 | 3680 | RRGVRRE | 33 |

As shown in Table 15, most sequences of XRRGVRR and RRGVRRX, which are both extended sequences of RRGVRR, had very high binding affinity for acetylcholine receptor.

### <EXAMPLE 7. Comparison of Binding Affinity between Top 2 Peptides in Each Library and Positive Control >

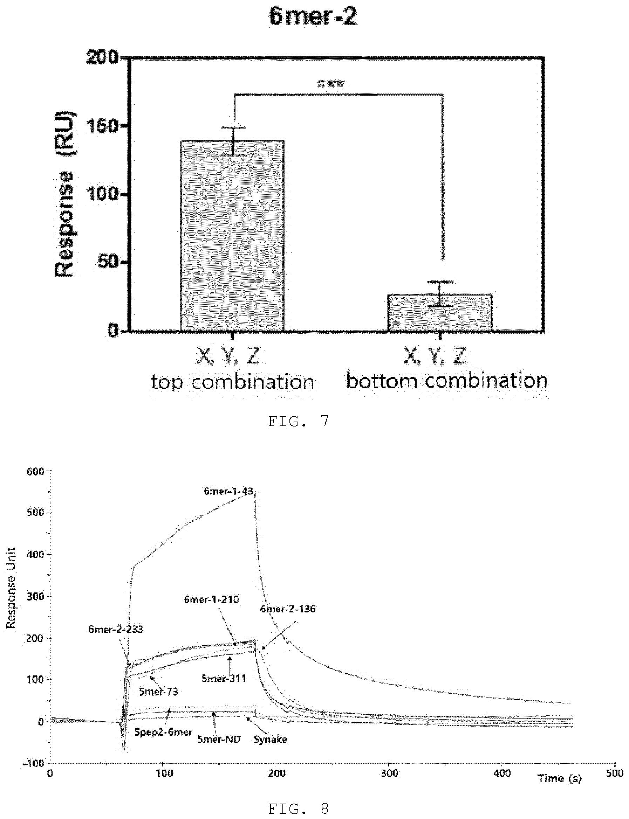

Representative peptides identified in Example 6, two in each library, including 5mers (73, 311) and 6mers-1 (43, 210) and 6mers-2 (136, 233), were compared with the pre-optimized 6mer, 5mer-ND, and Synake. The results are presented in Table 16 and FIG. 8. As can be seen in Table 16 and FIG. 8, the optimized shortened peptides of 5mer and 6mer for XYZ in Example 6 exhibited higher binding affinity for acetylcholine receptor than the pre-optimized ESP-2 short peptides as well as Synake.

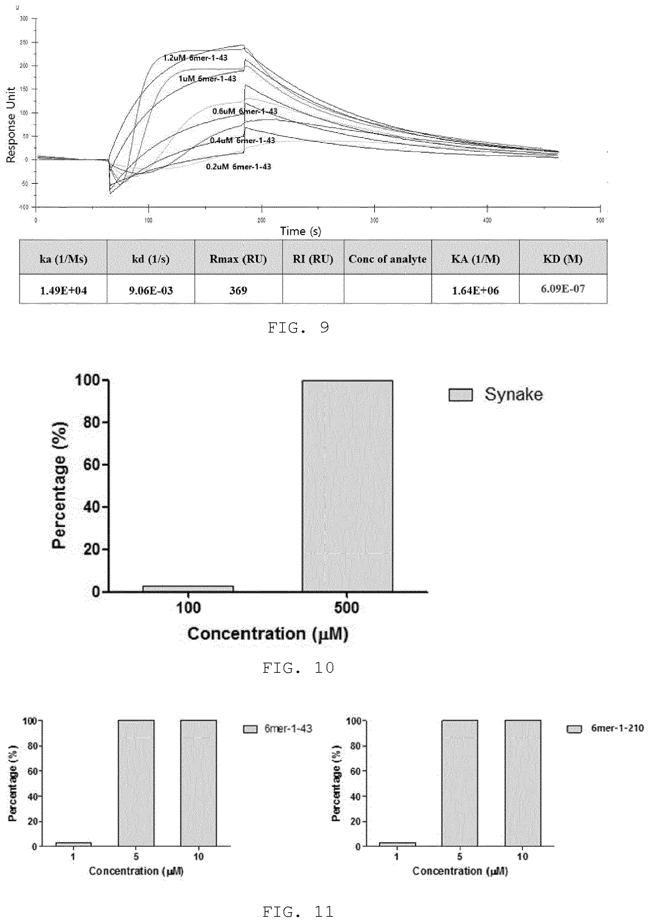

Of the optimized shortened peptides, 6mer-1-43 peptide was measured for binding affinity, and the measurement is shown in FIG. 9. The affinity of 6mer-1-43 peptide according to the present disclosure for acetylcholine receptor was 609 nM.

**TABLE 16**

| No. | Name | Sequence | Binding affinity (RU) |
|---|---|---|---|
| 1 | 5mer-ND | KSLLR | 23 |
| 2 | Synake | - | 14 |
| 3 | 5mer-73 | RRQRR | 179 |
| 4 | 5mer-311 | RSYSR | 167 |
| 5 | Spep 6mer | RKSLLR | 35 |
| 6 | 6mer-1-43 | RKRIRR | 548 |
| 7 | 6mer-1-210 | RRGVRR | 184 |
| 8 | 6mer-2-136 | RWRYKR | 194 |
| 9 | 6mer-2-233 | KWRQKR | 190 |

### <EXAMPLE 8. Acetylcholine Receptor Inhibition by Six Peptides with High Binding Affinity>

The 5mer (73, 311), 6mer-1 (43, 210), and 6mer-2 (136, 233) peptides, which were confirmed to have excellent binding affinity for AchR in Example 5, were examined for inhibitory effects on AchR action.

Acetylcholine receptor-overexpressing TE671 cells were cultured at 37°C in DMEM medium supplemented with 10% FBS and 1% P/S under 5% CO2. After the TE671 cells were sufficiently grown for 4 days, the cells were detached using trypsin and the cell culture was plated in an amount of 1 ml at a density of 2×104 cells/well into 12-well cell culture plate with an 18mm cover-slip lined therein, and cultured for 4 days.

The cover-slip on which the cells had grown was transferred to a fresh 12-well cell culture plate, and 997 µl of HBSS buffer and 3 µl of Fura-2-AM were added and gently mixed, followed by incubation at 37°C, 5% CO2 for 15 minutes. After incubation, the remaining Fura-2-AM was removed by washing 3-4 times with 1 ml of HBSS buffer, and an additional 1 ml was added. The cover-slip with the grown cells was placed in a chamber, and 500 µl of HBSS buffer was dispensed. The final concentration of nicotine was adjusted to 400 µM to observe the calcium imaging reaction. Then, while keeping the nicotine concentration fixed, the concentration of the sample was adjusted to find the inhibiting concentration. Synake was used as a control and the results are presented in FIGS. 10-13.

As shown in FIGS. 10-13, Synake inhibited acetylcholine receptors at 500 µM, while the precursor peptide 11mer did at 5 µM. Meanwhile, the optimized shortened peptides according to the present disclosure, 5mer (73, 311), 6mer-1 (43, 210), and 6mer-2 (136, 233), were found to exhibit inhibition at 10 µM. These newly discovered 6mer peptides showed similar inhibitory ability to the precursor 11mer peptide while being approximately 100 times more potent on average than Synake, and the 5mer peptides showed about 33 times more potent inhibitory ability.

### <EXAMPLE 9. Assay for Acetylcholine Receptor Inhibition by Modifying Peptide Terminal >

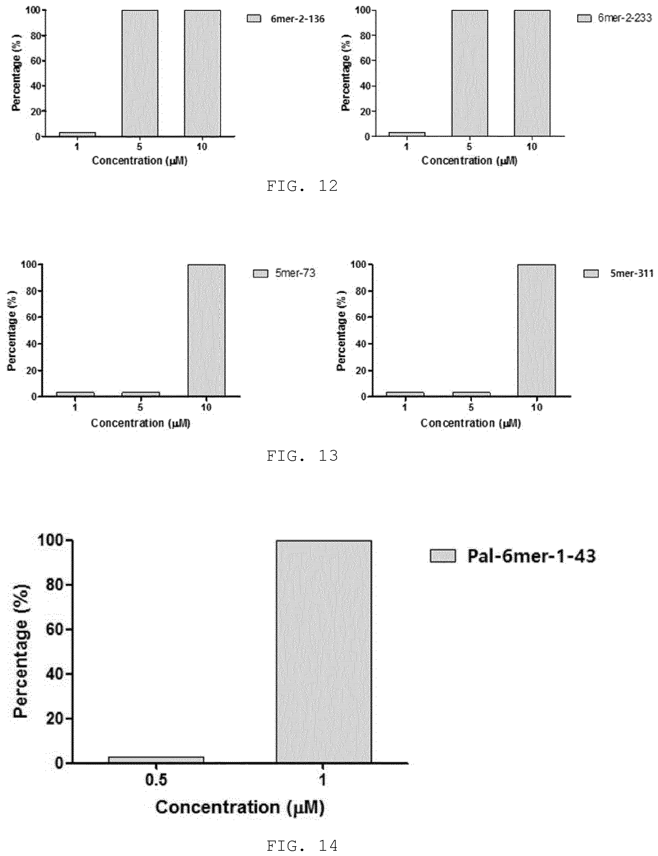

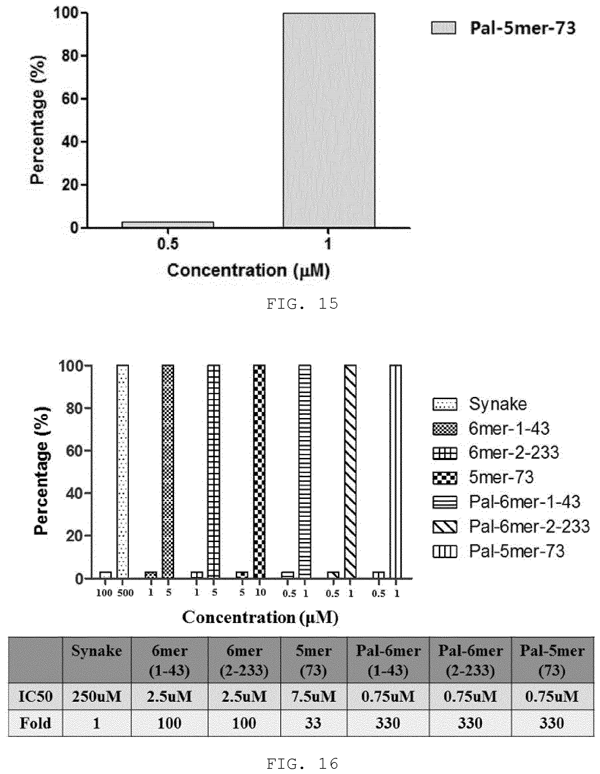

The terminals of the optimized shortened peptides according to the present disclosure were modified with palmitate to examine inhibitory ability against the acetylcholine receptor. The inhibitory ability of the Palmitate-6mer-1-43 and Palmitate-5mer-73 peptides on the acetylcholine receptor was examined in the same manner as in Example 6. As a result, the peptides exhibited 100% inhibition at 1µM, and the measurements are presented in FIGS. 14 and 15. Additionally, the inhibitory ability (IC50) of the optimized short peptides and the palmitate-modified peptides according to the present disclosure against acetylcholine receptor was compared with Synake and is depicted in FIG. 16. As shown in FIGS. 14 to 16, when the termini of the optimized shortened peptides were modified with palmitate, there was a significant increase in inhibitory ability on the acetylcholine receptor compared to the unmodified shortened peptides.

### <EXAMPLE 10. Assay for Cytotoxicity of Optimized Short Peptides>

The optimized shortened peptides according to the present disclosure were assayed for cytotoxicity. In this regard, the cytotoxicity was assessed by the MTT [(3-(4,5-dimethylthiazol-2-yl)-2,5-diphenyltetrazolium bromide] method using dermal fibroblast cells isolated from human skin. The pre-cultured cells were seeded at a concentration of 1×105 cells/mL in a 24-well plate and cultured for 18 hours. Subsequently, the medium was removed and the diluted sample was added to the medium free of FBS and incubated for 24 hours, after which the medium was removed and MTT solution was added at a concentration of 1 ug/mL and reacted for 3 hours. Unreacted MTT was removed, and DMSO 100 µL was added to dissolve the formed formazan, and the absorbance at 540 nm was read on an ELISA reader.

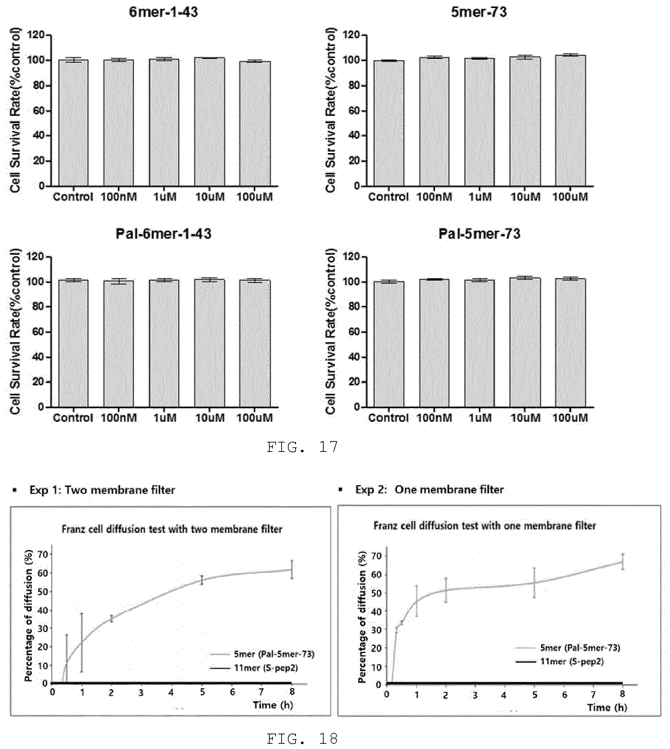

As shown in FIG. 17, the cytotoxicity levels of the 6mer-1-43 (6mer peptide), 5mer-73 (5mer peptide), Pal-6mer-1-43, AND Pal-5mer-73 peptides on dermal fibroblast cells were measured using the MTT method, and all the peptides were evaluated as non-toxic at concentrations ranging from 100nM to 100µM.

### <EXAMPLE 11. Analysis of Skin Permeability According to Peptide Length>

Using the Franz diffusion cell system, comparison was made of skin permeability between the precursor peptide, 11mer, and the optimized shortened peptides of the present disclosure.

To the receptor chamber of the Franz diffusion cell system was added 5ml of PBS (pH 7.4 containing 0.05% polysorbate 80), followed by insertion of one or two sheets of cellulose acetate membrane, which acts as artificial skin, between the receptor and donor chambers. After fixation of the chambers, the existing 11mer and optimized 5mer-73 (RRQRR) peptides were added to the donor chamber of the Franz cell. The receptor chamber was controlled to have the condition of 37°C and 600 rpm, and samples were collected every 0.5, 1, 2, 4, 8, 12, 18, and 24 hours in 500 µl quantities to measure the amount of drug that permeated through the skin over time using HPLC, and the results were displayed in FIG. 18. As seen in FIG. 18, the skin permeability of the optimized short peptide, 5mer, according to the present disclosure was confirmed to be 66.87% after 8 hours, whereas the 11mer did not permeate at all.

As above, the optimized shortened 5mer peptides of the present disclosure showed a similar acetylcholine receptor inhibitory activity to the 11mer peptide, which had shown excellent inhibitory effects on acetylcholine receptors in previous research, but dramatically increased in skin permeability and significantly decreased in production cost due to its shortened length to the half of the original length.

### <EXAMPLE 12. Clinical Evaluation of Efficacy of Peptide Cosmetic Formulation on Eye Wrinkles>

The clinical efficacy of the optimized shortened peptides according to the present disclosure on eye skin wrinkles was evaluated. Test products containing the optimized shortened peptides of the present disclosure and a control group were manufactured as described in Table 17, and human application tests were conducted. Twenty-one participants who met the selection criteria and did not fall under any exclusion criteria were recruited, and after one withdrew their consent to participate, the final number of participants was 20. All measurements were taken after the participants had rested for at least 30 minutes under constant temperature and humidity conditions (22±2°C, 50±10% RH) without direct sunlight or air movement. Selected participants visited the clinical institution before using the product (week 0) to measure eye skin texture (wrinkles) and conduct demographic surveys. They applied the test product twice daily (morning and evening) for 6 weeks and revisited the clinical institution at weeks 3 and 6 after using the product for evaluation under the same conditions as week 0.

**TABLE 17**

| No | TRADE NAME | INCI NAME | 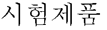 WT% | 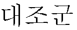 WT% |
|---|---|---|---|---|
| 1 | Distilled water1 | WATER | 50∼80 | 50∼80 |
| 2 | Adenosine | Adenosine | 0.01∼0.1 | 0.01∼0.1 |
| 3 | PANTHENOL | PANTHENOL | 0.1∼1.0 | 0.1∼1.0 |
| 4 | KMO-6 | 1,2-Hexandiol | 1∼5 | 1∼5 |
| 5 | 1,3 BG | BUTYLENE GLYCOL | 2∼8 | 2∼8 |
| 6 | DPG-FC | DIPROPYLENE GLYCOL | 2∼8 | 2∼8 |
| 7 | GLYCERINE | GLYCERIN | 3∼15 | 3∼15 |
| 8 | Viscomate NP 700 | SODIUM POLYACRYA\|LATE | 0.01∼0.1 | 0.01∼0.1 |
| 9 | KELTROL F | XANTHAN GUM | 0.1∼1.0 | 0.1∼1.0 |
| 10 | SIMULGEL NS | Hydroxyethyl Acrylate / Sodium | 1∼5 | 1∼5 |
| 11 | Na-HYALURONATE | Sodium Hyaluronate | 0.5∼5.0 | 0.5∼5.0 |
| 12 | Glucan Real | Beta-Glucan | 0.5∼5.0 | 0.5∼5.0 |
| 13 | Peptide | - | Suitable amount | - |
| 14 | Perfume | Fragrance | Suitable amount | Suitable amount |

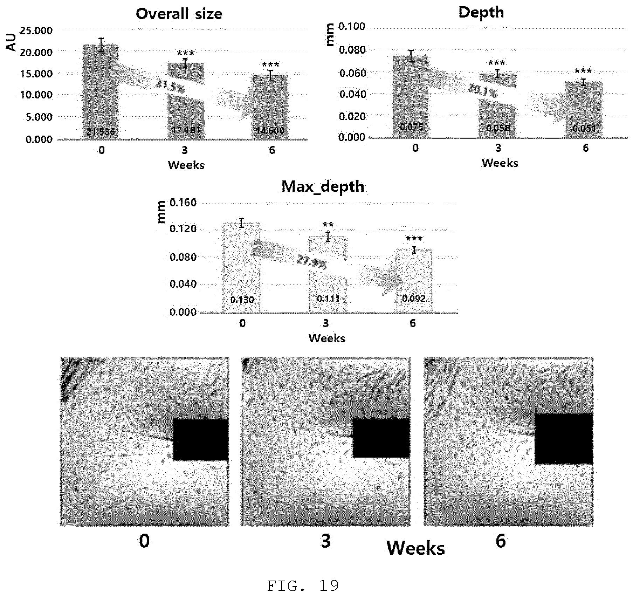

The evaluation items included the overall size of wrinkle depth, and maximum depth, which were measured and analyzed for eye skin wrinkles using ANTERA 3D^{®} CS (Miravex, Ireland), and the results were compared to those at week 0 and displayed in Table 18 and FIG. 19. The control group did not show any significant differences in all evaluation items.

**TABLE 18]**

| Item | Week | Mean ± SD. | Change(%) | p -value |
|---|---|---|---|---|
| Overall size (AU) | 0 | 21.536 ± 1.5225 | - | - |
| | 3 | 17.181 ± 1.0693 | ▼19.1 | <0.001*** |
| | 6 | 14.600 ± 1.0447 | ▼31.5 | <0.001*** |
| Depth (mm) | 0 | 0.075 ± 0.0048 | - | - |
| | 3 | 0.058 ± 0.0033 | ▼21.0 | <0.001*** |
| | 6 | 0.051 ± 0.0027 | ▼30.1 | <0.001*** |
| Maximum depth (mm) | 0 | 0.130 ± 0.0059 | - | - |
| | 3 | 0.111 ± 0.0066 | ▼14.2 | <0.004*** |
| | 6 | 0.092 ± 0.0048 | ▼27.9 | <0.001*** |

As shown in Table 18 and FIG. 19, the overall size of eye wrinkles decreased by 19.1% at week 3 (p<0.001) and 31.5% at week 6 (p<0.001) compared to before product use. Depth decreased by 21.0% at week 3 (p<0.001) and 30.1% at week 6 (p<0.001), and maximum depth decreased by 14.2% at week 3 (p<0.001) and 27.9% at week 6 (p<0.001) compared to before product use.

### <EXAMPLE 13. Clinical Evaluation of Efficacy of Peptide Cosmetic Formulation on Skin Elasticity>

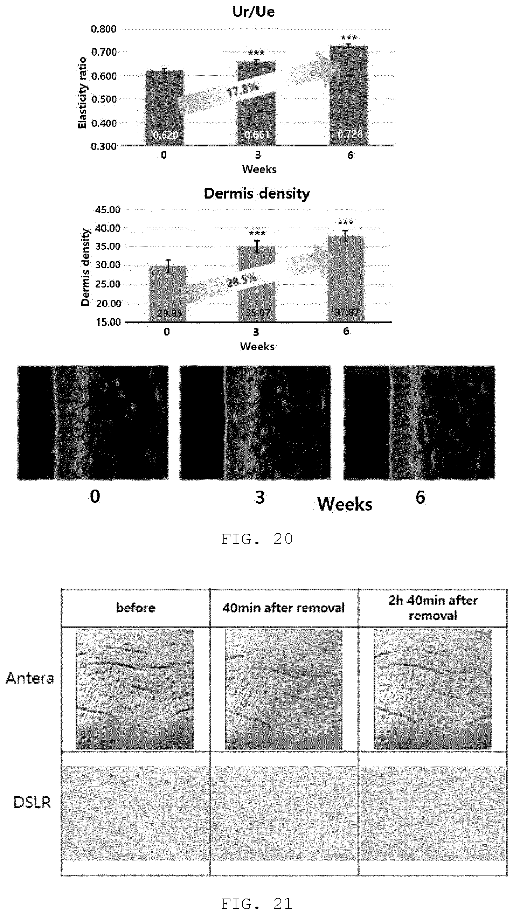

The clinical efficacy on skin elasticity (Ur/Ue) was evaluated in the same manner as in Example 12. The dermal density was measured using an ultrasound device, DUB^{®} Skinscanner (tpm, Germany). The dermal density of the participants' cheeks was measured before product use (week 0), and at 3 and 6 weeks after product use, and the results were compared and presented in FIG. 20.

As shown in FIG. 20, skin elasticity (Ur/Ue) increased by 6.9% at week 3 (p<0.001) and 17.8% at week 6 (p<0.001) compared to before product use. The dermal density increased by 18.9% at 3 weeks (p<0.001) and 28.5% at 6 weeks (p<0.001) compared to before product use.

### <Example 14. Clinical Evaluation of the Efficacy of Peptide Cosmetic Formulation on Forehead Wrinkles>

The clinical efficacy of the optimized short peptide cosmetic formulation according to the present disclosure on forehead wrinkles was evaluated. Forehead wrinkle areas were photographed using Antera 3D and DSLR before product use. Then, gauze (5cm×5cm) soaked with the test product and control group mentioned in Table 17 was applied to the forehead wrinkle area for 8 hours, after which the gauze was removed and the forehead wrinkle area was photographed again using Antera 3D and DSLR at 40 minutes and 2 hours and 40 minutes post removal, and the results are displayed in Figure 21.

As shown in FIG. 21, compared to before the removal of the product, a significant improvement in forehead wrinkles was observed, and the wrinkle improvement effect was maintained even after more than 2 hours.

## Claims

1. An acetylcholine receptor-binding peptide, comprising the amino acid sequence represented by the following Chemical Formula 1:
[Chemical Formula 1] (R/K) XXX (R/K)
(wherein R/K is arginine or lysine, X is any amino acid).

2. An acetylcholine receptor-binding peptide, comprising the amino acid sequence represented by the following Chemical Formula 1-1:
[Chemical Formula 1-1] (R/K) XYZ (R/K)
(wherein R/K represents either arginine or lysine, and XYZ represents a sequence of any three consecutive amino acids, with X being an amino acid selected from R, Q, G, V, L, S, and W, Y being an amino acid selected from R, Q, L, I, F, V, and Y, and Z being an amino acid selected from R, S, L, C, Y, Q, and T).

3. The acetylcholine receptor-binding peptide of claim 2, wherein the peptide comprises an amino acid sequence selected from the group consisting of the amino acid sequences of SEQ ID NOS: 1 to 600.

4. An acetylcholine receptor-binding peptide, comprising the amino acid sequence represented by the following Chemical Formula 1-2:
[Chemical Formula 1-2] XRRQRR
(wherein R represents arginine, Q represents glutamine, and X represents any one amino acid).

5. The acetylcholine receptor-binding peptide of claim 4, wherein the peptide comprises an amino acid sequence selected from the group consisting of the amino acid sequences of SEQ ID NOS: 3601 to 3620.

6. An acetylcholine receptor-binding peptide, comprising the amino acid sequence represented by the following Chemical Formula 1-3:
[Chemical Formula 1-3] RRQRRX
(wherein R represents arginine, Q represents glutamine, and X represents any one amino acid).

7. The acetylcholine receptor-binding peptide of claim 6, wherein the peptide comprises an amino acid sequence selected from the group consisting of the amino acid sequences of SEQ ID NOS: 3621 to 3640.

8. An acetylcholine receptor-binding peptide, comprising the amino acid sequence represented by the following Chemical Formula 2:
[Chemical Formula 2] (R/K) (R/K) XXX (R/K)
(wherein R/K represents either arginine or lysine, and X represents any one amino acid).

9. An acetylcholine receptor-binding peptide, comprising the amino acid sequence represented by the following Chemical Formula 2-1:
[Chemical Formula 2-1] (R/K) (R/K) XYZ (R/K)
(wherein R/K represents either arginine or lysine, XYZ represents a sequence of any three consecutive amino acids, with X being an amino acid selected from R, Q, G, V, L, S, and W, Y being an amino acid selected from R, Q, L, I, F, V, and Y, and Z being an amino acid selected from R, S, L, C, Y, Q, and T).

10. The acetylcholine receptor-binding peptide of claim 9, wherein the peptide comprises an amino acid sequence selected from the group consisting of the amino acid sequences of SEQ ID NO: 1201 to 1800.

11. An acetylcholine receptor-binding peptide, comprising the amino acid sequence represented by the following Chemical Formula 2-2:
[Chemical Formula 2-2] XRRGVRR
(wherein R represents arginine, G represents glycine, V represents valine, and X represents any one amino acid).

12. The acetylcholine receptor-binding peptide of claim 11, wherein the peptide comprises an amino acid sequence selected from the group consisting of the amino acid sequences of SEQ ID NOS: 3641 to 3660.

13. An acetylcholine receptor-binding peptide, comprising the amino acid sequence represented by the following Chemical Formula 2-3:
[Chemical Formula 2-3] RRGVRRX
(wherein R represents arginine, G represents glycine, V represents valine, and X represents any one amino acid).

14. The acetylcholine receptor-binding peptide of claim 13, wherein the peptide comprises an amino acid sequence selected from the group consisting of the amino acid sequences of SEQ ID NOS: 3661 to 3680.

15. An acetylcholine receptor-binding peptide, comprising the amino acid sequence represented by the following Chemical Formula 3:
(R/K) XXX (R/K) (R/K)
(wherein R/K represents either arginine or lysine, and X represents any one amino acid).

16. An acetylcholine receptor-binding peptide, comprising the amino acid sequence represented by the following Chemical Formula 3-1:
[Chemical Formula 3-1] (R/K)XYZ(R/K)(R/K)
(wherein R/K represents either arginine or lysine, XYZ represents a sequence of any three consecutive amino acids, with X being an amino acid selected from R, Q, G, V, L, S, and W, Y being an amino acid selected from R, Q, L, I, F, V, and Y, and Z being an amino acid selected from R, S, L, C, Y, Q, and T).

17. The acetylcholine receptor-binding peptide of claim 16, wherein the peptide comprises an amino acid sequence selected from the group consisting of the amino acid sequences of SEQ ID NOS: 2401 to 3000.

18. The acetylcholine receptor-binding peptide of any one of claims 1 to 17, wherein the peptide has a modification to the N-terminus or C-terminus thereof.

19. The acetylcholine receptor-binding peptide of claim 18, the modification to the N-terminus or C-terminus is palmitoylation, acetylation, formylation, PEGylation, or conjugation with 2-mercaptoacetic acid, 3-mercaptopropionic acid, 6-mercaptohexanoic acid, pyroglutamic acid, succinimide acid, amide, cystramine, methyl ester, ethyl ester, benzyl ester, or a fatty acid, such as at least one selected from the group consisting of myristic acid, stearic acid, palmitic acid, cholesterol, 6-amino hexanoic acid, and 8-amino octanoic acid.

20. A polynucleotide, coding for the peptide of any one of claims 1 to 17.

21. A cosmetic composition for relief of skin wrinkles, comprising the peptide of any one of claims 1 to 17.

22. A composition for prevention or treatment of an acetylcholine receptor hyperactivity-associated disease, comprising the peptide of any one of claims 1 to 17.

23. The composition of claim 22, wherein the acetylcholine receptor hyperactivity-associated disease is at least one selected from the group consisting of cervical dystonia, limb dystonia, truncal dystonia, blepharospasm, spasticity, hemifacial spasm, strabismus, nystagmus, tics, chronic pain, chronic migraine, neurogenic bladder, detrusor-sphincter dyssynergia, achalasia cardia, hyperhidrosis, neuropathic pain, skin wrinkles, square jaw, and sialorrhea.

24. The health functional food composition for alleviation of an acetylcholine receptor hyperactivity-associated disease, comprising the peptide of any one of claims 1 to 17.

25. A composition for a medical device, comprising the acetylcholine receptor-binding peptide of any one of claims 1 to 17.
